(19) Europäisches Patentamt / European Patent Office / Office européen des brevets

(11) EP 4 445 918 A1

(12) EUROPEAN PATENT APPLICATION

(43) Date of publication:
16.10.2024 Bulletin 2024/42

(21) Application number: 24170219.0

(22) Date of filing: 15.04.2024

(51) International Patent Classification (IPC):
*A61K 47/54* (2017.01) *A61K 47/64* (2017.01)
*A61K 39/395* (2006.01) *A61P 35/00* (2006.01)
*C07K 14/47* (2006.01) *C07K 16/28* (2006.01)
*A61K 39/00* (2006.01)

(52) Cooperative Patent Classification (CPC):
(C-Sets available)
**A61P 35/00; A61K 39/39558; A61K 47/542; A61K 47/645; C07K 14/4705; C07K 16/2818;**
A61K 2039/505; C07K 2319/10 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 14.04.2023 EP 23168126

(71) Applicant: **STipe Therapeutics ApS**
**8230 Åbyhøj (DK)**

(72) Inventors:
- **Sensfuss, Ulrich**
  **2720 Vanløse (DK)**
- **Strandh, Magnus**
  **21131 Malmö (SE)**
- **Krapp, Christian**
  **2300 København (DK)**
- **Bethell, Richard Charles**
  **11230 Stockholm (SE)**
- **Jakobsen, Martin Roelsgaard**
  **8240 Risskov (DK)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

# (54) STING SENSITISING AGENTS

(57) The invention relates to agents capable of sensitising STING activation, compositions comprising said agents and medical uses thereof.

*Figure 4 (continued)*

c

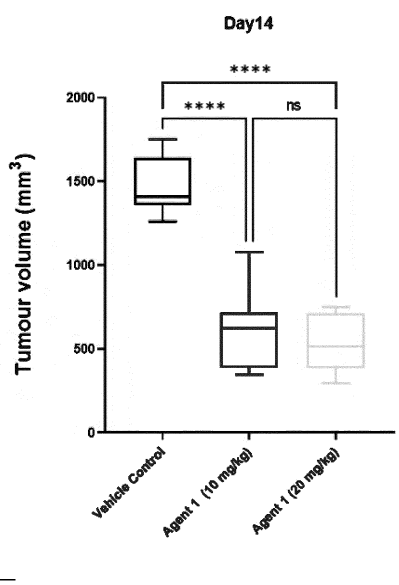

(52) Cooperative Patent Classification (CPC): (Cont.)

C-Sets
**A61K 39/39558, A61K 2300/00**

## Description

### *Field of the Invention*

The present invention relates to agents capable of sensitising STING activation, compositions comprising said agents and medical uses thereof.

### *Background*

Innate immune activation by cytosolic DNA from microbial pathogens is a potent trigger of type I interferon (IFN) and pro-inflammatory cytokine expression. The cytosolic DNA is detected by the cyclic GMP-AMP synthase (cGAS)-stimulator of interferon genes (STING) pathway. In particular, three proteins have been demonstrated to play a role in DNA-driven type I and III IFN responses. These are cGAS, IFN gamma-inducible factor 16 (IFI16) and STING.

cGAS is a cytosolic protein, which is important for sensing all forms of structured DNA and is recognised a key sensor of microbial DNA. Upon binding DNA, the protein cyclic GMP-AMP Synthase (cGAS) triggers the formation of cyclic GMP-AMP (cGAMP) from GTP and ATP. cGAMP docks onto the endoplasmic reticulum (ER)-bound STING, which induces a conformational change allowing STING to homodimerize, migrate from the ER, and recruit TANK-binding kinase 1 (TBK1). TBK1 binding to STING then initiates a complex cascade of events including phosphorylation of STING as well as recruitment and activation of interferon regulatory factor 3 (IRF3). IRF3 can then trigger transcription of inflammatory genes in the nucleus such as genes coding for Type I IFN.

IFI16 is a cytosolic and nuclear protein which has been associated with induction of type I IFNs (e.g., IFN-$\alpha$ and IFN-$\beta$) upon stimulation with single stranded and double stranded DNA as well as by infection with different herpes viruses, human immunodeficiency virus type 1 and bacteria. The inventors have previously shown that IFI16 is required for efficient cGAMP production through cGAS in response to DNA. It has also been shown to actively recruit TBK1 to the cGAMP-stimulated STING complex and promote phosphorylation of STING. This process is thought to rely on IFI16 pyrin domain-dependent protein-protein interactions. Thus, IFI16 is a regulator of STING activation and is an important component in the DNA sensing pathway (Jønsson et al., 2017).

The stimulator of interferon genes (STING) plays a crucial role in the activation of type I interferon (IFN) in response to viral and bacterial infections. STING is located in the endoplasmic reticulum and is activated by cGAMP as a result of the presence of cytoplasmic DNA, which is a common feature of many viral and bacterial pathogens. Upon activation, STING translocates to the nucleus and recruits and activates the TANK-binding kinase 1 (TBK1) and IKK$\varepsilon$ kinases, which then phosphorylate and activate the transcription factor interferon regulatory factor 3 (IRF3). Activated IRF3 migrates to the nucleus, where it induces the transcription of type I IFN genes.

The inventors have also previously identified compounds capable of mimicking the pyrin-domain of IFI16. These compounds, described in WO 2018/029256, comprise the pyrin-domain of IFI16 or a fragment thereof and are capable of interacting with STING and increasing STING activity. In WO 2019/154901, the inventors also identified polypeptides having the formula $KKX_3KNIVLLX_{10}LX_{13}VINX_{17}YHF$ (where X is selected from any proteinogenic and non-proteinogenic amino acid residue, and $X_3$ is not tyrosine (Y) and $X_{10}$ is not lysine (K) and $X_{13}$ is not glutamic acid (E) and $X_{17}$ is not aspartic acid (D)), which are also capable of increasing STING activity.

### *Summary of the Invention*

The invention includes the combination of the aspects and preferred features described except where such a combination is clearly impermissible or expressly avoided.

A first aspect provides an agent comprising an amino acid sequence, wherein the amino acid sequence comprises KKAKNIVLLKGLEVINDWHF (SEQ ID NO: 1).

The agent may further comprise one or more additional moieties that are conjugated to the amino acid sequence. For instance, the amino acid sequence may be conjugated to a membrane transit moiety and/or the amino acid sequence may be conjugated to a half-life extending moiety. The membrane transit moiety may be conjugated to the C-terminus of the amino acid sequence. The half-life extending moiety may be conjugated to the N-terminus of the amino acid sequence.

The agent of the invention may comprise a membrane transit moiety and a half-life extending moiety. In some embodiments, the agent of the invention has the formula:

X-Y-Z

where

X is a half-life extending moiety;
Y is an amino acid sequence comprising SEQ ID NO: 1; and
Z is a membrane transit moiety.

The membrane transit moiety may be a cell penetrating peptide (CPP). The CPP may be a cyclic CPP, such as cyclo[CrRrGrKkRrC] (SEQ ID NO: 2), where the lower case letters are D-amino acids and the cysteine side chains have been oxidized so as to form a disulfide bond rendering the CPP cyclic. The half-life extending moiety may be or comprise a lipophilic substituent and/or a plasma protein binding moiety such as an albumin binding moiety. In some embodiments, the half-life extending moiety is 15-carboxypentadecanoyl-Adoa-Adoa- or 15-carboxypentadecanoyl-yGlu-Adoa-Adoa-.

In some embodiments, the agent of the invention has the structure (15-carboxypentadecanoyl-Adoa-Adoa-KKAKNIVLLKGLEVINDWHF-c[CrRrGrKkRrC]-$NH_2$ (SEQ ID NO: 3)):

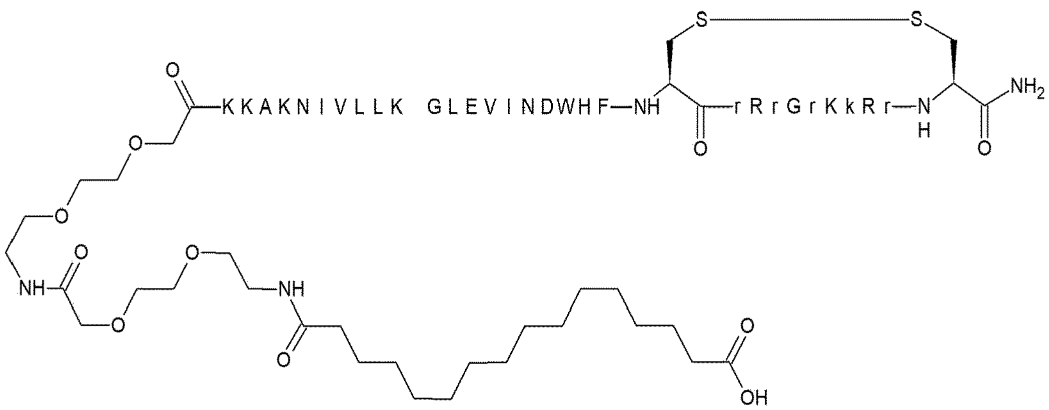

where the lower case letters are D-amino acids and the cysteine side chains form a disulfide bond.

The agent described herein may increase and accelerate trafficking of STING from the endoplasmic reticulum to the Golgi in a cell comprising the agent compared to an equivalent cell not comprising the agent, where both cells are exposed to or comprise a STING stimulating agent. In some embodiments, the level of phosphorylated STING (e.g., STING phosphorylated at Ser366) is increased in a cell comprising the agent compared to an equivalent cell not comprising the agent, where both cells are exposed to or comprise a STING stimulating agent. In some embodiments, the level of phosphorylated TBK1 (e.g. TBK1 phosphorylated at Ser172) is increased in a cell comprising the agent compared to an equivalent cell not comprising the agent, where both cells are exposed to or comprise a STING stimulating agent. In some embodiments, expression of type 1 IFN is increased in a cell comprising the agent compared to an equivalent cell not comprising the agent, where both cells are exposed to or comprise a STING stimulating agent. Additionally, or alternatively, expression of CXCL10 may be increased in a cell comprising the agent compared to an equivalent cell not comprising the agent, where both cells are exposed to or comprise a STING stimulating agent. The level of STING stimulating agent in both cells may be the same. The cell comprising the agent and the equivalent cell not comprising the agent may both comprise a STING stimulating agent at a level sufficient to activate the STING signalling pathway. In some embodiments, the STING stimulating agent is endogenous to the cells. In some embodiments, the source of STING stimulating agent is exogenous to the cells. The STING stimulating agent may be cGAMP.

The agent of the invention may sensitise STING activation. The agent of invention may decrease the $EC_{50}$ of STING stimulating agent-dependent expression of type I IFNs (e.g., IFN-β) in a cell comprising the agent compared to an equivalent cell not comprising the agent, where both cells are exposed to or comprise a STING stimulating agent. The agent may decrease the $EC_{50}$ of STING stimulating agent-dependent expression of CXCL1 0 in a cell comprising the agent compared to an equivalent cell not comprising the agent, where both cells are exposed to or comprise a STING stimulating agent. The agent of the invention may enhance STING activity in the presence of a STING stimulating agent. The agent may increase the $E_{max}$ of STING stimulating agent-dependent expression of type I IFNs (e.g., IFN-β) in a cell comprising the agent compared to an equivalent cell not comprising the agent, where both cells are exposed to or comprise a STING stimulating agent. The agent may increase the $E_{max}$ of STING stimulating agent-dependent expression of CXCL10 in a cell comprising the agent compared to an equivalent cell not comprising the agent, where both cells are exposed to or comprise a STING stimulating agent. The level of STING stimulating agent in both cells may be the same. The cell comprising the agent and the equivalent cell not comprising the agent may both comprise a STING stimulating at a level sufficient to activate the STING signalling pathway. In some embodiments, the STING stimulating agent is endogenous to the cells. In some embodiments, the source of STING stimulating agent is exogenous to the cells. The STING stimulating agent may be cGAMP.

In some embodiments, the cell is an immune cell. The cell may be a macrophage or monocyte. The cell may be a cancer cell or a cell infected with a virus. The cell may be a cancer cell treated with cancer therapies such as chemotherapies, radiation therapies or small molecules targeted therapies that increase the amount of cytoplasmic DNA and therefore activate the STING pathway. The type I IFN may be IFN-$\alpha$ or IFN-$\beta$. In some embodiments, the type I IFN is IFN-$\beta$.

The agent described herein may have reduced immunogenic potential compared to a reference agent. The reference agent may comprise an amino acid sequence, where the amino acid sequence comprises or consists of KKYKNIVLLKGLEVINDYHF, SEQ ID NO: 4.

A further aspect of the invention provides a nucleic acid encoding a peptide comprising an amino acid sequence, where the amino acid sequence comprises KKAKNIVLLKGLEVINDWHF (SEQ ID NO: 1). The nucleic acid may be delivered to a target cell via a viral vector. The nucleic acid may be delivered to a target cell via a non-viral carrier.

An aspect of the invention provides a composition comprising the agent of the invention. The composition may further comprise a STING stimulating agent. The STING stimulating agent may be selected from the group consisting of: a) a nucleic acid; b) a cyclic-di-nucleotide (CDNs); c) a non-CDN small molecule STING stimulating agent; d) an ENPP1 inhibitor; e) a nanovaccine; f) an antibody-drug conjugate; g) a bacterial vector, and/or h) an extracellular vesical comprising one or more STING stimulating agents (such as exoSTING). In some embodiments, the STING stimulating agent is selected from the group consisting of a) a nucleic acid; b) a cyclic-di-nucleotide (CDNs); c) a non-CDN small molecule STING stimulating agent; or d) an ENPP1 inhibitor. The STING stimulating agent may be a CDN.

Additionally, or alternatively, the composition may further comprise DNA damage inducing agent. The DNA damage inducing agent may be a PARP inhibitor, an ATM inhibitor, a POLQ inhibitor, a topoisomerase inhibitor, a DNA crosslinking agent, or a radionuclide.

Additionally, or alternatively, the composition may further comprise a checkpoint inhibitor. The checkpoint inhibitor may be an anti-PD1 antibody or an anti-PD-L1 antibody.

Another aspect of the invention provides an agent as described herein for use in therapy. The invention also provides an agent as described herein for use in a method of treating cancer.

In some embodiments, the method further comprises administering a STING stimulating agent. The agent and STING stimulating agent may be administered simultaneously. Alternatively, the agent and STING stimulating agent may be administered at different times (e.g., sequentially).

In some embodiments, the method further comprises administering a DNA damage inducing agent. The DNA damage inducing agent may be administered simultaneously or sequentially.

In some embodiments, the method further comprises administering a checkpoint inhibitor (e.g., an anti-PD1 antibody). The checkpoint inhibitor may be administered simultaneously or sequentially.

The invention also provides the composition as described herein for use in therapy. Further provided is the composition of the invention for use in treating cancer.

***Summary of the Figures***

Embodiments and experiments illustrating the principles of the invention will now be discussed with reference to the accompanying figures in which:

**Figure 1. Agent 1 enhances STING pathway activation.**
Western Blot of PMA-differentiated THP-1 cells after 2',3'-cGAMP stimulation and Agent 1 sensitization. Membrane stained to assess STING and TBK1 expression and phosphorylation. UT: unstimulated (lane 1); lanes 2-10: stimulated with 2'3'-cGAMP (8 $\mu$g/mL) without (lanes 2-5) or with 5 mM Agent 1 (lanes 7-10). No sample was loaded in lane 6. Last lane shows THP-1 cells treated with peptide only. Treatment times are indicated at the top of the blot (minutes). PMA-differentiated THP1 cells were either left untreated (time point 0) or activated with 8 $\mu$g/mL 2'3'-cGAMP. After 30 min pre-incubation Agent 1 (5 $\mu$M) was added to cell cultures and then lysates after 30, 60, 120 or 360 minutes.

**Figure 2. Agent 1 in combination with a STING agonist enhances STING trafficking to the Golgi.**
**A)** Graphic showing STING trafficking to the Golgi. **B)** The PMA-differentiated THP-1 cells were treated with 6 $\mu$M Agent 1 or peptide buffer alone, 6 $\mu$M Agent 1 or peptide buffer together with 50 $\mu$g/mL cGAMP and stained for STING, GM130 (Golgi marker) and PDI (ER marker). Between 17,000 and 20,000 cells from each sample were acquired on ImageStream®. Each point represents one sample from 5 independent experiments (n equals 2-5 depending on the sample) and bars represent mean +/- SD. **C)** The PMA-differentiated THP-1 cells were treated with 6 $\mu$M Agent 1 or peptide buffer alone, 6 $\mu$M Agent 1 or peptide buffer together with 50 $\mu$g/mL cGAMP and stained for STING, GM130 (Golgi marker) and PDI (ER marker). Between 17,000 and 20,000 cells from each sample were acquired on ImageStream®. Each point represents one sample from 5 independent experiments (n equals 2-5

depending on the sample, except sample peptide buffer + 50 μg/mL cGAMP where n=1) and bars represent mean +/- SD.

**Figure 3. Agent 1 combines with STING agonists to increase chemokine and cytokine expression when compared to different STING agonists (single agents).**

**A)** Figure showing timeline and procedure for the *in vitro* sensitization assay in THP1. **B)** CXCL10 release upon stimulation and sensitization with cGAMP +/- Agent 1. Undifferentiated THP1 cells were treated with cGAMP (300 - 0.59 μg/mL) for 30 minutes prior to sensitization with or without 2 μM Agent 1. At twenty-hours supernatants were collected and analyze for human CXCL10 (representative of n=4). Data represents means ± SD of technical triplicates and data was fitted to a bell-shaped dose response equation where x =concentration. Cell viability data at 20h is included for comparison of cGAMP effect. All data were analyzed using Synergy HI (Biotek Gen5 SW), Microsoft Excel and GraphPad Prism 9.3.1. **C)** IFN-β release upon stimulation and sensitization with cGAMP +/- Agent 1. Undifferentiated THP1 cells were treated with cGAMP (300 - 0.59 μg/mL) for 30 minutes prior to sensitization with or without 2 μM Agent 1. At six- hours supernatants were collected and analyze for human IFN-β (representative of n=4). Data represents means ± SD of technical triplicates and data was fitted Agonist vs response -variable slope (four parameters). **D)** Summary table showing the $EC_{50}$ and $E_{Max}$ for 2'3'-cGAMP effect on CXCL10 and IFN-b response with or without Agent 1 from THP-1 cells (mean ± SD), n=4. **E)** Summary table showing the $EC_{50}$ and $E_{Max}$ for diABZI compound 3 effect on CXCL10 and IFN-b response with or without Agent 1 from THP-1 cells (mean ± SD), n= 4. Summary table showing the $EC_{50}$ and $E_{Max}$ for ADU-S100 effect on CXCL10 and IFN-β response with or without Agent 1 from THP-1 cells (mean ± SD), n= 2.

**Figure 4. Agent 1 sensitizes MC38 tumours to endogenous STING pathway in *in vivo* syngeneic model.**

**A)** Figure showing administration timeline. **B)** Agent 1 induces tumour growth delay in a MC38 subcutaneous syngeneic tumours in a dose dependent manner when used as a single agent. MC38 tumour growth after IV dosing of Agent 1. (Curves represent the mean tumour volume after control or Agent 1 administration IV. **C)** The box graphs present the median tumour volume, calculated on the $14^{th}$ day. The differences between the groups were estimated using the Tukey's multiple comparison test (ns: not significant, ****p < 0.0001).

**Figure 5. Agent 1 synergizes with suboptimal dose of the STING agonist ADU-S100 and induces tumour growth delay in a MC38 subcutaneous syngeneic tumours.**

**A)** Schematic representation of the in vivo experiment. MC38 are implanted subcutaneously on the flank of C57B/6 mice. Once the tumours were clearly established (around 100 $mm^3$), the mice were randomized on day 7 into 4 different groups based on their tumour size. **B)** Table summarizing the treatment dose and schedules for the 4 groups. **C)** Spider plots of a representative experiment showing individual MC38 tumour volumes (n = 11 or 12 per group). Tumour-bearing mice were dosed IV and IT with Saline, Agent 1, ADU-S100 or a combination of both Agent 1 and ADU-S100 starting from day 7 following tumour cell implantation. **D)** Kaplan-Meier plot depicting the survival (as defined as the time to reach 400 $mm^3$) of mice injected with MC38 tumour and treated with Agent 1 (IV), suboptimal dose of ADU-S100 (IT) or a combination of both. The median survival for each group is indicated in the table. The result for the statistical analysis Log-Rank Mental-Cox are indicated between the two treatment groups dosed with ADU-S100 (p=0.07).

**Figure 6. Coadministration of Agent 1 and anti-PD1 produces significant anti-tumour growth delay against CT26.WT syngeneic mouse model.**

**A)** schematic representation of the in vivo experiment. CT26 were implanted subcutaneously on the flank of Balb/c mice. The randomization was conducted after 9 days (New Day 0) when the mean tumour size reached approximately 100 $mm^3$. **B)** Treatment groups and schedules. Day of randomization is denoted as day 0. Dosing is started on day 0; Dose Agent 1 was 1 hour post anti-PD1 on days when both compounds were administered. **C)** Spider plots of tumour volumes for each individual mouse in the 4 different groups. Day 0 is the day at which the treatments were initiated. **D)** Kaplan-Meier plot depicting the survival (as defined as the time to reach 1200 $mm^3$) of mice injected with CT26 tumour and treated with Agent 1 (IV), anti-PD1 (IP) or a combination of both. The median survival for each group is indicated in the table. The results for the statistical analysis Log-Rank Mental-Cox are indicated between the treatment groups that showed significant differences (* for p<0.05 and ** for p<0.01).

**Figure 7. *In silico* immunogenicity prediction for Agent 1 and Agent 2.**

EpiMatrix Analysis of the core sequence (20 amino acids) of Agent 1 and Agent 2 EpiMatrix (EMX) scores above 10 are comparable to those of known promiscuous Class II epitopes. JanusMatrix (JMX) human homology scores >3.00 are considered elevated. All the putative T cell epitopes identified by EpiMatrix were screened against the La Jolla Institute for Allergy and Immunology's Immune Epitope Database (IEDB), which contains T cell epitopes that

have been previously studied and evaluated.

**Figure 8. *In vitro* immunogenicity analysis.**
**A)** Table showing the structure and molecular weight of peptides tested. **B)** Table showing RI Values for each Test Protein as Generated by Percent Antigenicity and Percent Stimulation (Percentage Stimulation above Background ≥0.5%, SEM=2). **C)** Graph showing RI Values Calculated for each Test Protein (Percentage Stimulation above Background ≥0.5%, SEM=2).

**Figure 9. *In vitro* metabolic stability in blood.**
Table showing half life in fresh blood. Samples were incubated at the indicated concentrations in fresh blood at 37 °C. The concentrations of agents were determined with LC-MS/MS analysis at 0, 5, 15, 30, and 60 min and used to calculate the rate of disappearance and half life.

**Figure 10. Pharmacokinetic profile and parameter for peptides after intravenous administration.**
**A)** Mouse PK data (10 mg/kg, bolus IV, n=3 per timepoint). **B)** Rat PK data (10 mg/kg, bolus IV, n=3 per timepoint). **C**) Non-human primate (NHP) PK data (4 mg/kg, 30 min IV infusion, n=3). Error bars represent the standard deviation (SD).

***Detailed Description of the Invention***

Aspects and embodiments of the present invention will now be discussed with reference to the accompanying figures. Further aspects and embodiments will be apparent to those skilled in the art. All documents mentioned in this text are incorporated herein by reference.

Pharmacological activation of the STimulator of INterferon Genes (STING) pathway is an emerging strategy for stimulating both the innate and adaptive arms of the immune system to trigger an anti-tumour immune response in tumours that do not trigger a strong immune response (known as "cold" tumours). Although the first generation of STING agonists dosed intratumourally demonstrated a strong anti-tumour response in preclinical models, positive clinical data has so far been limited in metastatic patients, potentially due to reduced efficacy in non-injected tumours. Newer classes of STING agonists that are delivered systemically are expected to deliver more consistent and predictable levels of STING activation but may not be as well tolerated because of off-target activation of the immune system resulting in immune related adverse events.

It has previously been demonstrated that IFI16 promotes activation of the cGAMP-activated STING-TBK1 pathway by a process relying on IFI16 pyrin domain-dependent protein-protein interactions (Jønsson et al., 2017). The present inventors have identified a peptide-based therapeutic (e.g., agent) comprising an IFI16 pyrin domain-derived amino acid sequence as described herein. The amino acid sequence may be conjugated to a cell penetrating peptide. The agent as described herein is capable of effecting the agonist-dependent activation of STING pathway signalling by IF116.

The inventors have identified an agent that is capable of acting as a STING sensitiser for effective and safe induction of immune responses (such as anti-tumour immune responses) only at sites where STING agonists are present. Without wishing to be bound by theory, the agent of the present invention is anticipated to reduce the threshold necessary for STING pathway activation. The agent is believed to enhance STING activation and the duration of activation. It is also believed that the agent of the invention is capable of locally inducing supraphysiological levels of pro-inflammatory cytokines/chemokines in the presence of a STING agonist. This effect is particularly beneficial in treating patients with cancer.

The inventors have found that *in vitro*, the agent of the invention sensitises the STING pathway to pathway activators (e.g., cytoplasmic DNA and cGAMP) and pharmacological STING agonists including ADU-S100 and diABZI compound 3, reducing the $EC_{50}$ values of agonist-induced STING pathway activation while inducing supraphysiological (i.e., increasing the $E_{max}$) levels of pro-inflammatory cytokines/chemokines (e.g., Type I interferon and CXCL10) in THP1 cells and primary human immune cells. It is believed that the agent of the invention sensitises STING to a STING agonist (e.g., cGAMP) by accelerating and increasing STING translocation from the endoplasmic reticulum (ER) to the Golgi, resulting in longer activation of downstream STING pathway components, as determined by the phosphorylation kinetics of TBK1 and IRF3, as well as STING itself. The agent therefore induces rapid and high-level trafficking to the Golgi compared to a STING agonist alone.

It has also been found that the agent can be administered parenterally without evidence of peripheral STING activation. *In vivo* experiments in immunocompetent syngeneic mouse models demonstrate that the agent dosed intravenously was well tolerated and was associated with anti-tumour efficacy when used as a single agent or in combination with a pharmacological STING agonist at doses that did not cause the immune cell death that typically results from overdosing this type of agent. In addition, the agent dosed intravenously was well tolerated and was associated with anti-tumour efficacy in combination with immune checkpoint blockers such as anti-PD1.

The present application therefore provides a STING pathway sensitiser that enables safe systemic administration while giving rise to enhanced STING pathway activation only at sites at which STING agonists are present. The agent may therefore be beneficial in the treatment of disorders which can be treated or ameliorated by enhancing STING activity.

*Peptide-based therapeutic*

The agent of the invention is derived from the pyrin domain of Interferon-gamma-inducible protein 16 (IFI16). IFI16 is a cytosolic and nuclear protein also known as interferon-inducible myeloid differentiation transcriptional activator. In humans, IFI16 is encoded by *IFI16.* IFI16 comprises four domains including a pyrin-domain, 2 HIN domains (HIN-A and HIN-B) and a BFP domain.

The inventors have surprisingly found that an agent derived from the pyrin domain of !F!16 is capable of sensitising STING activity. Sensitisation of STING activity involves the amplification, prolongation and augmentation of STING pathway activation such that when a STING stimulating agent is present STING activation is accelerated and/or enhanced. In particular, sensitising STING activity decreases the threshold required for STING stimulating agent-dependent expression of cytokines and/or chemokines (e.g., decreases the $EC_{50}$ of STING stimulating agent-dependent expression of cytokines and/or chemokines).

Accordingly, an aspect of the invention provides an agent comprising an amino acid sequence, where the amino acid sequence comprises KKAKNIVLLKGLEVINDWHF (SEQ ID NO: 1). In some embodiments, the amino acid sequence consists of KKAKNIVLLKGLEVINDWHF (SEQ ID NO: 1). In some embodiments, the agent is a peptide. The peptide may be 20 amino acids in length to 50 amino acids in length. The peptide may consist of KKAKNIVLLKGLEVINDWHF (SEQ ID NO: 1).

SEQ ID NO: 1 is based on a 20 amino acid sequence derived from the pyrin-domain of IFI16. However, SEQ ID NO: 1 differs to the wild-type sequence of the same length (KKYKNIVLLKGLEVINDYHF, SEQ ID NO: 4) in that it comprises alanine at position 3 and tryptophan at position 18.

Another aspect of the invention provides a composition comprising the agent described herein.

The agent has been shown to induce rapid and high-level trafficking of STING to the Golgi in the presence of a STING stimulating agent compared to a STING stimulating agent alone. Without wishing to be bound by theory, it is believed that trafficking of STING occurs during STING activation. In some embodiments, the agent increases trafficking of STING from the endoplasmic reticulum (ER) to the Golgi in a cell comprising the agent compared to an equivalent cell not comprising the agent, where both cells are exposed to or comprise a STING stimulating agent. The levels of STING at the Golgi may therefore be increased compared to the ER in a cell comprising the agent compared to an equivalent cell not comprising the agent, where both cells are exposed to or comprise a STING stimulating agent. The increase in STING levels at the Golgi may be least 10%, 20%, 30%, 40%, 50%, 60%, 70%, 80%, 90%, 100%, 200%, or 500% higher than the level found in cells treated with a STING stimulating agent only. The present inventors have observed that STING trafficking from the ER to the Golgi is accelerated in the presence of the agent of the invention and a STING stimulating agent. Thus, in some embodiments, the agent accelerates trafficking of STING from the ER to the Golgi in a cell comprising the agent compared to an equivalent cell not comprising the agent, where both cells are exposed to or comprise a STING stimulating agent. The trafficking of STING may be measured by using an imaging flow cytometer (e.g., ImageStream® which combines the speed, sensitivity, and phenotyping abilities of flow cytometry with the detailed imagery and functional insights of microscopy) to track the colocalization of STING with either ER- or Golgi-specific markers in individual cells. Alternatively, this effect could be measured by confocal microscopy coupled with immunofluorescence. This may be carried out by culturing cell (such as THP-1 cells), treating them with the agent alone, agent and STING stimulating agent, or STING stimulating agent alone. The cells may be treated for 10 mins, 30 mins, 60 mins or 120 mins. The treated cells may then be analysed using an imaging flow cytometer to track the colocalization of STING with either ER- or Golgi-specific fluorescent markers.

The present inventors have found that an agent according to the invention is capable of sensitising the STING pathway to STING stimulating agents. It is believed this is achieved by reducing the threshold required for STING activation. The agent is also believed to enhance STING activation and/or the duration of activation in a cell exposed to or comprising a STING stimulating agent.

STING pathway activation may be determined by measuring:

(i) STING phosphorylation (phosphorylation of Ser366 of STING);
(ii) phosphorylation of TBK1;
(iii) expression of type I IFN or inflammatory cytokines;
(iv) activation of IFN-β promoter activity; and/or
(v) activation of NF-kB, IRF3 and/or other signalling pathways/signalling components acting downstream of STING

activation.

Thus, enhanced (e.g., increased or amplified) STING activation is associated with increased STING phosphorylation, increased expression of type I IFN or inflammatory cytokines such as CXCL10, increased activation of IFNβ promoter activity, and/or increased activation of NF-kB, IRF3 and/or other signalling pathways in a cell treated with the agent compared to an equivalent cell not comprising the agent. The effect of the agent can therefore be measured by determining any one of (i) to (v) in a cell comprising the agent of the invention compared to a cell not comprising the agent, where both cells are exposed to or comprise a STING stimulating agent.

The enhanced duration (e.g., the time span) of activation can be measured by measuring the duration of any one of (i) to (v) in a cell comprising the agent compared to a cell not comprising the agent, where both cells are exposed to or comprise a STING stimulating agent. The duration of activation may be measured over a 1 hr, 2 hr, 3 hr, 6 hr, 12 hr, 24 hr or 48 hr period.

The agent of the invention is capable of inducing STING phosphorylation in the presence of a STING stimulating agent. During activation STING is phosphorylated at serine 366. In some embodiments, the level of phosphorylated STING is increased in a cell comprising the agent compared to an equivalent cell not comprising the agent, where both cells are exposed to or comprise a STING stimulating agent. In some embodiments, the level of phosphorylated STING at Ser366 is increased in a cell comprising the agent compared to an equivalent cell not comprising the agent, where both cells are exposed to or comprise a STING stimulating agent. STING Ser366 phosphorylation may be detected using western blotting, ELISA, or any other suitable method. The increase in Ser366 phosphorylation may be at least 2-fold, 3-fold, 4-fold, 5-fold, 10-fold, 15-fold, 20-fold, 50-fold, or 100-fold higher than a cell not comprising the agent but which has been exposed to or comprises a STING stimulating agent. The increase in Ser 366 phosphorylation may be least 10%, 20%, 30%, 40%, 50%, 60%, 70%, 80%, 90%, 100%, 200%, or 500% higher than the level found in cells treated with a STING stimulating agent only. The present inventors have also observed that phosphorylation of STING is accelerated in cells treated with the agent compared to an equivalent cell not treated with the agent, where both cells are also treated with a STING stimulating agent. Acceleration of phosphorylation may be measured by measuring the levels of phosphorylated STING at certain time points (e.g., 30 mins, 60 mins, 120 mins and 360 mins) using, for example, western blot and determining the time points at which STING is phosphorylated in cells treated with the agent compared to untreated cells, where both cells are also treated with a STING stimulating agent. When determining the levels of phosphorylated STING, an anti-phospho-STING antibody may be used (e,g., an anti-STING-phosphoSer366 antibody).

The agent of the invention is capable of inducing phosphorylation of TANK-binding kinase 1 (TBK1) in the presence of a STING stimulating agent. During STING activation (with a STING stimulating agent), human TBK1 is phosphorylated at serine 172. In some embodiments, the level of phosphorylated TBK1 is increased in a cell comprising the agent compared to an equivalent cell not comprising the agent, where both cells are exposed to or comprise a STING stimulating agent. In some embodiments, the level of phosphorylated TBK1 at Ser172 is increased in a cell comprising the agent compared to an equivalent cell not comprising the agent, where both cells are exposed to or comprise a STING stimulating agent. TBK1 Ser172 phosphorylation may be detected using western blotting, ELISA, or any other suitable method. The increase in Ser172 phosphorylation may be at least 2-fold, 3-fold, 4-fold, 5-fold, 10-fold, 15-fold, 20-fold, 50-fold, or 100-fold higher than a cell treated with a STING stimulating agent only. The increase in Ser172 phosphorylation may be least 10%, 20%, 30%, 40%, 50%, 60%, 70%, 80%, 90%, 100%, 200%, or 500% higher than the level found in cells treated with a STING stimulating agent only.

The present inventors have also observed that phosphorylation of TBK1 is accelerated in cells treated with the agent compared to an equivalent cell not treated with the agent, where both cells are also treated with a STING stimulating agent. Acceleration of phosphorylation may be measured by measuring the levels of phosphorylated TBK1 at certain time points (e.g., 30 mins, 60 mins, 120 mins and 360 mins) using, for example, western blot and determining the time points at which TBK1 is phosphorylated in cells treated with the agent compared to untreated cells, where both cells are exposed to or comprise a STING stimulating agent. When determining the levels of phosphorylated TBK1, an anti-phospho-TBK1 antibody may be used (e.g., an anti-TBK1-phosphoSer172 antibody).

In some embodiments, the cell is an immune cell. The cell may be a macrophage or monocyte. The cell may be a cancer cell or a cell infected with a virus (e.g., a DNA virus). The cell may be a cancer cell treated with cancer therapies such as chemotherapies, radiation therapies or small molecules targeted therapies that increase the amount of cytoplasmic DNA and therefore activates the STING pathway. The immune cell may be a lymphocyte, a macrophage, a dendritic cell, a leukocyte, a monocyte, a neutrophil, or an eosinophil.

The agent has been shown to induce production of proinflammatory cytokines (e.g., Type I IFN) and chemokines (e.g., CXCL10) in the presence of a STING stimulating agent. In particular, production of cytokines and/or chemokines may be supraphysiological (e.g., production of the cytokines and/or chemokines is much higher than normally found in the human body).

Type I interferons (IFN) are cytokines which play roles in inflammation, immunoregulation, tumour cell recognition, and T cell responses. The Type I IFN family is a multi-gene cytokine family comprising IFN-α, IFN-β, IFNε, IFNτ,

IFNκ, IFNω, IFNδ and IFNζ. IFN-α and IFN-β are the most broadly expressed type I IFNs. IFN-α and IFN-β are secreted by many cell types including lymphocytes (NK cells, B cells and T cells), macrophages, plasmacytoid dendritic cells, leukocytes, fibroblasts, endothelial cells, osteoblasts and others. They stimulate both macrophages and NK cells to elicit an anti-viral response and have also been shown to be active against tumours. IFN-β has been shown to inhibit immune cell production of growth factors, thereby slowing tumour growth. It has also been shown to inhibit other cells from producing vessel producing growth factors, thereby blocking tumour angiogenesis and hindering the tumour from connecting into the blood vessel system. The type I IFNs bind to a specific cell surface receptor complex known as the IFN-α receptor.

C-X-C motif chemokine ligand 10 (CXCL10) also known as Interferon gamma-induced protein 10 (IP-10) or small-inducible cytokine B10 is a small cytokine belonging to the CXC chemokine family. The CXCL10 protein in humans is encoded by the *CXCL10* gene. CXCL10 is thought to be secreted by several cell types, including monocytes, macrophages, neutrophils, eosinophils, endothelial cells and fibroblasts, in response to IFN-γ. CXCL10 is an important cytokine produced downstream of type I IFN after STING activation and is commonly used to measure STING activity in human cancer models (Cañadas et al.,2018 and Falahat et al., 2021). It is believed that CXCL10 is involved in (i) chemoattraction for monocytes/macrophages, T cells, NK cells, and dendritic cells, (ii) promotion of T cell adhesion to endothelial cells, (iii) antitumour activity, and (iv) inhibition of bone marrow colony formation and angiogenesis. CXCL10 elicits these effects by binding to the cell surface chemokine receptor CXCR3.

Expression of cytokines and/or chemokines may be increased in a cell comprising the agent compared to an equivalent cell not comprising the agent, where both cells are exposed to or comprise a STING stimulating agent. In some embodiments, expression of type 1 IFN is increased in a cell comprising the agent compared to an equivalent cell not comprising the agent, where both cells are exposed to or comprise a STING stimulating agent. The type I IFN may be IFN-α or IFN-β. In some embodiments, expression of CXCL10 is increased in a cell comprising the agent compared to an equivalent cell not comprising the agent, where both cells are exposed to or comprise a STING stimulating agent. Expression of the cytokines and/or chemokines (e.g., type 1 IFN and/or CXCL10) in a cell comprising the agent of the invention may be at least 2-fold, 3-fold, 4-fold, 5-fold, 10-fold, 15-fold, 20-fold, 50-fold, or 100-fold higher than a cell not comprising the agent, where both cells are exposed to or comprise a STING stimulating agent. Expression of the cytokines and/or chemokines may be at least 10%, 20%, 30%, 40%, 50%, 60%, 70%, 80%, 90%, 100%, 200%, or 500% higher than the level found in cells treated with a STING stimulating agent only.

The agent may decrease the threshold required for STING stimulating agent-dependent expression (e.g., STING agonist-dependent expression) of cytokines and/or chemokines. The agent may decrease the $EC_{50}$ of STING stimulating agent-dependent expression of cytokines and/or chemokines in a cell comprising the agent compared to an equivalent cell not comprising the agent, where both cells are exposed to or comprise a STING stimulating agent. Half maximal effective concentration, known as $EC_{50}$, is a measure of the concentration of a drug/agent which induces a response halfway between the baseline and maximum after a specified exposure time. It can be a measure of the decrease in the threshold required for STING stimulating agent-dependent expression of the cytokines and/or chemokines when using the agent of the invention. In some embodiments, the agent decreases the $EC_{50}$ of STING stimulating-dependent expression of type I IFNs (e.g., IFN-β) and/or CXCL10 in a cell comprising the agent compared to an equivalent cell not comprising the agent, where both cells are exposed to or comprise a STING stimulating agent. The $EC_{50}$ may be at least 5%, 10%, 15%, 20%, 25%, 30%, 35%, 40%, 45%, 50%, 55%, 60%, 65%, 70%, 75%, 80%, 85%, 90% or 95% lower than the $EC_{50}$ of cells treated with a STING stimulating agent only.

The agent described herein may enhance the STING stimulating agent-dependent expression (e.g., STING agonist-dependent expression) of cytokines and/or chemokines. The agent may increase the $E_{max}$ of STING stimulating agent-dependent expression of cytokines and/or chemokines in a cell comprising the agent compared to an equivalent cell not comprising the agent, where both cells are exposed to or comprise a STING stimulating agent. $E_{max}$ is the maximal effect of a drug/agent, and so it can measure the increase in the STING stimulating agent-dependent expression of cytokines and/or chemokines when using the agent of the invention (e.g., the supraphysiological increase). In some embodiments, the agent increases the $E_{max}$ of STING stimulating agent-dependent expression of type I IFNs (e.g., IFN-β) and/or CXCL10 in a cell comprising the agent compared to an equivalent cell not comprising the agent, where both cells are exposed to or comprise a STING stimulating agent. The $E_{max}$ of STING stimulating agent-dependent expression of cytokines and/or chemokines in a cell comprising the agent of the invention may be at least 2-fold, 3-fold, 4-fold, 5-fold, 10-fold, 15-fold, 20-fold, 50-fold, or 100-fold higher than a cell not comprising the agent, where both cells are exposed to or comprise a STING stimulating agent. The $E_{max}$ of the cytokines and/or chemokines may be at least 10%, 20%, 30%, 40%, 50%, 60%, 70%, 80%, 90%, 100%, 200%, or 500% higher than the $E_{max}$ of cells treated with a STING stimulating agent only.

In some embodiments, the chemokine is CXCL10. In some embodiments, the cytokine is type 1 IFN. The type 1 IFN may be IFN-α or IFN-β.

Expression of cytokines and/or chemokines may be in an immune cell, such as lymphocyte, macrophage, dendritic cell, leukocytes, monocyte, neutrophil, or eosinophil. In some embodiments, the cell is an immune cell. In some

embodiments, the cell is a lymphocyte, macrophage, dendritic cell, leukocytes, monocyte, neutrophil, or eosinophil. In some embodiments, the cell is a macrophage or monocyte.

mRNA and protein expression of the cytokines and/or chemokines may be measured using standard techniques known in the art, such as ELISA, Quanterix SIMOA, Luminex, western blot or through mRNA analysis such as RT-PCR, or via the use of reporter constructs. Expression of cytokines/chemokines may be measured at particular time points, for example, at least 1 hr, 2 hrs, 3 hrs, 4 hrs, 5 hrs, 6 hrs, 7 hrs, 8 hrs, 9 hrs, 10 hrs, 11 hrs, 12 hrs, 13 hrs, 14 hrs, 15 hrs, 16 hrs, 17 hrs, 18 hrs, 19 hrs, 20 hrs, 21 hrs, 22 hrs, 23 hrs, or 24 hrs after contacting a cell with the agent described herein, where the cell is exposed to or comprises a STING stimulating agent. The time points may be 6 hrs or 20 hrs after contacting a cell with the agent, where the cell is exposed to or comprises a STING stimulating agent. In an example, protein expression of CXCL10 and/or IFN-β may be measured by ELISA. In this example, cells, such as THP1 cells, may be cultured and treated with a STING stimulating agent for 30 mins following sensitization with or without the agent of the invention. In another example, cells, such as THP1 cells, may be cultured and treated with the agent of the invention for 30 mins or left untreated following treatment with a STING stimulating agent. In an alternative example, the cells (e.g., THP1 cells) are treated either with (i) both the agent and a STING stimulating agent or (ii) a STING stimulating agent alone.

In some embodiments, STING pathway activation may be determined by measuring activation of the IFN-β. Activation of said promoter may be determined in recombinant cells comprising a nucleic acid construct encoding a reporter protein under the control of the IFN-β promoter. IFN-β promoter may also be determined in cell free expression systems allowing expression of a reporter protein under the control of the IFN-β promoter. In some embodiments, agent increases expression of the reporter protein in a cell treated with the agent and a STING stimulating agent at least 10%, 20%, 30%, 40%, 50%, 60%, 70%, 80%, 90%, 100%, 200%, or 500% higher than the level found in cells treated with a STING stimulating agent only. Expression of the reporter protein may be at least 2-fold, 3-fold, 4-fold, 5-fold, 10-fold, 15-fold, 20-fold, 50-fold, or 100-fold higher in cell comprising both the agent and a STING stimulating agent than a cell only comprising the STING stimulating agent. Expression of the reporter protein may be measured by western blot.

In some embodiments, STING pathway activation may be determined by measuring activation of NF-kB, IRF3 and/or other signalling components acting downstream of STING activation, particularly NF-kB and IRF3 signalling pathways. A non-limiting example of a method which may be used to measure NF-κB activity at the transcriptional activation level is to use a gene reporter assay, which for example introduces an exogenous NF-κB consensus promoter sequence linked to reporter gene, such as luciferase. In some embodiments, agent increases expression of the reporter protein at least 10%, 20%, 30%, 40%, 50%, 60%, 70%, 80%, 90%, 100%, 200%, or 500% higher in cells treated with both the agent of the invention and a STING stimulating agent than the level found in cells treated with a STING stimulating agent only. Expression of the reporter protein may be at least 2-fold, 3-fold, 4-fold, 5-fold, 10-fold, 15-fold, 20-fold, 50-fold, or 100-fold higher in cell comprising both the agent and a STING stimulating agent than a cell comprising the STING stimulating agent only. IRF3 pathway activation may be measured by measuring the level of phospho-IRF3 in a cell or by assessing the translocation from cytoplasm to the cell nucleus of IRF3. In some embodiments, the level of phosphorylated IRF3 is increased in a cell comprising the agent compared to an equivalent cell not comprising the agent, where both cells are exposed to or comprise a STING stimulating agent. The increase in phospho-IRF3 may be at least 2-fold, 3-fold, 4-fold, 5-fold, 10-fold, 15-fold, 20-fold, 50-fold, or 100-fold higher in cell comprising both the agent of the invention and a STING stimulating agent than a cell comprising a STING stimulating agent only. The increase in phosphoIRF3 may be least 10%, 20%, 30%, 40%, 50%, 60%, 70%, 80%, 90%, 100%, 200%, or 500% higher than the level found in cells treated with the STING stimulating agent only. Expression of the reporter protein and/or phosphor-IRF3 may be measured by western blot.

The effect on the cell comprising the agent and the equivalent cell not comprising the agent may be determined in the presence of a STING stimulating agent. In some embodiments, both the cell comprising the agent and the equivalent cell not comprising the agent may comprise a STING stimulating agent. The level of STING stimulating agent in both cells may be the same. The STING stimulating agent may be at a level sufficient to activate the STING signalling pathway. In some embodiments, the STING stimulating agent is endogenous to the cells. In some embodiments, the source STING stimulating agent is exogenous to the cells. The STING stimulating agent may be cGAMP. By "exposed to" it is meant that a cell is contacted with the STING stimulating agent for period sufficient to induce STING pathway activation (for example, 30 mins). The exposure to the STING stimulating agent may be transient, e.g., typically for enough time that the exposure of the STING stimulating agent overlaps with the treatment of the agent of the invention.

The agent described herein may have reduced immunogenic potential compared to a reference agent. The reference agent may comprise an amino acid sequence, where the amino acid sequence comprises or consists of KKYKNIVLLKGLEVINDYHF, SEQ ID NO: 4. The reference agent may further comprise one or more additional moieties as described herein. The immunogenic potential of the agent may be at least 5%, 10%, 15%, 20%, 25%, 30%, 35%, 40%, 45%, 50%, 55%, 60%, 65%, 70%, 75%, 80%, 85%, 90%, 95% or 100% lower than the immunogenic potential of the reference agent. The term "immunogenic potential" as used herein refers to the ability of an agent (e.g., polypeptide) to elicit an immune response against the agent. The immunogenic potential may be determined *in silico*, for example

by determining the number of epitopes within a given sequence (e.g., amino acid sequence). An example of a suitable *in silico* model is the EpiMatrix system. The immunogenic potential may be determined *in vitro*, for example by using the Dendritic Cell - T cell (DC-T) proliferation assay (Pro!mmune Ltd) as described herein. The *in vitro* determination of the immunogenic potential may be carried out by loading dendritic cells (DCs) with the agent and the reference agent. The loaded DC cells may then be co-cultured with a fluorescent label. The cells may be co-cultured for 7 days. Proliferation of T cells (and therefore an immune response) may be measured by measuring a decrease in intensity of the fluorescent label associated with the T cell. This may be determined using flow cytometry. In some embodiments, the fluorescent intensity for the agent of the invention is at least 5%, 10%, 15%, 20%, 25%, 30%, 35%, 40%, 45%, 50%, 55%, 60%, 65%, 70%, 75%, 80%, 85%, 90%, 95% or 100% lower than the fluorescent intensity for the reference agent.

*Additional moieties*

The agent of the invention may further comprise one or more additional moieties (e.g., heterologous moieties). The agent of the invention may comprise two additional moieties (e.g., one at each terminus of the amino acid sequence).

The additional moiety may be conjugated to the N-terminus of the amino acid sequence, or the additional moiety may be conjugated to the C-terminus of the amino acid sequence. The additional moiety may be linked directly or indirectly via a linker.

The additional moiety may be peptide or polypeptide, a carbohydrate molecule, a polymer (e.g., a synthetic polymer), or a lipid. Where the additional moiety is a peptide or polypeptide, it may be defined as a non-IFI16 peptide sequence.

The additional moiety may improve the physical and pharmacokinetic properties of the peptide, such as its solubility, stability, transit across a plasma membrane, or *in vivo* half-life. The additional moiety may be a compound that masks the peptide from the host immune system, such as polyethylene glycol (PEG) or a modified PEG, such as NPEG. In some embodiments, the additional moiety is a membrane transit moiety or a half-life extending moiety.

*Membrane transit moiety:*

The agent may comprise an additional moiety that facilitates membrane transit of the agent. The membrane transit moiety may be conjugated to the C-terminus of the amino acid sequence. Alternatively, the membrane transit moiety may be conjugated to the N-terminus of the amino acid sequence. The membrane transit moiety may also be conjugated to the amino acid sequence via an amino acid side chain. The membrane transit moiety may be conjugated to the amino acid sequence either directly or indirectly via a linker.

In some embodiments, the additional moiety is a cell penetrating peptide (CPP). A CPP is a short peptide (typically 5 to 30 amino acids in length) that facilitates cellular intake/uptake of another peptide or polypeptide. CPPs typically have an amino acid composition that either contains a high abundance of positively charged amino acids such as lysine or arginine (polycationic) or have sequences that contain an alternating pattern of polar/charged amino acids and non-polar, hydrophobic amino acids (amphipathic). Alternatively, the CPP may be a hydrophobic peptide, containing only apolar residues, with low net charge or have hydrophobic amino acid groups that facilitate cellular uptake.

CPPs can mediate cell penetrating through different pathways, such as by direct penetration, endocytosis-mediated translocation, or translocation through the formation of a transitory structure (e.g., inverted micelles).

The CPP may be conjugated to the N-terminus of the amino acid sequence or conjugated to the C-terminus of the amino acid sequence. In some embodiments, the CPP is conjugated to the C-terminus of the amino acid sequence.

A non-limiting list of example CPPs known in the art are shown in Table 1 below (See for example, Xie at al., Front. Pharmacol., 20 May 2020, Sec. Experimental Pharmacology and Drug Discovery, Derakhshankhah and Jafari, Biomedicine & Pharmacotherapy 108 (2018) 1090-1096, and Bottens & Yamada. Cell-Penetrating Peptides (CPPs) as Therapeutic and Diagnostic Agents for Cancer. Cancers 2022, 14, 5546).

**Table 1: Example CPPs**

| Peptide | Sequence | SEQ ID NO: |
|---|---|---|
| Tat (49-57) | RKKRRQRRR | 5 |
| Tat (48-57) | GRKKRRQRRR | 6 |
| Poly-arginine, R8 | RRRRRRRR | 7 |
| DPV3 | RKKRRESRKKRRES | 8 |
| DPV6 | GRPRESGKKRKRKRLKP | 9 |

(continued)

| Peptide | Sequence | SEQ ID NO: |
|---|---|---|
| Penetratin | RQIKIWFQNRRMKWKK | 10 |
| R9-TAT | GRRRRRRRRRPPQ | 11 |
| pVEC | LLIILRRRIRKQAHAHSK | 12 |
| ARF (19-31) | RVRVFVVHIPRLT | 13 |
| MPG | GALFLGFLGAAGSTMGAWSQPKKKRKV | 14 |
| MAP | KLALKLALKALKAALKLA | 15 |
| Transportan | GWTLNSAGYLLGKINLKALAALAKKIL | 16 |
| Bip4 | VSALK | 17 |
| C105Y | CSIPPEVKFNPFVYLI | 18 |
| Melittin | GIGAVLKVLTTGLPALISWKRKRQQ | 19 |
| gH625 | HGLASTLTRWAHYNALIRAF | 20 |
| Pept 1 (TP1) | PLILLRLLRGQF | 21 |
| Pept 2 (TP2) | PLIYLRLLRGQF | 22 |
| IW-14 | KLWMRWYSPTTRRYG | 23 |
| HRSV | RRIPNRRPRR | 24 |
| Tat (48-60) | GRKKRRQRRRPPQ | 25 |
| Maurocalcine | GDCLPHLKLCKENKDCCSKKCKRRGTNIEKRCR | 26 |
| Pep=1 | KETWWETWWTEWSQPKKKRKV | 27 |
| CADY | GLWRALWRLLRSLWRLLWRA | 28 |
| P28 | LSTAADMQGWTDGMASGLDKDYLKPDD | 29 |

In some embodiments, the CPP is any one of the CPPs listed in Table 1. Fragments or variants of CPPs which retain cell penetrating activity may also be used. This includes CPPs which have one or more substitutions compared to the sequence shown. Substitutions with D-equivalents of the original amino acids may be desirable. D-equivalents of the amino acid may be denoted by a lower case letter (e.g., "r" = D-Arg).

Cyclic CPPs may be indicated by the notation "cyclo" or "c". They may have increased stability *in vivo* (e.g., in plasma) when compared to the corresponding linear CPP. It has also been reported that cyclisation may enhance membrane transport (Qian et al. 2016). Cyclisation may be achieved by any appropriate means, such as side chain cyclisation or head-to-tail cyclisation. In some embodiments, the CPP is a cyclic CPP.

The cyclic CPP may be:

- cyclo[GRKKRRQRRR] (SEQ ID NO: 30), which is backbone-cyclised ("head-to-tail");
- cyclo[Grkkrrqrrr] (SEQ ID NO: 31), which is backbone-cyclised ("head-to-tail");
- cyclo[KrRrGrKkRrE] (SEQ ID NO: 32; known as cyclic TAT, as described in Lätting-Tünnemann et al., 2011 and Nischan et al. 2015), which is cyclised via a lactam bridge formed between the terminal lysine and glutamic acid side chains; or
- cyclo[CrRrGrKkRrC] (SEQ ID NO: 2), where the lower case letter are D-amino acids and where the CPP is cyclised via a disulphide bond between the terminal cysteines.

In some embodiments, the CPP is cyclo[CrRrGrKkRrC] (SEQ ID NO: 2). In some embodiments, cyclo[CrRrGrKkRrC] (SEQ ID NO: 2) is conjugated to the C-terminus of the amino acid sequence. In some embodiments, the agent is KKAKNIVLLKGLEVINDWHF-cyclo[CrRrGrKkRrC] (SEQ ID NO: 33), where the lower case letter are D-amino acids and where the CPP is cyclised via a disulphide bond between the terminal cysteines.

*Half-life extending moiety:*

Additionally, or alternatively, the agent of the invention may further comprise an additional moiety that increases the stability of the agent. The additional moiety may increase the *in vivo* half-life of the agent.

The half-life extending moiety may be conjugated to the N-terminus of the amino acid sequence. Alternatively, the half-life extending moiety may be conjugated to the C-terminus of the amino acid sequence. Where the agent already comprises a membrane transit moiety, the half-life extending moiety is typically conjugated to a terminus of the amino acid sequence which is not already conjugated to the membrane transit moiety. The half-life extending moiety may also be conjugated to the amino acid sequence via an amino acid side chain. The half-life extending moiety may be conjugated to the amino acid sequence either directly or indirectly via a linker.

In some embodiments, the half-life extending moiety is a peptide or polypeptide. The half-life extending moiety may be at least 20, 50, 100, 200, 300, 400 or 500 amino acids in length. The half-life extending moiety may be an Fc fragment, serum albumin (e.g., human serum albumin (HSA)), fibrinogen, glutathione S-transferase, transferrin, or streptavidin.

In some embodiments, the half-life extending moiety is a carbohydrate molecule. For example, dextran, glycosylation, polysialylation, hydroxyethylation (HESylation), heparosanylation (HEPylation), and hyaluronic acid polysaccharide (HAylation).

In other embodiments, the half-life extending moiety is a synthetic polymer, for example polyethylene glycol (PEG) or modified PEG. Example polypeptides polymers include XTEN (protein polymer developed by Amunix), PASylation (proline-alanine-serine polymer), ELPylation (elastin-like polypeptides), HAPylation (repeated sequence of a glycine rich - Gly4Ser)n polypeptide, gelatin-like protein (GLK - has a (Gly-XY)n structure).

In some embodiments, the moiety is or comprises a lipophilic substituent. Without wishing to be bound by theory, it is thought that lipophilic substituents such as fatty acid derivatives (and some other types of half-life extending moiety) reversibly bind albumin in the body, thus protecting the agent from renal filtration as well as enzymatic degradation thereby enhancing the half-life of the agent *in vivo.* In some embodiments, the half-life extending moiety comprises a fatty acid derivative. The fatty acid derivative may have the formula:

$$HO\text{-}CO\text{-}(CH_2)_x\text{-}CO\text{-}*,$$

where x is an integer in the range of 12 to 18 and * is the attachment point to the amino acid sequence comprising SEQ ID NO: 1.

The fatty acid derivative as described herein may further comprise a hydrophilic linker connecting the fatty acid derivative to the amino acid sequence comprising SEQ ID NO: 1. The hydrophilic linker may comprise or consists mainly of ethyleneglycol ether and/or propylenegycol ether and/or natural amino acids and/or unnatural amino acids. The length of the hydrophilic linker may be up to 60 atoms.

The half-life extending moiety may be represented by the formula:

$$HO\text{-}CO\text{-}(CH_2)_x\text{-}CO\text{-}(\gamma Glu)_y\text{-}(Adoa)_z\text{-}*,$$

where x is an integer in the range of 12 to 18, optionally where x is 14, 15, 16, 17, or 18,
y is an integer in the range of 0 to 2, optionally where y is 0 or 1,
z is an integer in the range of 1 to 5, optionally where z is 2, 3, or 4.

The half-life extending moiety may be selected from:

19-carboxynonoadecanoyl-Adoa-Adoa-*,
19-carboxynonoadecanoyl-γGlu-Adoa-Adoa-*,
17-carboxyheptadecanoyl-Adoa-Adoa *,
17-carboxyheptaadecanoyl-γGlu-Adoa-Adoa-*,
15-carboxypentadecanoyl-Adoa-Adoa-*, or
15-carboxypentadecanoyl-yGlu-Adoa-Adoa-*.

In some embodiments, the half-life extending moiety is 15-carboxypentadecanoyl-Adoa-Adoa.

As described herein "Adoa" denotes 8-amino-3,6-dioxaoctanoic acid. "γGlu" denotes glutamic acid where the gamma-carboxy group of glutamic acid is linked to the amino group of the neighbouring residue (e.g., of the linker or peptide). The linker may comprise "Adoa" and/or "γGlu". The neighbouring residue may be "Adoa" or "γGlu". In embodiments, without a linker, the gamma-carboxy group of glutamic acid may be linked to the amino group of the amino acid

sequence. "*" is the attachment point to the amino acid sequence comprising SEQ ID NO: 1. The attachment point to the amino acid sequence ("*") may be at the amino group of the amino acid sequence. The amino group of the amino acid sequence may be the amino group of the N-terminal amino acid, such as lysine.

In some embodiments, the agent comprises both a half-life extending moiety and a membrane transit moiety. The half-life extending moiety may be conjugated to the N-terminus of the amino acid sequence and the membrane transit moiety may be conjugated to the C-terminus of the amino acid sequence.

In some embodiments, the agent of the invention has the formula:

X-Y-Z

where

X is a half-life extending moiety as described herein;
Y is the amino acid sequence comprising SEQ ID NO: 1; and
Z is a membrane transit moiety as described herein.

In some embodiments, the amino acid sequence consists of SEQ ID NO: 1. The half-life extending moiety may be or comprise a lipophilic substituent. The membrane transit moiety may be a CPP. In some embodiments, the CPP is a cyclic CPP.

In some embodiments, the agent of the invention is selected from:

15-carboxypentadecanoyl-Adoa-Adoa-KKAKNIVLLKGLEVINDWHF-c[CrRrGrKkRrC]-$NH_2$ (SEQ ID NO: 3); and
15-carboxypentadecanoyl-Adoa-Adoa-KKAKNIVLLKGLEVINDWHF-c[CrRrGrKkRrC]-OH (SEQ ID NO: 34);
or any pharmaceutically acceptable salt, amide, ester or prodrug thereof.

The agent of the invention may have the structure (15-carboxypentadecanoyl-Adoa-Adoa-KKAKNIVLLK-GLEVINDWHF-c[CrRrGrKkRrC]-$NH_2$ (SEQ ID NO: 3)):

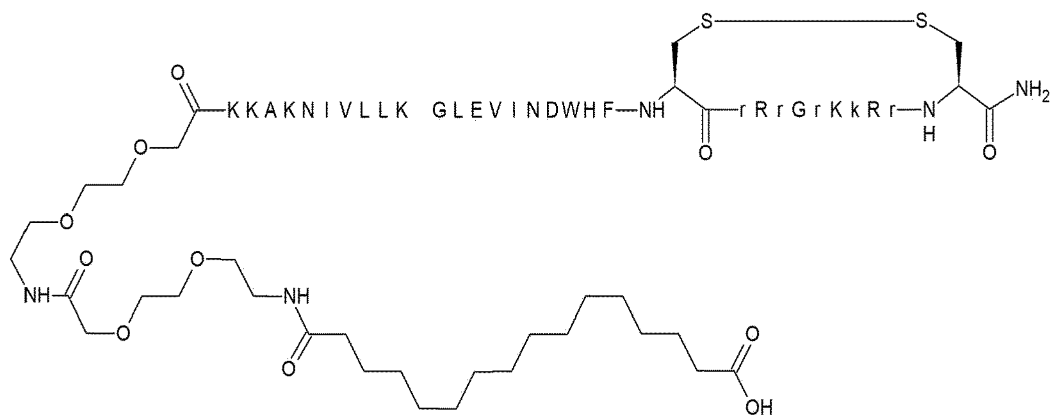

where the lower case letters are D-amino acids and the cysteine side chains form a disulfide bond.

*Nucleic acid-based therapeutic*

Another aspect provides a nucleic acid encoding a peptide comprising an amino acid sequence, where the amino acid sequence comprises KKAKNIVLLKGLEVINDWHF (SEQ ID NO: 1).

The term "nucleic acid" herein is meant either DNA or RNA, or molecules which contain both ribo- and deoxyribonucleotides. The nucleic acid may be double stranded, single stranded, or contain portions of both double stranded or single stranded sequence. In some embodiments, the nucleic acid is a recombinant nucleic acid. The nucleic acid described herein may comprise or be operably linked to any suitable transcriptional and/or translational regulatory sequences.

In some embodiments, the nucleic acid is an episome. The nucleic acid may be a plasmid or a minicircle. In some embodiments, the nucleic acid is messenger RNA (mRNA) or circular RNA (circRNA).

The nucleic acid may be delivered to a target cell via a viral vector. The nucleic acid may be viral vector genome. The viral vector may be selected from the group consisting of adeno-associated virus vector, an adenovirus vector, a

retrovirus vector, a lentivirus vector, and a herpes simplex virus vector. Any suitable viral vector may be employed.

Nonviral systems for introducing the nucleic acid described herein may also be employed. Nonviral DNA vectors can include a plasmid or minicircle. Nonviral RNA vectors can include a messenger RNA or circular RNA. In some embodiments, the non-viral carrier is selected from the group consisting of nanoparticles, liposomes, cationic polymer, and calcium phosphate particles.

In some embodiments, the nucleic acid is delivered to the target cell via a non-viral delivery system. In some embodiments, the non-viral delivery system is selected from the group consisting of nanoparticles, liposomes, cationic polymer, calcium phosphate particles, gene gun, electroporation, particle bombardment, ultrasound utilisation, and magnetofection.

The peptide encoded by the nucleic acid described herein may be expressed directly in target cells. Alternatively, or additionally, the peptide encoded by the nucleic acid described herein may be expressed in non-target cells of a subject. In this embodiment, the peptide preferably further comprises a CPP as described herein. The peptide may also further comprise a peptide-based half-life extending moiety as described herein. A target cell may be an immune cell, a cancer cell or a cell infected with a virus. The target cell may be any cell present in the TME, for example, an endothelial cell, a fibroblast, a pericyte/smooth muscle cell, an immune cell. Thus, the nucleic acid can be expressed by any cell in the TME and act as a bystander sensitizer. The immune cell may be a lymphocyte, a macrophage, a dendritic cell, a leukocyte, a monocyte, a neutrophil, or an eosinophil.

In some embodiments, the amino acid sequence consists of KKAKNIVLLKGLEVINDWHF (SEQ ID NO: 1). In some embodiments, the peptide encoded by the nucleic acid described herein consists of KKAKNIVLLKGLEVINDWHF (SEQ ID NO: 1).

*STING stimulating agent*

A STING stimulating agent (or STING agonist) is an agent that induces STING pathway activation. As described herein, STING activation may lead to increased STING phosphorylation, and downstream activation of IRF3 and NF-kB. STING activation may lead to the upregulation of transcription of type I Interferon genes, cytokines and chemokines which support activation and infiltration of immune cells. In an example, these immune cells may present new cancer antigens, search and destroy cancer cells.

The agent is capable of amplifying STING pathway activation in the presence of an endogenous STING stimulating agent (e.g., cytoplasmic DNA or cGAMP). The inventors have also found the agent is capable of amplifying STING pathway activation in the presence of an exogenous STING stimulating agent. The agent of the invention is not itself a STING stimulating agent (or STING agonist) but is capable of amplifying the STING activation pathway. Without a STING stimulating agent, the agent of the invention is not believed to trigger phosphorylation of STING.

An exogenous STING stimulating agent may be used in combination with the agent of the invention.

An aspect of the present invention provides a composition comprising the agent described herein and a STING stimulating agent. Any suitable STING stimulating agent may be used in the present invention.

The STING stimulating agent may be a nucleic acid, such as DNA or RNA. The DNA or RNA may be single stranded or double stranded. In some embodiments, the DNA may be modified to increase stability. Examples modifications include, but are not limited to, locked nucleic acid (LNA), phosphorothioate (PS) linkages, phosphoramidite, morpholine, 2'O-methyl modified bases, 2'Fluoro modified bases, and inverted dT and ddT which are resistant to degradation. In some embodiments, the RNA may be modified to increase stability. Example modifications include, but are not limited to, locked nucleic acid (LNA), phosphorothioate (PS) linkages, phosphoramidite, morpholine, 2'O-methyl modified bases, and 2'Fluoro modified bases.

STING stimulating agents may include cyclic di-nucleotides (CDNs). In some embodiments, the CDNs are natural CDNs or synthetic CDNs. An example of a natural CDN is cyclic 2'3' GMP-AMP (cGAMP). Also anticipated are chemical stabilized forms of 2'3' cGAMP. In some embodiments, the CDN can be selected from cGMP, cyclic di-GMP (c-di-GMP), cAMP, cyclic-di-AMP (c-di-AMP), cyclic-GMP-AMP (cGAMP), cyclic-di-IMP (c-di-IMP), cyclic-AMP-IMP (cAIMP), and any analogue thereof that acts as a STING stimulatory agent. The CDNs may have 2'2', 2'3', 2'5', 3'3' or 3'5' bonds linking the cyclic di-nucleotides. Also anticipated are purine dinucleotides. Modifications include, but are not limited to, phosphorothioate, biphosphorothioate, fluorinate, and difluorinated modifications. Synthetic CDNs can include, but are not limited to, 2'3'-cGSASMP, ML RR-S2 CDG, ML RR-S2 cGAMP, MK-1454, MK-2118, and ML RR-S2 CDA (ADU-S100), BI-STING, BMS-986301, GSK532, JNJ-4412, MK-1454, SB11285, 3'-3'-cyclic AIMP.

Further examples of CDNs include but are not limited to E7766, SB-11285, ML RR-S2 CDA, ML RR-S2c-di-GMP, ML-RR-S2 cGAMP, 2' 3' -c-di-AM(PS)2, 2'3'-cGAMP, 2'3'-cGAMPdFHS, 3'3'-cGAMP, 3'3'-cGAMPdFSH, cAIMP, cAIM(PS)2, 3'3'-cAIMP, 3'3'-cAIMPdFSH, 2'2'-cGAMP, 2'3'-cGAM(PS)2, 3'3'-cGAMP, c- di-AMP, 2'3'-c-di-AMP, 2'3'-c-di-AM(PS)2, c-di-GMP, 2'3'-c-di-GMP, c-di-IMP, c-di-UMP or any combination thereof.

In some embodiments, the STING stimulating agent may be selected from the group consisting of:

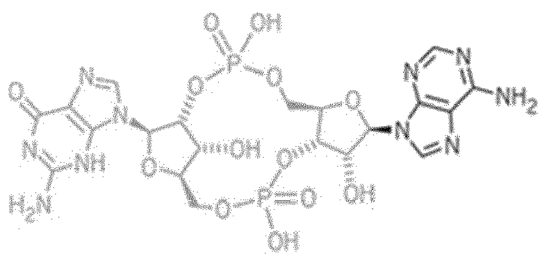

2'3'-cGAMP

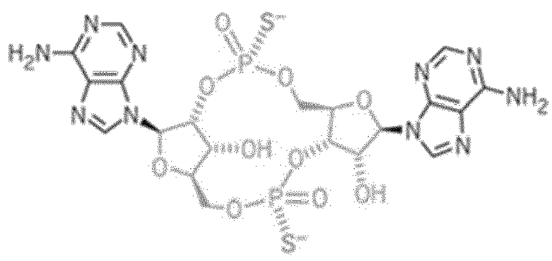

Aduro Biotech's ADU-S100

,

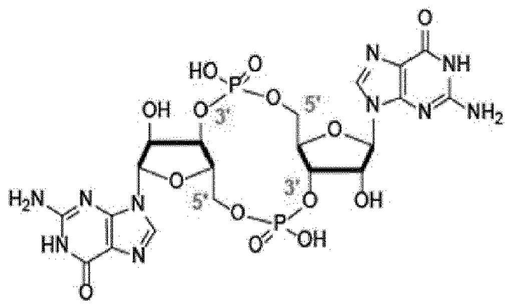

CDG

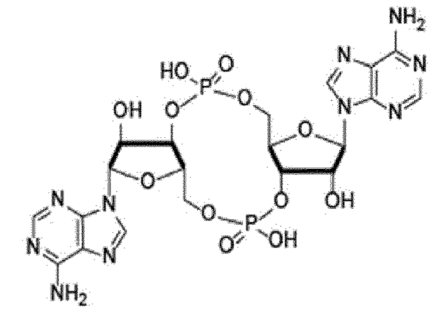

CDA

,

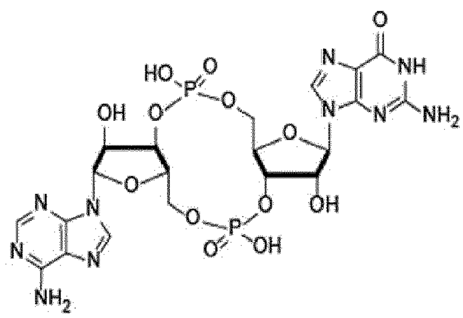

3',3'-cGAMP

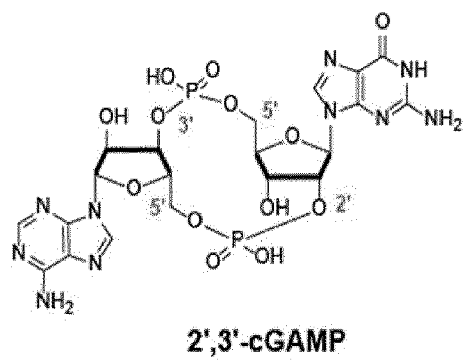

2',3'-cGAMP

, or .

Also anticipated are non-cyclic di-nucleotide STING stimulating agents (non-CDN compounds). Other examples of small molecule STING stimulating agents (agonists), include but are not limited to 5,6-dimethylxanthenone-4-acetic acid (DMXAA), ABZI, diABZI, GSK3745417, BMS-986301, STING agonist-1, FAA, CMA, α-Mangostin, BNBC, DSDP, diABZI, Bicyclic Benzamides, Benzothiophenes, MSA-2 (benzothiophene oxobutanoic acid), SR-717, SB11285, IMSA-101 (as described in Aval et al. J. Clin. Med. 2020, 9, 3323). Further examples include, ALG-031048, E7766, JNJ-'6196, MK-2118, MSA-1, MSA-2, SNX281, SR-717, TAK676, TTI-10001 (as described in Amouzegar et al., Cancers, 2021, 13(11), 2695).

In some embodiments, the STING agonist is a small molecule STING agonist. For example, di-amidobenzimidazole (also known as diABZI compound 3, and more recently also known as GSK3745417, as shown below).

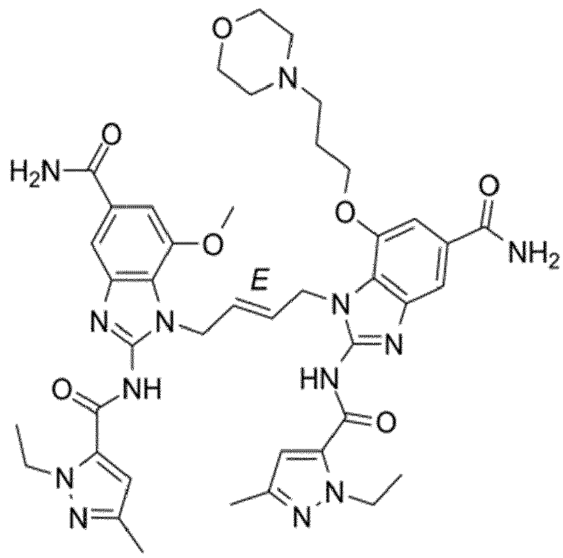

HCl HCl HCl

In some embodiments, the STING pathway can be activated by an ENPP1 inhibitor. ENPP1 inhibitors include, but are not limited to, MV-626, SR-8314, SR-8291, SR8541A, MAVU-104 (Mavupharma), rosmarinic acid (also known as SYL-001), ARL67156, and etidronic acid.

Other STING stimulating agents include nanoparticle (NP) vaccines or nanovaccines. Nanoparticle (NP) vaccines or nanovaccines are a platform capable of optimizing the spatiotemporal coordination of antigen presentation to APCs with innate immune responses in order to amplify the cytotoxic T cell responses. An example includes ONM-500 nanovaccine. ONM-500 is a novel pH-sensitive polymer that is capable of forming an antigen-encapsulating nanoparticle. It is anticipated to be capable of functioning both as a carrier for antigen delivery to DCs and as an adjuvant, activating the STING pathway.

Also considered as antibody-drug conjugates (ADCs). In this context, the ADC contains a STING agonist as payload or a payload known to cause DNA damage (e.g. Topoisomerase inhibitor, DNA intercalating agents etc.). Antibody drug conjugates (ADCs) represent a novel approach for systemic yet targeted STING agonist delivery. Further considered are immune-stimulating antibody conjugates (ISACs). ISACs are engineered (monoclonal) antibodies coupled to synthetic pattern recognition receptor (PRR) ligands that potentiate immune responses by triggering the production of proinflammatory cytokines and chemokines. Non limiting examples of ADCs include XMT-2056, CRD-5500, and TAK-500. XMT-2056 is an ADC composed of a monoclonal antibody directed against HER2 linked to a STING agonist. CRD-5500 similar comprises a monoclonal antibody directed against HER2 (Trastuzumab) linked to a STING agonist. TAK-500 comprises three parts: a STING agonist payload based on TAK-676, an IgG1 anti-CCR2 antibody, and a self-immolating maleimide-containing protease-cleavable peptide linker.

A bacterial vector may also be used to activate the STING pathway. Bacterial vectors have been used as a novel approach for delivering STING agonists to intratumoural antigen-present cells. An example is SYNB1891. SYNB1891 is a non-pathogenic *E.coli Nissle* strain engineered to express cyclic-di-AMP-producing enzymes in response to the hypoxic environment of a tumour, while remaining localised to the tumour microenvironment (TME). Another example is STACT-TREX-1. STACT-TRAX-1 is an attenuated Salmonella Typhimurium strain that carries an inhibitory microRNA to TREX-1 , which is an exonuclease capable of preventing activation of STING pathway degrading cytosolic DNA.

The STING pathway may be activated by an extracellular vesicle comprising one or more STING agonists. The STING agonists may be exogenously loaded into the engineered extracellular vesicle. The extracellular vesicle may an exosome. The extracellular vesicle may be modified to expresses high levels of an exosomal surface glycoprotein. The glycoprotein may be a PTGFRN protein. In some embodiments, the STING agonist is exoSTING (Codiak Biosciences, US). exoSTING is an engineered extracellular vesicle (EV) therapeutic that is capable of delivering STING agonists to target cells (e.g., tumour resident APCs). exoSTING is capable of enhancing the potency of the STING agonist (e.g., a CDN) and preferentially activating antigen present cells in the TME.

The STING stimulating agent (agonist) may be any of those described in Amouzegar et al., Cancers, 2021, 13(11), 2695.

*DNA damage inducing therapies*

The agent of the invention may be used in combination with a DNA damage inducing therapy. Without wishing to be bound by theory, STING activation may be indirectly activated by promoting DNA damage. Such therapies may be used to increase the local amount/concentration of the cytoplasmic DNA and/or cGAMP production. The therapy may be chemotherapy or radiotherapy (or radiation therapy). The chemotherapy may include an anti-cancer agent that is capable of inducing DNA damage such as alkylating agents, anthracyclines or platinum-based drugs. The radiotherapy may include external beam radiation therapy, brachytherapy (sealed source radiotherapy), radionuclide therapy, or intraoperative radiotherapy.

The agent of the invention may thus be used in combination with a DNA damage inducing agent. In some embodiments, STING activation can be indirectly induced by DNA damage repair (DDR) inhibitors. Examples include, but are not limited to, PARP inhibitors, ATM inhibitors, POLQ inhibitors, topoisomerase inhibitors, and DNA crosslinking agents. The DNA damage inducing agent may also be a radionuclide. The radionuclide may be conjugated to a targeting moiety such as an antibody (e.g., a radionuclide antibody-conjugate).

Poly (ADP-ribose) polymerase (PARP) inhibitors are inhibitors capable of inhibiting PARP proteins. PARP is a family of proteins comprising PARP1 and PARP2, which are both enzymes involved in a DNA repair pathway for single stranded breaks (SSB) called the base excision repair pathway (BER). Non-limiting examples of PARP inhibitors include AG014699, Veliparib, Olaparib, Iniparib, Rucaparib, Niraparib, Talazoparib, MK4827, CEP9722, BMN-673 and E7016.

Ataxia-telangiectasia (ATM) plays a central role in response to DNA double strand breaks and other lesion to protect the genome against DNA damage. ATM inhibitor can therefore be used to promote DNA damage. Non-limiting examples of ATM inhibitors include AZD0156, KU-59403, KU-60019, KU-55933, AZ31, CP466722, GSK635416A, AZD1390, AZ32, M3541, and M4076. Some ATM inhibitors are also capable of targeting other members of the phosphatidylinositol 3-kinase-related kinase (PIKK) family, such as ATR. Examples of these inhibitors include but are not limited to Torin2, ETP-46464, NVP-BEZ235, Wortmannin, and CGK733.

POLQ inhibitors are a new class of drugs, which are capable of interrupting a third DNA repair pathway, known as theta-mediated end joining (TMEJ). DNA polymerase theta (known as POLQ) is an enzyme encoded by the *POLQ* gene, which plays a key role in TMEJ. TMEJ is one of the three main double strand break repair pathways. Typically, most double-strand breaks (DSBs) are repaired by non-homologous end joining (NHEJ) or homology directed repair (HDR), which requires *BRCA1 and BRCA2.* In cells that lack HR, such as BRCA-gene deficient cancer cells, TMEJ serves as an essential backup pathway to repair DSBs. Example POLQ inhibitors include ART558 (described in Zatreanu et al. 2021), novobiocin (NVB) (described in Zhou et al., 2021), and ART899 (described in Rodriguez-Berriguete et al., 2023). Any suitable POLQ inhibitor may be used.

Topoisomerase inhibitors are capable of blocking the action of topoisomerases, which are involved in the modulation of the topological structure of the double helix. Topoisomerases include type I topoisomerases and type II topoisomerases. In some embodiments, the topoisomerase inhibitor is a type I topoisomerase inhibitor. Type I topoisomerases are capable of forming a transient type I topoisomerase-DNA intermediate that allows for the rotation of the cleaved strand around the helical axis and then re-ligating the cleaved strand to reform duplex DNA. Type I topoisomerase inhibitors are capable of stabilizing the intermediate cleavable complex, prevent DNA re-ligation, and inducing DNA strand breaks. Non-limiting examples include, Camptothecin-derived inhibitors, indenoisoquinoline, phenanthridines, and indolocarbazoles. In some embodiments, the topoisomerase inhibitor is a type II topoisomerase inhibitor. Type II topoisomerases alter DNA topology by generating double-stranded breaks in DNA. In some embodiments, the topoisomerase inhibitor is a type II topoisomerase inhibitor. Type II topoisomerase inhibitors comprise catalytic inhibitors or poisons. In some embodiments, the topoisomerase inhibitor is a type II topoisomerase catalytic inhibitor. Type II topoisomerase catalytic inhibitors are capable of binding to the N-terminal ATPase subunit of a type II topoisomerase, preventing the release of the separated DNA strands from the enzyme dimer. A non-limiting example is ICRF-187. In some embodiments, the topoisomerase inhibitor is a type II topoisomerase poison. Type II topoisomerase poisons are capable of either promoting the formation of covalent type II topoisomerase-DNA cleavage complexes or by inhibiting re-ligation of the cleaved strand. Non-limiting examples include doxorubicin, daunorubicin, epirubicin, idarubicin, etoposide, teniposide, dexrazoxane, novobiocin, merbarone, and anthrycycline aclarubicin.

DNA crosslinking agents is an agent that is capable of binding DNA nucleotides together blocking DNA synthesis. Non-limiting examples include nitrogen mustards, cisplatin, chloro ethyl nitroso urea (CENU (such as carmustine (BCNU)), psoralens, Pyrrolo[2,1-c][1,4]benzodiazepine (PBD) dimers and mitomycin C.

Alternatively, or additionally, radiotherapy (also known as radiation therapy) may be used to induce DNA damage. Radiotherapy is a therapy involving ionizing radiation which damages the DNA of target cells, typically cancer cells, leading to cell death. In some embodiments, the radiotherapy is selected from the group consisting of external beam radiation therapy, brachytherapy (sealed source radiotherapy), radionuclide therapy, or intraoperative radiotherapy. External beam radiation therapy comprises pointing an external source of ionizing radiation at subject. In other words, external beam radiotherapy involves directing radiation at a tumour from outside of the body. Non-limiting examples of

external beam radiotherapy include photon therapy (e.g., X-rays), particle therapy (e.g., proton beam therapy) and FLASH radiotherapy (FLASH-RT). Brachytherapy is delivered by placing radiation source(s) inside or next to the area requiring treatment, meaning that a subject can be treated with very high doses of localised radiation, reducing the possibility of damage to nearby tissues. Radionuclide therapy (also known as systemic radioisotope therapy, radiopharmaceutical therapy, or molecular radiotherapy), is a form of targeted therapy. The radionuclides may emit alpha- or beta-particles. Targeting may be achieved by attaching the radioisotope to a targeting polypeptide (e.g., an antibody) to guide it to the target tissue. Thus, in some embodiments, the radiotherapy is a radionuclide antibody-conjugate. The radioisotopes (or radionuclides) are typically delivered through infusion (into the bloodstream) or ingestion. Known examples of radionuclide therapy include the use of metaiodobenzylguanidine (MIBG) to treat neuroblastoma, iodine-131 to treat thyroid cancer or thyrotoxicosis, hormone-bound lutetium-177 and yttrium-90 to treat neuroendocrine tumours (peptide receptor radionuclide therapy), radioactive yttrium-90 or holmium-166 microspheres treat liver tumours or liver metastases. Intraoperative radiotherapy involves applying therapeutic levels of radiation to an exposed target area during surgery.

*Checkpoint inhibitors*

The agent described herein may be used in combination with a checkpoint inhibitor (also known as immune checkpoint inhibitors (ICIs)). Checkpoint inhibitors relieve inhibitory signals from receptors such as PD1 or CTLA4 on tumour-infiltrating lymphocytes (TILs), restoring antitumour immunity via $CD8^+$ cytotoxic T lymphocytes (CTLs). Check point inhibitors include but are not limited to, PD-1 inhibitors, PD-L1 inhibitors, and CTLA-4 inhibitors. For example, anti-PD-1 antibodies, anti-PD-L1 antibodies, anti-CTLA-4 antibodies, etc. Examples of such antibodies include, but are not limited to, spartalizumab, pembrolizumab, nivolumab, ipilimumab, atezolizumab, avelumab, durvalumab and cemiplimab.

Without wishing to be bound by theory, it is believed that secretion of type I interferons (IFN $\alpha$ and $\beta$) released after STING stimulation may lead to an increase in the expression of programmed death-ligand 1 (PD-L1) in the TME (Lee et al., 2022). Thus, STING activation may result in an upregulation of PD-L1. It is believed that using the agent in combination with a checkpoint inhibit may induce higher levels of tumour growth delay and/or inhibition. In some embodiments, the checkpoint inhibitor is anti-PD-L1 inhibitor. The anti-PD-L1 inhibitor may be an anti-PDL1 antibody, such as atezolizumab, avelumab or durvalumab. In some embodiments, the checkpoint inhibitor is an anti-PD1 inhibitor. The anti-PD1 inhibitor may be an anti-PD1 antibody, such as nivolumab, pembrolizumab, or cemiplimab.

*Medical use*

Another aspect provides the agent for use in therapy. Also considered is the composition described herein for use in therapy.

Since the agent is capable of enhancing STING activation, it may be used in the treatment of a disease or disorder which can be treated, prevented or ameliorated by activation of the STING pathway. For example, the disease or disorder may be associated with insufficient STING activity.

The disease or disorder which can be treated, prevented or ameliorated by activation of the STING pathway may also be an infection with a DNA pathogen, particularly where IFN is deleterious. Example disorders include hepatisis, malaria, listeria, or infection with herpes simplex virus (HSV), human immunodeficiency virus (HIV), or human papillovirus (HPV).

Another aspect provides the agent for use in a method of treating cancer. The disclosure also provides the agent in the manufacture of a medicament for treating cancer. Also described is a method of treating cancer, the method comprising administering therapeutically- or prophylactically-effective amount of the agent to a subject in need of treatment.

The present disclosure also provides the agent for use in a method of treating cancer, wherein the method further comprises administering a STING stimulating agent. The disclosure further provides the use of the agent in the manufacture of a medicament for use in a method of treating cancer, wherein the method further comprises administering a STING stimulating agent. Also described is a method of treating cancer, the method comprising administering therapeutically- or prophylactically-effective amount of the agent to a subject in need of treatment, wherein the method further comprises administering a STING stimulating agent.

The present disclosure also provides the agent and a STING stimulating agent for use in treating cancer. Also provided is the use of an agent and a STING stimulating agent in the manufacture of a medicament for use in a method of treating cancer. Further provided is a method of treating cancer, the method comprising administering therapeutically- or prophylactically-effective amount of the agent and STING stimulating agent to a subject in need of treatment. The agent and STING stimulating agent may be in the form of a pharmaceutical combination or pharmaceutical composition comprising the agent and the STING stimulating agent.

In some aspects and embodiments, the agent and the STING stimulating agent may be provided as a combination therapy. In some embodiments, the agent and STING stimulating agent may be administered simultaneously or sequentially. Simultaneous administration refers to administration of the two or more agents together, for example as a pharmaceutical composition containing both agents (i.e., as a combined preparation), or immediately after one another (e.g., within 1, 4, 6, 8 or 12 hours), and optionally via the same route of administration, e.g., to the same artery, vein, or other blood vessel. Sequential administration refers to administration of one of the agents followed after a given time interval by separate administration of another agent. It is not required that the agents are administered by the same route, although this is the case in some embodiments. The time interval may be any time interval. The STING stimulating agent may be selected from any of the STING stimulating agents (or STING agonists) disclosed herein. The pharmacological STING stimulating agent may be delivered locally. The STING stimulating agent may be administered parenterally (e.g., intramuscularly (IM), subcutaneously (SC) and intravenously (IV)). In some embodiments, the STING stimulating agent is administered orally.

For example, the STING stimulating agent may be administered after the agent is administered to a subject. This may be about 5 minutes to 24 hours, 10 minutes to 18 hours, 15 minutes to 12 hours, 30 mins to 8 hours, 1 hour to 6 hours following administration of the agent to the subject. In some embodiments, the STING stimulating agent is administered within 2 half-lives, 3 half-lives, 4 half-lives, or 5 half-lives of the agent of the invention. In another example, the agent is administered after the STING stimulating agent is administered to a subject. This may be about 5 minutes to 24 hours, 10 minutes to 18 hours, 15 minutes to 12 hours, 30 mins to 8 hours, 1 hour to 6 hours following administration of the STING stimulating agent to the subject. Alternatively, the agent of the invention may be administered within 2 half-lives, 3 half-lives, 4 half-lives, or 5 half-lives of the STING stimulating agent. In some embodiments, the agent of the invention is administered within 2 half-lives of the STING stimulating agent.

It is considered that a broad range of cancers can be treated using the agent or composition of the present invention. For example, colon carcinoma, breast cancer, pancreatic cancer, ovarian cancer, prostate cancer, fibrosarcoma, myxosarcoma, liposarcoma, chondrosarcoma, osteogenic sarcoma, chordoma, angiosarcoma, endotheliosarcoma, lymphangeosarcoma, lymphangeoendothelia sarcoma, synovioma, mesothelioma, Ewing's sarcoma, leiomyosarcoma, rhabdomyosarcoma, squamous cell carcinoma, basal cell carcinoma, adenocarcinoma, sweat gland carcinoma, sebaceous gland carcinoma, papillary carcinoma, papillary adenocarcinomas, cystandeocarcinoma, medullary carcinoma, bronchogenic carcinoma, renal cell carcinoma, hepatoma, bile duct carcinoma, choriocarcinoma, seminoma, embryonal carcinoma, Wilms' tumour, cervical cancer, testicular tumour, lung carcinoma, small cell lung carcinoma, bladder carcinoma, epithelial carcinoma, glioblastomas, neuronomas, craniopharingiomas, schwannomas, glioma, astrocytoma, medulloblastoma, craniopharyngioma, ependymoma, pinealoma, hemangioblastoma, acoustic neuroama, oligodendroglioma, meningioma, melanoma, neuroblastoma, retinoblastoma, leukemias and lymphomas, acute lymphocytic leukemia and acute myelocytic polycythemia vera, multiple myeloma, Waldenstrom's macroglobulinemia, and heavy chain disease, acute nonlymphocytic leukemias, chronic lymphocytic leukemia, chronic myelogenous leukemia, Hodgkin's Disease, non-Hodgkin's lymphomas, rectum cancer, urinary cancers, uterine cancers, oral cancers, skin cancers, stomach cancer, brain tumours, liver cancer, laryngeal cancer, esophageal cancer, mammary tumours, childhood-null acute lymphoid leukemia (ALL), thymic ALL, B-cell ALL, acute myeloid leukemia, myelomonocytoid leukemia, acute megakaryocytoid leukemia, Burkitt's lymphoma, acute myeloid leukemia, chronic myeloid leukemia, and T cell leukemia, small and large non-small cell lung carcinoma, acute granulocytic leukemia, germ cell tumours, endometrial cancer, gastric cancer, cancer of the head and neck, chronic lymphoid leukemia, hairy cell leukemia and thyroid cancer.

The present inventors have found that the agent of the invention potentiates the immunological benefit of cytoplasmic DNA observed in tumours with high mutation loads and/or chromosomal instability (CIN), which results in a local induction of supraphysiological expression levels of proinflammatory cytokines and/or chemokines in the tumour microenvironment (TME). Without wishing to be bound by theory, the agent is anticipated to be capable of sensitising tumour and innate immune cells to improve the adaptive anti-tumour immune response.

The cancer therapy described herein may further comprise treatment with a DNA damage inducing therapy, such as chemotherapy or radiotherapy. Thus, the cancer therapy may comprise treatment with radiation or a DNA damage inducing agent, such as a PARP inhibitor, an ATM inhibitor, a POLQ inhibitor, a topoisomerase inhibitor, a DNA crosslinking agent, or a radionuclide, to increase the local amount/concentration of the cytoplasmic DNA and/or cGAMP production. Thus, the present disclosure provides the agent for use in a method of treating cancer, wherein the method further comprises administering a DNA damage inducing agent. The method may further comprise administering a PARP inhibitor, an ATM inhibitor, a POLQ inhibitor a topoisomerase inhibitor, a DNA crosslinking agent, and/or a radionuclide. The present disclosure also provides the agent for use in a method of treating cancer in a subject, wherein the method further comprises subjecting the subject to radiotherapy.

Also provided is the use of the agent in the manufacture of a medicament for use in a method of treating cancer, wherein the method further comprises administering a DNA damage inducing agent. Further provided is a method of treating cancer, the method comprising a therapeutically- or prophylactically-effective amount of the agent, wherein the method further comprises administering a DNA damage inducing agent to a subject in need of treatment. The disclosure also provides a method of treating cancer, the method comprising a therapeutically- or prophylactically-effective amount

of the agent to subject in need of treatment, wherein the method further comprises subjecting the subject to radiotherapy.

The present disclosure also provides the agent and a DNA damage inducing agent for use in treating cancer. Also provided is the use of an agent and DNA damage inducing agent in the manufacture of a medicament for use in a method of treating cancer. Further provided is a method of treating cancer, the method comprising administering a therapeutically- or prophylactically-effective amount of the agent and a DNA damage inducing agent. The agent and a DNA damage inducing agent may be in the form of a pharmaceutical combination or pharmaceutical composition comprising the agent and the DNA damage inducing agent such as a PARP inhibitor, an ATM inhibitor, a POLQ inhibitor, a topoisomerase inhibitor, a DNA crosslinking agent, and/or a radionuclide to a subject in need of treatment.

In some aspects and embodiments, the agent and the DNA damage inducing agent may be provided as a combination therapy. In some embodiments, the agent and the DNA damage inducing agent may be administered simultaneously or sequentially. The DNA damage inducing agent may be as described herein.

In some embodiments, the composition described herein further comprises a DNA damage inducing agent, such as a PARP inhibitor, an ATM inhibitor, a POLQ inhibitor, a topoisomerase inhibitor, a DNA crosslinking agent, or radionuclide.

The cancer therapy described herein may further comprise treatment with a checkpoint inhibitor. Thus, the present disclosure provides the agent for use in a method of treating cancer, wherein the method further comprises administering a checkpoint inhibitor. Also provided is the use of the agent in the manufacture of a medicament for use in a method of treating cancer, wherein the method further comprises administering a checkpoint inhibitor. Further provided is a method of treating cancer, the method comprising a therapeutically- or prophylactically-effective amount of the agent, wherein the method further comprises administering a checkpoint inhibitor to a subject in need of treatment.

The present disclosure also provides the agent and a checkpoint inhibitor for use in treating cancer. Also provided is the use of an agent and a checkpoint inhibitor in the manufacture of a medicament for use in a method of treating cancer. Further provided is a method of treating cancer, the method comprising administering a therapeutically- or prophylactically-effective amount of the agent and a checkpoint inhibitor. The agent and a checkpoint inhibitor may be in the form of a pharmaceutical combination or pharmaceutical composition comprising the agent and the checkpoint inhibitor to a subject in need of treatment.

In some aspects and embodiments, the agent and the checkpoint inhibitor may be provided as a combination therapy. In some embodiments, the agent and the checkpoint inhibitor may be administered simultaneously or sequentially. The checkpoint inhibitor may be as described herein.

In some embodiments, the composition described herein further comprises a checkpoint inhibitor.

The agent as described herein may be used as an adjuvant therapy (i.e., given in addition to the primary or initial therapy to maximise the downstream effects of activating the STING pathway). For example, the agent may be used as a vaccine adjuvant. The agent may be added to DNA vaccines to enhance the body's immune response to a said DNA vaccine. The use of the agent of the invention may increase the strength and the duration of the immune response, as well as the breadth of the response to the vaccine. Thus, an aspect provides a vaccine composition (e.g., a DNA vaccine composition) comprising a vaccine (e.g., DNA vaccine) and the agent of the invention. The vaccine composition described herein may be for use in therapy.

The terms "treatment", "treat", or "treating" are used herein to refer to the reduction in severity of a disease or condition, the reduction in the duration of a disease; the amelioration or elimination of one or more symptoms associated with a disease or condition, or the provision of beneficial effect to a subject with a disease or condition. The term also encompasses prophylaxis of a disease or condition or its symptoms thereof. "Prophylaxis" is known in the art to mean decreasing or reducing the occurrence or severity of a particular disease outcome. For example, delaying progression of cancer in a subject.

As described herein, the agent of the invention acts as a STING sensitizer, capable of enhancing selective and potent immune responses exclusively at sites where STING is activated, such as tumour lesions. The use of the STING sensitizer beneficially results in local cytokine release meaning that there is no generalised cytokine induction after systemic administration. Thus, the present inventors have found that the agent of the invention can be safely delivered systemically. The agent described herein is believed to locally enhance the STING activation pathway.

In some embodiments, the agent, combination, or composition of the invention is administered to a subject. In some embodiments, the agent is administered to a subject systemically. The agent, combination, or composition may be administered parenterally. The agent, combination, or composition described herein may be administered in a suitable format/route, including, but not limited to intravenous, subcutaneous, intradermal, intraperitoneal, intrapleural, intraarticular, intrathecal, intratumourally, locally into organ the target organ(s) or administered as part of a medical device, e.g., medical or aesthetic implants. In some embodiments, the agent, combination, or composition is administered orally.

Administration is preferably in a "prophylactically effective amount" or a "therapeutically effective amount", this being sufficient to show benefit to the individual. The actual amount administered, and rate and time-course of administration, may depend on the individual subject and the nature and severity of their condition.

As used herein, the term "subject" refers to a human or any non-human animal (e g, mouse, rat, rabbit, dog,

cat, cattle, swine, sheep, horse or primate). In many embodiments, a subject is a human being. A subject can be a patient, which refers to a human presenting to a medical provider for diagnosis or treatment of a disease. The term "subject" is used herein interchangeably with "individual" or "patient". In some embodiments, the subject is human. A subject can be afflicted with or is susceptible to a disease or disorder but may or may not display symptoms of the disease or disorder. In some embodiments, the subject is affected or is likely to be affected with cancer.

*Pharmaceutical compositions:*

The agent described herein can be formulated in a pharmaceutical composition. Pharmaceutical compositions may comprise, in addition to one of the above substances, a pharmaceutically acceptable excipient, carrier, buffer, stabiliser or other material well known to those skilled in the art. Such substances should be non-toxic and should not interfere with the efficacy of the active ingredient. In some embodiments, the composition further comprises a pharmaceutically acceptable excipient.

Pharmaceutical compositions may be prepared using a pharmaceutically acceptable "carrier" composed of materials that are considered safe and effective. The term "carrier" refers to diluents, binders, lubricants and disintegrants. Those with skill in the art are familiar with such pharmaceutical carriers and methods of compounding pharmaceutical compositions using such carriers. "Pharmaceutically acceptable" refers to molecular entities and compositions that are "generally regarded as safe", e.g., that are physiologically tolerable and do not typically produce an allergic or similar untoward reaction, such as gastric upset and the like, when administered to a human. In some embodiments, this term refers to molecular entities and compositions approved by a regulatory agency of the US federal or a state government, as the GRAS list under section 204(s) and 409 of the Federal Food, Drug and Cosmetic Act, that is subject to premarket review and approval by the FDA or similar lists, the U.S. Pharmacopeia or another generally recognised pharmacopeia for use in animals, and more particularly in humans.

The pharmaceutical compositions provided herein may include one or more excipients, e.g., solvents, solubility enhancers, suspending agents, buffering agents, isotonicity agents, antioxidants or antimicrobial preservatives. When used, the excipients of the compositions will not adversely affect the stability, bioavailability, safety, and/or efficacy of the active ingredients. Thus, the skilled person will appreciate that compositions are provided wherein there is no incompatibility between any of the components of the dosage form. Excipients may be selected from the group consisting of buffering agents, solubilizing agents, tonicity agents, chelating agents, antioxidants, antimicrobial agents, and preservatives.

***Numbered paragraphs:***

1. An agent comprising an amino acid sequence, wherein the amino acid sequence comprises KKAKNIVLLKGLEVINDWHF (SEQ ID NO: 1).

2. The agent according to paragraph 1, wherein the agent further comprises a membrane transit moiety and/or a half-life extending moiety conjugated to the amino acid sequence.

3. The agent according to paragraph 1 or paragraph 2, wherein the agent has the formula:

X-Y-Z

wherein

X is a half-life extending moiety;
Y is the amino acid sequence comprising SEQ ID NO: 1; and
Z is a membrane transit moiety.

4. The agent according to paragraph 2 or paragraph 3, wherein the membrane transit moiety is a cell penetrating peptide (CPP).

5. The agent according to paragraph 4, wherein the CPP is a cyclic CPP.

6. The agent according to paragraph 5, wherein the cyclic CPP is cyclo[CrRrGrKkRrC] (SEQ ID NO: 2).

7. The agent according to any one of paragraphs 2 to 6, wherein the half-life extending moiety is a peptide or polypeptide, a carbohydrate molecule, a synthetic polymer, or comprises a fatty acid derivative.

8. The agent according to paragraph 7, wherein the half-life extending moiety comprises a fatty acid derivative of the formula:

$$HO\text{-}CO\text{-}(CH_2)_x\text{-}CO\text{-}*,$$

wherein x is an integer in the range of 12 to 18
wherein the half-life extending moiety optionally further comprises a hydrophilic linker connecting the fatty acid derivative with the amino acid sequence comprising SEQ ID NO: 1.

9. The agent according to paragraph 8, wherein the hydrophilic linker comprises (i) ethyleneglycol ether, (ii) propylenegycol ether, (iii) natural amino acids, and/or (iv) unnatural amino acids, and wherein the length of the hydrophilic linker is up to 60 atoms.

10. The agent according to paragraph 8 or paragraph 9, wherein the half-life extending moiety is represented by the formula:

$$HO\text{-}CO\text{-}(CH_2)_x\text{-}CO\text{-}(\gamma Glu)_y\text{-}(Adoa)_z\text{-}*,$$

wherein x is an integer in the range of 12 to 18,
y is an integer in the range of 0 to 2,
z is an integer in the range of 1 to 5.

11. The agent according to paragraph 10,

wherein x is 14, 15, 16, 17 or 18,
y is 0 or 1, and
z is 2, 3 or 4.

12. The agent according to any one of the paragraphs 7 to 11, wherein the half-life extending moiety is selected from

19-carboxynonoadecanoyl-Adoa-Adoa-,
19-carboxynonoadecanoyl-γGlu-Adoa-Adoa-,
17-carboxyheptadecanoyl-Adoa-Adoa-,
17-carboxyheptaadecanoyl-γGlu-Adoa-Adoa-,
15-carboxypentadecanoyl-Adoa-Adoa-, or
15-carboxypentadecanoyl-γGlu-Adoa-Adoa-.

13. The agent according to paragraph 12, wherein the half-life extending moiety is 15-carboxypentadecanoyl-Adoa-Adoa-.

14. The agent according to any one of paragraphs 7 to 14, wherein the agent is selected from:

15-carboxypentadecanoyl-Adoa-Adoa-KKAKNIVLLKGLEVINDWHF-c[CrRrGrKkRrC]-$NH_2$ (SEQ ID NO: 3); and
15-carboxypentadecanoyl-Adoa-Adoa-KKAKNIVLLKGLEVINDWHF-c[CrRrGrKkRrC]-OH (SEQ ID NO: 34);
or any pharmaceutically acceptable salt, amide, ester or prodrug thereof.

15. The agent according to any one of the preceding paragraphs, wherein the agent is capable of sensitising STING activation.

16. The agent according to any one of the preceding paragraphs, wherein the agent increases and/or accelerates trafficking of STING from the endoplasmic reticulum to the Golgi in a cell comprising the agent compared to an equivalent cell not comprising the agent, where both cells are exposed to or comprise a STING stimulating agent.

17. The agent according to any one of the preceding paragraphs, wherein the level of phosphorylated STING is

increased in a cell comprising the agent compared to an equivalent cell not comprising the agent, where both cells are exposed to or comprise a STING stimulating agent.

18. The agent according to any one of the preceding paragraphs, wherein the level of phosphorylated TBK1 is increased in a cell comprising the agent compared to an equivalent cell not comprising the agent, where both cells are exposed to or comprise a STING stimulating agent.

19. The agent according to any one of the preceding paragraphs, wherein expression of type 1 IFN and/or CXCL10 is increased in a cell comprising the agent compared to an equivalent cell not comprising the agent, where both cells are exposed to or comprise a STING stimulating agent.

20. The agent according to any one of the preceding paragraphs, wherein the agent decreases the $EC_{50}$ of STING stimulating agent-dependent expression of type I IFNs and/or CXCL10 in a cell comprising the agent compared to an equivalent cell not comprising the agent, wherein both cells are exposed to or comprise a STING stimulating agent.

21. The agent according to any one of the preceding paragraphs, wherein the agent increases the $E_{max}$ of STING stimulating agent-dependent expression of type I IFNs and/or CXCL10 in a cell comprising the agent compared to an equivalent cell not comprising the agent, wherein both cells are exposed to or comprise a STING stimulating agent.

22. The agent according to any one of paragraphs 19 to 21, wherein the type 1 IFN is IFN-$\beta$.

23. The agent according to any one of the preceding paragraphs, wherein the agent has reduced immunogenic potential compared to a reference agent.

24. The agent according to paragraph 23, wherein the reference agent comprises an amino acid sequence comprising KKYKNIVLLKGLEVINDYHF (SEQ ID NO: 4).

25. A nucleic acid encoding a peptide comprising an amino acid sequence, wherein the amino acid sequence comprises KKAKNIVLLKGLEVINDWHF (SEQ ID NO: 1).

26. The nucleic acid according to paragraph 25, wherein the nucleic acid is delivered to a target cell via a viral vector.

27. The nucleic acid according to paragraph 26, wherein the viral vector is selected from the group consisting of adeno-associated virus vector, an adenovirus vector, a retrovirus vector, a lentivirus vector, and a herpes simplex virus vector.

28. The nucleic acid according to paragraph 25, wherein the nucleic acid is delivered to a target cell via a non-viral carrier.

29. A composition comprising the agent according to any one of paragraphs 1 to 24.

30. The composition according to paragraph 29, wherein the composition further comprises a STING stimulating agent.

31. The composition according to paragraph 30, wherein the STING stimulating agent is selected from the group consisting of:

a) a nucleic acid;
b) a cyclic-di-nucleotide (CDNs);
c) a non-CDN small molecule STING stimulating agent;
d) an ENPP1 inhibitor;
e) a nanovaccine;
f) an antibody-drug conjugate;
g) a bacterial vector; and/or
h) an extracellular vesicle comprising one or more STING agonists, optionally exoSTING.

32. The composition according to paragraph 31, wherein the STING stimulating agent is a cyclic di-nucleotide (CDN).

33. The composition according to any one of paragraphs 29 to 32, wherein the composition further comprises a DNA damage inducing agent.

34. The composition according to paragraph 33, wherein the DNA damage inducing agent is selected from the group consisting of a PARP inhibitor, a POLQ inhibitor, an ATM inhibitor, a topoisomerase inhibitor, a DNA crosslinking agent, and a radionuclide.

35. The composition according to any one of paragraphs 29 to 34, wherein the composition further comprises a checkpoint inhibitor.

36. The composition according to any one of paragraphs 29 to 35, wherein the checkpoint inhibitor is an anti-PD-1 antibody.

37. An agent according to any one of paragraphs 1 to 24 for use in therapy.

38. An agent according to any one of paragraphs 1 to 24 for use in a method of treating cancer.

39. The agent for use according to paragraph 38, wherein the method further comprises administering a STING stimulating agent.

40. The agent for use according to paragraph 39, wherein the STING stimulating agent is a cyclic di-nucleotide (CDN).

41. The agent for use according to paragraph 39 or paragraph 40, wherein the STING stimulating is administered simultaneously or sequentially.

42. The agent for use according to any one of paragraphs 38 to 41, wherein the method further comprises administering a DNA damage inducing agent.

43. The agent for use according to paragraph 42, wherein the DNA damage inducing agent is selected from the group consisting of a PARP inhibitor, an ATM inhibitor, a POLQ inhibitor, a topoisomerase inhibitor, a DNA crosslinking agent, and a radionuclide.

44. The agent for use according to paragraph 42 or paragraph 43, wherein the DNA damage inducing agent is administered simultaneously or sequentially.

45. The agent for use according to any one of paragraphs 38 to 44, wherein the method further comprises administering a checkpoint inhibitor.

46. The agent for use according to paragraphs 45, wherein the checkpoint inhibitor is an anti-PD-1 antibody.

47. The agent for use according to paragraph 45 or paragraph 46, wherein the checkpoint inhibitor is administered simultaneously or sequentially.

48. The agent for use according to any one of paragraphs 38 to 47, wherein the agent is administered systemically.

49. A composition according to any one of paragraphs 29 to 36 for use in therapy.

50. A composition according to any one of paragraphs 29 to 36 for use in a method of treating cancer.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof. While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of

improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## *Examples*

All compounds (agents) of the present invention can be synthesised by those skilled in the art of organic synthesis, and peptide synthesis. For common procedures in solid-phase peptide synthesis using Fmoc protecting groups (Fmoc-SPPS), see also: "Fmoc Solid Phase Synthesis - A Practical Approach", Edited by W. C. Chan and P. D. White, Oxford University Press, 2000.

Agents tested in the following examples are shown in Table 2.

**Table 2. Agents**

| Name | Structure |
|---|---|
| **Agent 1** (SEQ ID NO: 3) | *15-carboxypentadecanoyl-Adoa-Adoa*-KKAKNIVLLKGLEVINDWHF-**c[CrRrGrKkRrC]-$NH_2$** |
| **Agent 2** (SEQ ID NO: 35) | KKYKNIVLLKGLEVINDYHF**GRKKRRQRRRPQ-$NH_2$** |
| **Agent 3** (SEQ ID NO: 36) | KKAKNIVLLKGLEVINDWHF**-c[CrRrGrKkRrC]-$NH_2$** |

The sequence shown in bold above denotes the cell penetrating peptide. The underlined sequence shows a mutation relative to the wild type core sequence (e.g., the core sequence of Agent 2 ("KKYKNIVLLKGLEVINDYHF", SEQ ID NO: 4)). The N-terminal modification is shown in italics, where Adoa denotes 8-amino-3,6-dioxaoctanoic acid. In some embodiments, the agent is SEQ ID NO: 3. In some embodiments, the agent is SEQ ID NO: 36.

### *EXAMPLE 1: Agent 1 enhances STING pathway activation*

*Materials and methods:*

*Cultivation of THP-1 cells:*

THP-1 cells were kept in culture for 7 weeks (up to passage number 20) in culture medium with RPMI 1640 supplemented with 10% fetal bovine serum (FBS) + 1% PS (Penicillin and Streptomycin) and an additional 2 mM L-glutamine (R10). Cells were counted with an automated cell counter (NucleoCounter NC-250) and adjusted to a $4\times10^5$ live cells per mL 24 hours prior to the experiment.

*Differentiation of THP-1 cells into a macrophage-like phenotype:*

On the day of the experiment, THP-1 cells were seeded into 48-well plates at $2\times10^5$ cells per well in 200 $\mu$L R10 with 200 nM phorbol 12-myristate 13-acetate (PMA). 24 hours post-seeding culture medium were changed to R10 (without PMA). 48 hours post seeding the culture medium was changed to RPMI 1640 without supplements.

*Sensitization experiment:*

The undifferentiated and PMA-differentiated THP-1 cells had the final media change before starting the sensitization experiment. The cells were then stimulated with 2'3'-cGAMP (8 μg/μL) or mock/UT (vehicle). After 30 minutes of incubation, cells were treated with either Agent 1 (5 μM), a peptide buffer control (labelled "No peptide") or mock/UT (RPMI 1640 media). The protein extract was isolated from two separate wells and then combined for the WB analysis. After 30 minutes, 1 hour, 2 hours, and 6 hours of incubation, the lysates were harvested in 65 μL ice-cold RIPA lysis buffer with 1% benzonase, CompleteTM Protease inhibitor, Pierce Protease Inhibitor, and NaF (10 mM) and then stored at -80 °C until analyzed by Western Blotting. The data presented here is representative of three independent experiments.

*Western blotting:*

Concentration of lysates were measured by Pierce™ Rapid Gold BCA Protein Assay Kit. For each condition, 10 μg protein were loaded on the gel, according to the manufacturer's instructions After measurements of protein concentrations, cell lysates were prepared for western blotting.

*Results:*

Both undifferentiated and PMA-differentiated THP-1 cells showed an increase of phospho-STING and phospho-TBK1 when treated with 2'3'-cGAMP in combination with Agent 1 compared to cells that have only been exposed to 2'3'-cGAMP. Phosphorylation of STING and TBK1 is significantly increased as early as 30 minutes post 2'3'-cGAMP and Agent 1 addition when compared to 2'3'-cGAMP treated cells. At 360 minutes after stimulation, the phosphorylation has returned to low levels. The results are shown in Figure 1.

*EXAMPLE 2: Agent 1 in combination with a STING agonist enhances STING trafficking to the Golgi.*

*Materials and methods:*

*Cell culture for Imagestream experiment:*

THP-1 cells were kept in culture for 7 weeks (up to passage number 20) in culture medium with RPMI 1640 supplemented with 10% fetal bovine serum (FBS) + 1% PS (Penicillin and Streptomycin) and an additional 2 mM L-glutamine (R10). Cells were then differentiated with Phorbol 12-myristate 13-acetate (PMA).

*Treatment:*

The THP-1 cells were seeded at 0.75 × $10^6$ cells/mL in 6-well plates, 2 mL/well and PMA-differentiated after two days. On the day of experiment the overnight medium was replaced with 1 mL of fresh R10 medium, and cells were incubated for 1 h at 37°C, 5% $CO_2$. The cells were treated with 6 μM Agent 1 alone, 6 μM Agent 1 in combination with 50 μg/mL cGAMP or peptide buffer together with 50 μg/mL cGAMP for 10-, 30-, 60- or 120-min. The control samples comprised of cells treated with peptide buffer (to mimic the buffer-media composition of 6 μM Agent 1), untreated cells (UT) and a positive control where cells were treated with 100 μg/mL cGAMP for 30 min.

*ImageStream:*

The colocalization of STING-ER and STING-Golgi was determined by the ImageStream MK II Imaging Flow Cytometer (Amnis). The treated cells were trypsinized with TrypLE for 10 min at 37 °C and stained with Live/Dead marker for 15 min at 4 °C. Next, the cells were fixed using 4 % formaldehyde for 20 min at room temperature, and then permeabilized with 0.25 % Triton X-100 for 7 min. The cells were incubated with primary antibodies, sheep anti-STING (1:50), rabbit anti-GM130 (1:1000), mouse anti-PDI (1:50), for 1 h on ice. Further, the samples were incubated with the Alexa-Fluor-labeled secondary antibodies, antimouse Alexa 488 (1:1000), anti-rabbit Alexa 647 (1:1000), anti-sheep Alexa 568 (1:1000) for 1 h on ice. After every step, the cells were washed with 1x PBS three times. Finally, the cells were resuspended in 1x PBS with 2 mM EDTA and 3 % BSA. Magnification of ×60 was used for all samples. Images of 17,000 to 20,000 single cells were acquired in the ImageStream and the data were analyzed using IDEAS software v6.3 (Amnis Corporation). After gating on focused, single, and positive cells with the defined fluorescent markers, the colocalization of STING-PDI (ER marker) and STING-GM130 (Golgi marker) was analyzed with the Bright Detail Similarity feature for the corresponding channels. The accuracy of the cell populations gating was controlled by visual inspection of multiple individual pictures in the borders of gated cell populations. Additionally, for gating accuracy a set of single

stained and FMO controls was acquired, Obtained values were plotted using GraphPad Prism 9.3.1. The statistical significance was assessed using one-way analysis of variance (ANOVA) with Tukey test for multiple comparison performed in GraphPad Prism 9.3.1. Symbols ns, *, **, *** and **** were given for p value > 0.05, ≤ 0.05, ≤ 0.01, ≤ 0.001 and ≤ 0.0001, respectively.

*Results:*

To model human macrophages, THP-1 cells were differentiated using PMA. The cells were treated with 6 μM of Agent 1 or peptide buffer either alone or with addition of 50 μg/mL of cGAMP for 10, 30, 60 or 120 min. The effect of Agent 1 on STING exit from endoplasmic reticulum (ER) and movement to the Golgi was studied by harvesting the cells, staining with antibodies against STING and against organelle-specific markers and analyzed using the ImageStream. Agent 1 combined with cGAMP significantly increases STING trafficking to from the ER to Golgi when compared to cGAMP single agent at all tested time points. In addition, Agent 1 induces a rapid STING trafficking to the Golgi which can be observed already after 10 min of treatment with Agent 1 and cGAMP (albeit not yet significant at this timepoint). Finally, STING residing in ER is significantly reduced at 60 and 120 min of treatment with Agent 1 and cGAMP. Single agent Agent 1 did not induce STING trafficking to Golgi at tested timepoints in PMA-differentiated THP-1 cells. The results are shown in Figure 2.

*EXAMPLE 3: Agent 1 combines with STING agonists to increase chemokine and cytokine expression when compared to different STING agonists (single agents)*

*Material and methods:*

*Cell culture:*

THP1 cells were purchased from ATCC (TIB-202™) and cultured in RPMI 1640 supplemented with 10% FBS and 1% Penicillin/Streptomycin (P/S) at 37 °C and 5% $CO_2$. Passaging of THP1 cells was kept below 20 passages.

*Compounds:*

2'3'-cGAMP was dissolved according to manufacturer's protocol (Invivogen tlrl-nacga23m) to 1 mg/mL in LAL water and subsequently diluted in RPMI 1640, 10% FBS, 1% (P/S) for usage in assay. ADU-S100 was dissolved according to manufacturer's protocol (tlrl-nacda2r) to 0.5 mg/mL in LAL and subsequently diluted in R10. diABZ! Compound 3 was diluted from a 1 mM stock in 100% DMSO into R10. Agent 1 was dissolved to a stock solution of 1250 μM in 50% DMSO, 25 mM HEPES. Concentration of dissolved Agent 1 was determined by A280 measurement using Synergy HI (Biotek Gen5 SW). Subsequently, Agent 1 dilutions were prepared in RPMI 1640, 10% FBS, 1% P/S (R10).

*Stimulation and sensitization assay:*

THP1 cells were seeded at a density of 80,000 cells/well in R10 in a 96 well plate and allowed to rest for 1 hour at 37 °C and 5% $CO_2$. Cells were stimulated with 2'3'-cGAMP (300 - 0.59 μg/mL) or ADU-S100 (200 - 0.39 μM) or diABZI C3 (20 - 0.039 μM) for 30 minutes followed by sensitization with or without 2 μM Agent 1, all in technical triplicates. Control cells were left untreated (UT) or treated with dilution buffer (DB) in technical triplicates. At six and twenty hours post STING agonist treatment, supernatants were collected and kept at -20 °C.

*Cell viability:*

Cell viability at twenty hours post STING agonist treatment was determined using AlamarBlue HS reagent according to manufacturer's protocol. Supernatants were harvested at 20 hours after addition of STING agonist and cells were then incubated with 100 μL staining solution (1:10 dilution of AlamarBlue HS reagent in R10) for a minimum of 4 hours at 37 °C and 5% $CO_2$. Absorbance was measured using Synergy HI plate reader (Biotek Gen5 SW) at 570 nm and 600 nm.

*CXCL10 and IFN-β interferon protein quantification:*

Concentrations of CXCL10 and IFN-β in the collected supernatants were determined by Duo-Set ELISA from R&D. Absorbance measurements at 450/570 nm and quantification were done using Synergy HI plate reader (Biotek Gen5 SW), Microsoft Excel and GraphPad prism v. 9.3.1.

*Statistical analysis:*

Data analysis and statistical analysis were done using GraphPad prism 9.3.1. Non-linear regression of the bell-shaped dose-response curves was performed using the model:

$$Span1 = Plateau1 - Dip$$

$$Span2 = Plateau2 - Dip$$

$$Section1 = Span1 / (1+(EC_{50}1/X)^\wedge nH1)$$

$$Section2 = Span2 /(1+(X/EC_{50}2)^\wedge nH2)$$

$$Y = Dip + Section1 + Section2$$

Plateau1: is the response plateau at the low doses (left end of the curve), same unit as y-axis.

Plateau2: is the response plateau at the high doses (right end of the curve), same unit as y-axis.

Dip: is the plateau in the middle of the curve, corresponding to $E_{Max}$, same unite as y-axis. The $E_{Max}$ determined here are not derived from this regression, due to the limitations using bell shaped dose response. Instead, $E_{Max}$-values were determined as the maximum response at any given STING agonist concentration for both IFN-β and CXCL10 from all experiments.

$EC_{50}1$: concentration of STING agonist giving half maximum response ($EC_{50}$) at the stimulation part of the curve (low dose increase = increase response), same unit at x-axis.

$EC_{50}2$: concentration of STING agonist giving half maximum response ($EC_{50}$) at the inhibition part of the curve (high dose increase = decrease response), same unit at x-axis.

nH1/nH2: Hill slopes associated with respective $EC_{50}$ values, not constrained.

*Results:*

THP1 wild type cells were titrated with increasing concentrations of 2'3'-cGAMP, ADU-S100 or diABZI C3 with or without 2 μM Agent 1. From the quantifications of CXCL10 and IFN-β release at six and twenty hours post agonist stimulation, four independently obtained $EC_{50}$ and $E_{Max}$ values were derived for the different STING agonists. 2'3'-cGAMP stimulation in THP1 cells affects cell viability negatively at 2'3'-cGAMP concentrations above 150 μg/mL as cell viability decreases to below 70% of untreated. This was independent of the presence of 2 μM Agent 1. The $E_{Max}$ for each conditions/timepoints were determined as the average of the maximum response at any given STING agonist concentration in combination with or without the fixed dose of Agent 1. The IFN-β response was higher at 6 hours post stimulation and the CXCL10 response was more pronounced at 20 hours. Agent 1 sensitizes THP1 cells to 2'3'-cGAMP in an agonist dose-dependent manner as shown by the effect of the combination on CXCL10 and IFN-β release. Collectively for 2'3'-cGAMP, ADU-S100 and diABZI C3, the addition of Agent 1 significantly increased the CXCL10 $E_{Max}$ induced by these STING agonists. This Agent 1-dependent change in the $E_{Max}$ was not observed for the IFN-β response. The $EC_{50}$ values derived from the CXCL10 responses were determined from bell-shaped dose response curves. In all cases, addition of Agent 1 reduced the $EC_{50}$ of the different STING agonists in THP1 cells when assessing CXCL10 production. As 2'3'-cGAMP did not reach an IFN-β response plateau, no $EC_{50}$ value could be derived from 2'3'-cGAMP single agent. Overall, addition of Agent 1 resulted in a statistically significant increases in IFN expression at lower concentrations of cGAMP, thus confirming sensitization with a $EC_{50}$ around 20 μg/mL. Similarly, Agent 1 reduced ADU-S100 $EC_{50}$ by a factor of 2, although no significant improvement in $EC_{50}$ for type I FN production was observed for diABZI C3. The results are shown in Figure 3.

*EXAMPLE 4: Agent 1 sensitizes MC38 tumours to endogenous STING pathway in in vivo syngeneic model*

*Material and methods:*

The cell line MC38 was retrieved from the CRO *In-vitro* cell bank, propagated and used for cell injection with

following details. MC38 (Murine colon carcinoma) cells (passage number 9) with a viability of >90% was chosen for the study. Approximately 1 × $10^6$ cells suspended in 100 μL of serum free media were used for cell injection per mouse. Mice were subcutaneously injected with MC38 cells (approximately 1 × $10^6$ cells per mouse) and monitored for tumour growth. Female C57B/6 mice housed in Individually Ventilated Cages (IVCs) were used for the study. MC38 cell line were propagated into the animals by injecting the cells subcutaneously in the right flank region of the animals. The implanted area was monitored for growth of tumour. Once the tumour attained palpable stage and required tumour volume, total 50 mice with optimum tumour volume were selected for the study. Mice were randomized into treatment groups keeping 10 mice per group based on tumour volume (mean tumour volume ≈94$mm^3$) and IV dosing was initiated according to the table below (day 0 define as the day of randomization).

**Table 3. IV dosing**

| Group | N | Dose | Route | Frequency |
|---|---|---|---|---|
| Group-I, Vehicle control | 10 | 150 μL/animal | i.v | Q3D × 4 (on day 0, 3, 6 & 9) |
| Group-II, Agent 1 (10mg/kg) | 10 | 10 mg/kg | i.v | Q3D × 4 (on day 0, 3, 6 & 9) |
| Group-III, Agent 1 (20mg/kg) | 10 | 20 mg/kg | i.v | Q3D × 4 (on day 0, 3, 6 & 9) |

Table 3 shows the treatment route and schedule. Day 0 corresponds to the day of randomization based on tumour size pre-treatment.

Agent 1 formulation was prepared as follow. Buffer preparation -25 mM HEPES in endotoxin-free water with 2% propylene-glycol. Agent 1 was dissolved with buffer to get a predicted concentration. Raw Absorbance at 280nm using Thermo Scientific™ NanoDrop 2000C were taken and actual concentration was calculated for each compound using following formulae: (Absorbance / ε $M^{-1}$ $cm^{-1}$) × MW = concentration. Where, ε (Agent 1 - 5504 $M^{-1}$ $cm^{-1}$). For intravenous administration, the stock was used for dosing as 5mL/kg.

Individual body weight was measured once every three days during the study period. Animals were observed and recorded for any visible clinical sign during the study period. The tumour volume was determined by two-dimensional measurement with a digital Vernier calliper on the day of randomization (Day 0) and then once every three days. Using a Vernier calliper the length (L) and width (W) of the tumour was measured. Tumour volume (TV) was calculated using the following formula:

$$Tumor\ Volume\ (mm^3) = \frac{L \times W^2}{2}$$

where, *L* = Length (mm); *W* = Width (mm).

Mean, Standard Deviation (SD) or Standard Error of Mean (SEM) were calculated for individual groups. For the evaluation of the statistical significance of tumour inhibition, Two-way ANOVA followed by Bonferroni post hoc test was performed using Graph Pad Prism p values <0.05 indicate statistically significant differences between groups.

*Results:*

Vehicle control treated animals showed progressive tumour growth throughout the experiment. Most of the animals from vehicle control (6/10 mice) were removed from study after day 14 measurements due to tumour size. The efficacy evaluation against vehicle control group was performed based on day 14 tumour measurements due to removal of animals (humanely euthanized) from vehicle groups. Both the test groups (group II- Agent 1 (10 mg/kg), group III-Agent 1 (20 mg/kg)) exhibited significant antitumour activity against vehicle control in MC38 syngeneic tumour model when statistical evaluation was performed up to day 14 by Two-way ANOVA followed by Bonferroni post hoc test using Graph Pad Prism. Agent 1 showed better antitumour efficacy at its high dose when compared to the low doses. In conclusion, Agent 1 exhibited potential antitumour activity in the MC38 model. The results are shown in Figure 4.

*EXAMPLE 5: Agent 1 synergizes with suboptimal dose of the STING agonist ADU-S100 and induces tumour growth delay in a MC38 subcutaneous syngeneic tumours*

*Material and methods:*

For this experiment, the MC38 cells were cultured in DMEM + high glucose (Sigma-Aldrich D6429) with 10%

heat inactivated FCS, 2 mM glutamine and P/S. The cells were typically split twice a week at 1/30 using TrypLe when they reached 80-90% confluency. Cells were then centrifuged at 300xg for 5 minutes and resuspended in fresh medium. The cells were split one day prior inoculation to guarantee exponential growing phase. The day before MC38 cell inoculation the GFR Matrigel® was thawed on ice at 4°C. The cells were be washed and resuspended at $20\times10^6$ live cells/ml in sterile PBS-/- (without $Ca^{2+}/Mg^{2+}$) and subsequently diluted 1:1 in GFR Matrigel® to a working concentration of $10\times10^6$ live cells/mL.

The C57Bl/6 female mice were used for this study. Mice were be shaved on right flank and MC38 cells ($1.0\times10^6$ live cells in 100 uL 1:1 sterile PBS and GFR Matrigel®) were grafted subcutaneously under isoflurane anesthesia, using a 1 ml syringe and a 25g needle. The needles were kept on ice prior to cell inoculation in order to prevent the solution from solidifying. The mice were monitored for the appearance of palpable tumours every third day.

At 7 days post tumour inoculation, the mice had reached an average tumour volume of approx. 70-80 $mm^3$. Following this the 46 most homogenous mice, in terms of tumour volume, were selected and randomized based on the tumour volume. The animals were assigned a permanent number by ear-clipping. The cages were identified by cage cards indicating the study code, sex, cage number and number of animals. Treatment was initiated with vehicle or ADU-S-100 i.t. administration in combination with Agent 1 by i.v. administration under isoflurane anesthesia. ADU-S100 was formulated from lyophilized compound in sterile LAL water in the laminar flow hood to a concentration of 0.5 $\mu$g in 20 $\mu$L. Agent 1 peptide was formulated from lyophilized compound 24 hours prior the first administration to an initial concentration of 8.61 mg/mL and further diluted down to 2 mg/mL in 20 mM Histidine HCl 5% Mannitol pH 5. ADU-S100 and Agent 1 peptide were stored at 4°C during the study. Individual body weight was measured once every three days during the study period. Animals were dosed as described in Figure 5B and were observed and recorded for any visible clinical sign during the study period. The tumour volume were measured 3 time a week and calculated as for example 4.

*Results:*

Dosing with Agent 1 had a synergistic effect when dosed in combination with local intratumoural administration of a suboptimal amount of the STING agonist ADU-S100 compared to both monotherapy with ADU-S100 and Agent 1. This effect was particularly evident during the dosing period for ADU-S100. Also, treatment in the combination therapy group lead to more animals in clearing the tumour burden compared to both monotherapy with ADU-S100 and Agent 1. The overall survival as defined as time to reach a tumour volume of 400 $mm^3$ was also longer (just narrowly missing significance) in the treatment group which received the combination therapy with Agent 1 and ADU-S100 compared to both ADU-S100 and Agent 1 single agent. The results are shown in Figure 5.

*EXAMPLE 6: Coadministration of Agent 1 and anti-PD1 produces significant anti-tumour growth delay against CT26.WT syngeneic mouse model*

*Introduction:*

Published studies (Schreiner et al. 2004 and Garcia-Diaz et al., 2017) have found that IFN can induce expression of PD-L1 in different cancer types and STING signaling pathway is essential for the anti-tumour efficacy of PD-L1 antibody blockage. Furthermore, the combination of a STING agonist and PD-1 pathway blockage could reduce the tumour growth in mice, therefore, STING and PD-L1 have important interactions that control the cancer prognosis. Here we aimed to demonstrate if the sensitizer Agent 1 can also combined with anti-PD1. The idea is that the sensitizer may potentiate the low levels of STING agonist/activation present in cancer cells. Although this may not be sufficient to see strong anti-tumour efficacy as Agent 1 when used single agent, it may combine with anti-PD1 in resistant models such as CT26 and induce higher level of tumour growth delay/inhibition when compared to both agents dosed individually.

*Materials and methods:*

The CT26.WT cells cancer cells from CrownBio were maintained in vitro with RPMI-1640 medium supplemented with 10% fetal bovine serum at 37°C in an atmosphere of 5% $CO_2$ in the air. The cells in exponential growth phase were harvested and quantitated by cell counter before tumour inoculation. Each mouse was inoculated subcutaneously at the right front flank region with CT26.WT tumour cells ($5\times10^5$) in 0.1 mL of PBS for tumour development. The randomization was conducted after 9 days when the mean tumour size reached approximately 75 -150 $mm^3$. Sixty mice were enrolled in the for the 4 groups and the animals were randomly allocated to the study groups. The treatment was performed on the day of randomization (day 0) as per study design (Figures 6A&B). Animals were observed and body weight and tumour volumes were measured three times per week after randomization in two dimensions using a caliper, and the volume was expressed in $mm^3$ using the formula:

$$V = (L \times W \times W)/2,$$

where V was tumour volume, L was tumour length (the longest tumour dimension) and W was tumour width (the longest tumour dimension perpendicular to L).

Dosing as well as tumour and body weight measurements were conducted in a Laminar Flow Cabinet. Commercial anti-PD1 clone RMP 1-14 was used and dosed IP (6 doses). Agent 1 peptide was formulated from lyophilized compound 24 hours prior the first administration to working concentration of 4 mg/mL in 20 mM Histidine HCl, 5% Mannitol pH 5 and was dosed IV (7 doses).

*Results:*

Agent 1 administered at 10 mg/kg mouse, resulted in a slight anti-tumour efficacy against CT26.WT model, with TGI value of 21.09% on day 11, however no statistically significant difference was observed compared with control group in the Kaplan Meier analysis (Log Rank Mantel Cox test). Similarly, anti-PD1 administered at 0.2 mg/mouse, also induced a slight anti-tumour efficacy against CT26.WT model, with TGI value of 30.35% on day 11, however this was not significant compared with control group. Coadministration of anti-PD1 (0.2 mg/mouse) and Agent 1 (10 mg/kg mouse), produced significant anti-tumour efficacy against CT26.WT model, with TGI value of 46.98% on day 11, yielding a statistically significant difference of P<0.01 vs the vehicle control group. The results are shown in Figure 6.

*EXAMPLE 7: In silico immunogenicity prediction for Agent 1 and Agent 2*

*Introduction:*

Using the EpiMatrix system (EpiVax, Inc., US), peptide sequences were screened for the presence of Class II (HLA "DR") restricted HLA ligands and putative T cell epitopes. Therapeutic peptides and proteins carrying significant numbers of HLA-DR restricted T cell epitopes may be more likely to induce anti-therapeutic responses. Thus, HLA Class II restricted epitopes can be used as an effective proxy for immunogenic potential.

The core sequence (20 amino acids) of each peptide tested was provided with an EpiMatrix Immunogenicity Score and a JanusMatrix Human Homology Score.

The greater the number of HLA ligands (i.e., EpiMatrix hits) contained in a given peptide or proteins, the more likely that peptide or protein is to induce an immune response. The EpiMatrix Score is the difference between the number of predicted T cell epitopes expected in a randomly generated peptide or proteins of a given amino acid length and the number of putative epitopes predicted by the EpiMatrix System. The EpiMatrix Immunogenicity Score therefore describes the immunogenic potential of a peptide. It determines whether an input sequence carries more (+ve scores) or less (-ve scores) predicted epitope content than expected by random chance. The threshold for significance is +10. Scores above 10 suggest significant potential for immunogenicity.

In order to determine whether a given putative T cell epitope identified by EpiMatrix is homologous to predicted epitopes found within the human proteome, and thereby less likely to drive anti-therapeutic immune response, the putative epitope identified by EpiMatrix is screened against the human proteome using the JanusMatrix algorithm developed by EpiVax Inc., (Moise et al., 2013). The JanusMatrix algorithm is a screening tool for any given peptide epitope.

The JanusMatrix Human Homology Score describes the conservation of putative epitopes identified in an input sequence with epitopes derived from the balance of the human proteome. Higher scores indicate potential for homology-induced tolerance. The threshold of significance for human proteins is 3. For a given EpiMatrix Score, a high JanusMatrix Human Homology Score suggests a bias towards immune tolerance. In other words, high JanusMatrix Human Homology Scores can offset high EpiMatrix scores.

Finally, all the putative T cell epitopes identified within the core sequences of Agent 2 and Agent 1 were screened against the Immune Epitope Database (IEDB) of previously published MHC ligands and T cell epitopes.

*Results:*

Agent 2:

The core peptide sequence of Agent 2 (position 1-20) is composed of the N-terminal region derived from the IFI16 human protein. The sequence contains 15 EpiMatrix hits. The EpiMatrix Score of 21.58 is significantly above the threshold for immunogenicity (+10). No homologues to this peptide were found on the IEDB database. The JanusMatrix Human Homology Score of this peptide (2.73) falls just below the cutoff value of 3.0, suggesting no greater than average probability for homology-induced tolerance (Figure 7).

Agent 1:

The core peptide sequence of Agent 1 (position 1-20) is composed of the N-terminal region derived from the IFI16 human protein. This sequence contains ten EpiMatrix hits. The EpiMatrix Score of 10.28 is just above the standard threshold for immunogenicity (+10). No homologues to this peptide were found on the IEDB database. The JanusMatrix Human Homology Score of this peptide (3.80) is above the cutoff value of 3.0.
Relative to the Agent 1 peptide sequence, two amino acid substitutions have been introduced in the Agent 2 sequence: Y3A and Y18W. These substitutions appear to be associated with a decrease in HLA binding affinity (five predicted epitopes lost in the core amino acid sequence compared to Agent 2). Due to the overall decrease in the net putative T cell epitope content and slightly elevated human cross-conservation observed here, it is believed that these mutations reduce the immunogenic potential of the Agent 1 relative to Agent 2.
The results are shown in Figure 7.

*Summary:*

Agent 1 has two amino acid substitutions in its core sequence (Y3A and Y18W) from the human IFI16 sequence (represented by Agent 2). The *in silico* analysis screened the core sequences (position 1-20) of each agent for the presence of Class II (HLA "DR") restricted HLA ligands and putative T cell epitopes. The number of EpiMatrix hits and the EMX score were both lower in Agent 1 (10 vs 15, and 10.28 vs 21.58, respectively). The difference in JanusMatrix homology score also indicates a reduced risk of anti-therapeutic responds with Agent 1 compared to Agent 2. In summary, the results showed a reduction of the potential for immunogenicity for the core sequence of Agent 1 compared to the corresponding segment in Agent 2. The difference can be attributed to the two amino acid substitutions.

*EXAMPLE 8: In vitro immunogenicity analysis*

*Materials and methods:*

*Donor selection:*

A panel of peripheral blood mononuclear cell (PBMC) samples from healthy human donors was selected from the ProImmune Ltd (UK) cell bank. Each PBMC sample was HLA (Human Leukocyte Antigen) - typed and stored in liquid nitrogen (vapor-phase) prior to use. The panel was selected so that HLA class II alleles known to be highly expressed in the global population were well represented (data not shown). Donors were predominantly selected by DRB1 allele expression.

*DC-T cell assay:*

A Dendritic Cell - T cell (DC-T) proliferation assay (Pro!mmune Ltd) was used to identify the presence or potential T cell epitopes within the peptides. Adherent donor PBMCs were cultured with appropriate growth factors to generate monocyte-derived DCs. DCs were then loaded with a control (control 1 and control 2), the peptide (Agent 1, Agent 2, and Agent 3, see Figure 8A) or left untreated. The peptides were loaded at a final concentration of 0.34 $\mu$M. The donor cells were expected to react to the controls (70-100% for control 1 and 50-80% for control 2).
Mature antigen-loaded DCs were then co-cultured at a fixed ratio with autologous CFSE-labelled T cells in multi-well plates. Each test condition was set up in eight replicate wells and cells were incubated for 7 days. At the end of the incubation period, cell samples were stained with anti-CD4 antibody, then washed and fixed for by flow cytometric analysis. Proliferation was determined by measuring a decrease in CFSE intensity.

*Analysis:*

Data were compiled to determine antigen-induced proliferation per individual donor, calculated as Percentage Stimulation above Background. The frequency of positive donor cell responses (Percentage Antigenicity) was calculated as the proportion of responding donors out of total donors. Values were then standardized against the strength of response (an average of the Percentage Stimulation value obtained across accepted donors for each protein) to give a response index (RI).

*Determination of Percentage Stimulation above background:*

Data obtained by flow cytometric evaluation of cell samples was analyzed using FlowJo Software (Tree Star,

Inc.). The number of CFSE-dim (i.e., proliferating) cells was determined for each sample in eight replicate wells. Counts for the $CD4^+$ CFSE dim population in each sample were expressed as a proportion of the total $CD4^+$ population. The eight replicate values were then used to calculate Percentage Stimulation above Background (proportion of $CD4^+$ CFSE dim cells with antigen stimulation minus the proportion of $CD4^+$ CFSE dim cells without antigen stimulation).

A mean and standard error of the mean (SEM) of the eight values was calculated for each sample. A one-way analysis of variance (ANOVA) was also performed on the data to determine (to a significance level of $p \leq 0.05$) whether the CFSE dim population size obtained from each protein differs significantly from the unstimulated controls.

A response that has a Percentage Stimulation above Background ≥0.5%, and is also two standard errors greater than background (SEM = 2), is considered to be positive. Background threshold values are based on data from unstimulated cells for which a maximum acceptable resting proliferation threshold is established. Any assay results with background values greater than established cut-off values are discarded and do not form part of the final analysis.

*Determination of Percentage Antigenicity:*

In evaluating antigenicity, it is also appropriate to take account of the frequency of donor cell responses across the study cohort. A positive response (Percentage Stimulation above Background ≥0.5%) in 2 or more independent donor samples is considered indicative of a potential T cell epitope.

Depending on the size of the study, (20, 40 or 50 donors), two responding donors sets a threshold of 10%, 5% or 4% respectively. Thus, following calculation of Percentage Stimulation above Background, the frequency of positive donor cell responses (Percentage Antigenicity) is expressed as the proportion of responding donors out of total accepted donors screened.

*Response Index:*

When calculating the antigenicity of a protein it is important to consider the strength of a response as well as the frequency of that response. This is because some binding responses may be pan-allele, but these responses may not be very strong. The strength of positive donor cell responses was determined by taking an average of the percentage stimulation value obtained across accepted donors for each protein. Determining a Response Index (RI) through the multiplication of strength and frequency values is more representative of the level of antigenicity.

*Results:*

For both controls all 22 donors responded having 100% antigenicity (percentage stimulation above background ≥0.5%, where SEM=2) (data not shown).

A positive response in 2 or more independent donor samples is considered indicative of a potential antigen. In this study, where data from 22 donors was utilized, this sets a threshold of 9.1% antigenicity.

The Response Index values (RI) were calculated from Percentage Antigenicity values multiplied by the strength of response (i.e., the mean Percentage Stimulation above Background) values divided by 100.

When assessed at the most stringent statistical cut-offs (Percentage stimulation above background ≥0.5%, SEM = 2), the response to control 1 was 100.00% antigenicity, RI = 59.372, and the response to control 2 was 100.00% antigenicity, RI = 34.339.

At the set threshold (Percentage Stimulation above background ≥0.5%, SEM=2), the percentage antigenicity elicited by the test proteins ranged between 22.73% ("Agent 1"; RI = 0.294; "Agent 3", RI = 0.306) and 31.82% ("Agent 2"; RI = 0.445). Figures 8B and C show the RI values in tabular and corresponding graphical form for the peptides (Agent 1, Agent 2, and Agent 3), where Percentage Stimulation above >_0.5% has been set (where SEM=2).

*Summary:*

The results of the dendritic cell - T cell proliferation provide the "relative antigenicity" between the structurally similar molecules Agent 1, Agent 2, and Agent 3. Agent 1 and Agent 3 exhibited lower percentage antigenicity (22.73%) and response index (0.294 and 0.306, respectively) compared to Agent 2 (31.82% and RI = 0.445), indicating that the Y3A and Y18W amino acid substitutions from the human IFI16 sequence (represented by Agent 2) reduce the risk of the development of a $CD4^+$ T-cell mediated immune response.

*EXAMPLE 9: In vitro metabolic stability in blood*

*Materials and methods:*

Fresh blood from mouse (CD-1) and rat (Wistar) was collected from lab animals. Human blood was collected from healthy donors at local hospital or acquired from commercial vendor (BioIVT). Blood from non-human primates (Cynomolgus macaque) was acquired from commercial vendor (BioIVT).

*Incubations:*

For mouse, rat and human blood, 5.0 μg/mL of tested agent (except Agent 1 in human blood) was diluted from stock solution in DMSO to a final DMSO concentration of 0.5% and incubated in 1000 μL of blood at 37 °C, in duplicate. At 0, 5, 15, 30, and 60 minutes, 180 μL was sampled and centrifuged at 2000 g for 1 min, 80 μL supernatant was taken and diluted 1:1 with water followed by precipitation with acetonitrile containing 5% TFA (internal standard 100 nM leucine enkephalin + Glu1 fibrinopeptide B). The obtained supernatant 60 μL was further diluted with 30 μL 20% DMSO in ultra-pure water and submitted for immediate analysis by LC-MS/MS. The controls were 1 μM propantheline bromide (mouse, human) or 1 μM enalapril (rat).

Incubation of Agent 1 in blood from human and monkey was performed in a similar manner. A 25 μM solution of Agent 1 was prepared with 5% DMSO and 22.5% methanol in water. For each time point sample, four μL was added to 96 μL of fresh blood (pooled, mixed gender) from cynomolgus monkey or human, to achieve 1 μM of final concentration. Positive control samples with 2 μM propantheline was prepared in the same way. Each time point sample (0, 10, 30, 60, 120 min in duplicate) was incubated at 37°C. At the end of incubation, 100 μL water was mixed with the 100 μL spiked blood sample, then 400 μL of 1% TFA, 200 ng/mL tolbutamide and 200 ng/mL labetalol (internal standard) in acetonitrile was added to Agent 1 samples to precipitate the proteins. For positive control samples, 500 μL of 200 ng/mL tolbutamide and 200 ng/mL labetalol in acetonitrile was added. Samples were mixed thoroughly and centrifuged at 4,000 rpm for 15 min. 200 μL of supernatant was transferred to sample plates and submitted to LC-MS/MS analysis.

*Quantification:*

For all samples except Agent 1 in human and monkey blood: Separation by liquid chromatography was done with a C18 column (Phenomenex Kinetex XB-C18 (100 × 2.1 mm, 1.7 μm), with guard filter), using a gradient between 1% formic acid in water and 2% DMSO in acetonitrile. The mass spectrometry instrument (Thermo QE Orbitrap Focus) was set to positive electrospray ionization mode.

For Agent 1 samples in human and monkey blood: Separation by liquid chromatography was done with a C18 column (Waters Acquity UPLC BEH C18, 50 × 2.1 mm, 1.7 μm)), using a gradient between 2 mM ammonium formate and 0.1% formic acid in water and 0.1% formic acid in acetonitrile. The mass spectrometry instrument (Thermo QE Orbitrap Focus) was set to positive electrospray ionization mode.

*Analysis:*

The peak areas were determined against a calibration curve and the $T_0$ sample was set to 100%. The disappearance rate (k) was calculated and the half-life for each incubation was determined (t½ = ln2/k).

*Results:*

The metabolic stability in fresh blood from mouse, rat, human and NHP (Agent 1 only) is presented as the half-lives (T½) in Figure 9. Agent 1 exhibits better stability compared to Agent 2 and Agent 3 in all species.

*Summary:*

The addition of a fatty acid derivative in the N' terminus of Agent 1 considerably reduces the metabolism in fresh blood from mouse, rat and human.

*EXAMPLE 10: Pharmacokinetic profile and parameter for peptides after intravenous administration*

*Materials and methods:*

Test agents were formulated in 2 % (v/v) propylene glycol in 25 mM HEPES, pH 7.4 and administered with a

bolus injection at 10.0 mg/kg via the tail vain to male CD-1 mice (Charles River Laboratories) or male Wistar rats (Charles River Laboratories), or with a 30-min intravenous infusion at 4 mg/kg to male cynomolgus monkeys.

Blood samples were collected at 2.5, 5, 10, 30, 60, 120, 240 and 480 minutes after administration (mouse and rat), or at 5, 15, 30, 60, 120, 240, 360, 480 and 1440 min (Agent 1) or at 5, 15, 30, 45, 60, 90, 150, 240 and 480 min (Agent 2) after the start of infusion in monkeys. Samples were immediately mixed with a protease inhibitor cocktail (Roche cOmplete™ EDTA-free) before being cooled and centrifuged for plasma separation. Plasma samples were frozen and stored at -20 °C until analysis.

*Sample preparation, mouse and rat:*

To rat samples, blank rat plasma was added up to a volume of 100 μl. To mouse samples, blank mouse plasma was added up to a volume of 300 μl. To plasma samples, 50 μl of internal standard solution (in 50% DMSO:HEPES 50 mM) and 300 μl (mouse samples) or 100 μl (rat samples) of precipitation solution (52% acetonitrile + 5% trifluoro acetic acid) were added with vortexing after each addition. Samples were then centrifuged (20 min at 4000 g) and 350 μl (mouse samples) or 150 μl (rat samples) of supernatant was diluted with 650 μl of 1% formic acid for solid phase extraction. Solid phase extraction plates (Oasis HLB elution plate) were preconditioned with 350 μl of methanol, followed by 350 μl of 1% formic acid (Waters Positive Pressure-96). Mouse samples (1 ml) or rat samples (700 μl) were applied to the extraction plate in three parts (4psi, 1 min and then 6psi, 30s). Extraction plate was washed twice with 300 μl of 0.5% formic acid in 10% methanol (6psi until dry). Samples were eluted form the extraction plate twice with 40 μl of 65% acetonitrile/35% $H_2O$ with 1% formic acid (3psi, 30s) directly to 96 well plate for MS analysis.

Standard plasma samples were prepared by spiking blank mouse or rat plasma to obtain concentrations from 4 to 20000 ng/ml in plasma by using one volume of spiking solution and nine volumes of plasma. These samples were then prepared for analysis similarly as the actual samples. Similarly, quality control (QC) samples were prepared to concentrations 25, 125, 1250 and 5000 ng/ml.

*Agent 1 sample preparation, monkey:*

The sample preparation was performed on wet ice. 100 μL unknown sample, calibration standard, or control was quenched by mixing with 200 μL internal standards in acetonitrile with 1% TFA and then centrifuged for 15 min at 12000 g, 4 °C. 50 μL supernatant was transferred to a 96-well plate and centrifuged for 5 min at 3220 g, 4 °C. The supernatant was subsequently injected for LC-MS/MS analysis.

*Agent 2 sample preparation, monkey:*

Internal standards were dissolved in DMSO/50 mM HEPES in $H_2O$ (50/50, v/v), with further dilutions in DMSO/50 mM HEPES in $H_2O$ (50/50, v/v) with 100 nM Leucine Enkephalin and spiked at a concentration of 1.5 μg/mL. Control monkey plasma or test sample or calibration standards/QC (50 μL) were aliquoted to the bottom of a 96 well Lo-bind plate extraction carried out on iced water. 20 μL of internal standard to calibration standards, QCs, single blanks and test samples (20 μL 100 nM Leucine Enkephalin in DMSO/50 mM HEPES (50/50, v/v) to double blanks). 150 μL of ACN/$H_2O$/HCOOH (80,20/1, v/v/v) was added to all wells and the plate was vortex mixed and centrifuged for 5 minutes, 2400 x g at a temperature set to maintain 4°C. Using a pipetting robot, 100 μL of supernatant was aliquoted to a clean 96 well Lo-bind plate, followed by an addition of 100 μL of $H_2O$ to all wells. The plate was then vortex mixed and centrifuged for 5 minutes, 2400 x g at a temperature set to maintain 4°C prior to analysis.

*Liquid chromatography-mass spectrometry:*

Mouse and rat samples: Separation was done with a C18 column (Phenomenex Kinetex XB-C18 (100 x 2.1 mm, 1.7 μm)), using a gradient between 1% formic acid in water and 2% DMSO in acetonitrile (Agent 1) or acetonitrile (Agent 2). The mass spectrometry instrument (Waters TQ-S triple quadrupole MS) was set to positive electrospray ionization mode. The range of quantitation was 4- 20,000 ng/mL.

Monkey samples, Agent 1: Separation was done with a C18 column (Waters Acquity UPLC BEH C18 (50 x 2.1mm, 1.7 μm)), using a gradient between 0.1% formic acid in water and 0.1% formic acid in acetonitrile. The mass spectrometry instrument (Sciex Triple Quad 6500 Plus) was set to positive electrospray ionization mode. The range of quantitation was 10- 3,000 ng/mL.

Monkey samples, Agent 2: Separation was done with a C18 column (Waters Acquity CSH C18 (50 x 2.1 mm, 1.7 μm)), using a gradient between 0.2% formic acid in acetonitrile and 0.2% formic acid in water. The mass spectrometry instrument (Sciex Triple Quad 6500 Plus) was set to positive electrospray ionization mode. The range of quantitation was 10- 10,000 ng/mL.

*Pharmacokinetic analysis:*

Non-compartmental PK analysis on plasma concentration versus time data was analyzed by non-compartmental approaches using the WinNonlin software program (version 6.3 or above, Pharsight, Mountain View, CA). Average plasma concentrations were used form mouse and rat (composite PK sampling).

*Results:*

The pharmacokinetic profiles of Agent 1 and Agent 2 after intravenous administration to mouse, rat and cynomolgus monkey, and key pharmacokinetic parameters are presented in Figures 10A-10C.

*Summary:*

The pharmacokinetic properties of Agent 1 result in approximately 32-, 27- or 13-fold increase in exposure ($AUC_{0\text{-}inf}$) compared to Agent 2, at equal dose, which is consistent with the differences in *in vitro* metabolic stability between the two agents.

***References***

A number of publications are cited above in order to more fully describe and disclose the invention and the state of the art to which the invention pertains. Full citations for these references are provided below. The entirety of each of these references is incorporated herein.


Jansson, K., Laustsen, A., Krapp, C. et al. IFI16 is required for DNA sensing in human macrophages by promoting production and function of cGAMP. Nat Commun 8, 14391 (2017). https://doi.org/10.1038/ncomms14391

Lättig-Tünnemann, G. et al. Backbone rigidity and static presentation of guanidinium groups increases cellular uptake of arginine-rich cell-penetrating peptides. Nat. Commun. 2:453 doi: 10.1038/ncomms1459 (2011).

Nischan et al., Covalent Attachment of Cyclic TAT Peptides to GFP Results in Protein Delivery into Live Cells with Immediate Bioavailability. Angew. Chem. Int. Ed. 2015, 54, 1950 - 1953.

Moise L et al. The two-faced T cell epitope: examining the host-microbe interface with JanusMatrix. Hum Vaccin Immunother. 2013 Jul;9(7):1577-86

For standard molecular biology techniques, see Sambrook, J., Russel, D.W. Molecular Cloning, A Laboratory Manual. 3 ed. 2001, Cold Spring Harbor, New York: Cold Spring Harbor Laboratory Press

For common procedures in solid-phase peptide synthesis using Fmoc protecting groups (Fmoc-SPPS), see also: "Fmoc Solid Phase Synthesis - A Practical Approach", Edited by W. C. Chan and P. D. White, Oxford University Press, 2000.

Amouzegar et al., STING Agonists as Cancer Therapeutics. Cancers 2021, 13(11), 2695.

Lee et al. Improvement of STING-mediated cancer immunotherapy using immune checkpoint inhibitors as a game-changer. Cancer Immunol Immunother. 2022 Dec;71(12):3029-3042.

Cañadas et al., Tumor innate immunity primed by specific interferon-stimulated endogenous retroviruses. Nat. Med. 24, 1143-1150 (2018).

Falahat, et al., Epigenetic reprogramming of tumor cell: Intrinsic STING function sculpts antigenicity and T cell recognition of melanoma. Proc. Natl. Acad. Sci. U.S.A. 118, e2013598118 (2021).

Qian et al., Discovery and Mechanism of Highly Efficient Cyclic Cell-Penetrating Peptides. Biochemistry. 2016 May 10;55(18):2601-12.

Schreiner B, Mitsdoerffer M, Kieseier BC, Chen L, Hartung HP, Weller M, Wiendl H. Interferon-beta enhances monocyte and dendritic cell expression of B7-H1 (PD-L1), a strong inhibitor of autologous T-cell activation: relevance

for the immune modulatory effect in multiple sclerosis. J Neuroimmunol. 2004 Oct;155(1-2):172-82.

Garcia-Diaz A, Shin DS, Moreno BH, Saco J, Escuin-Ordinas H, Rodriguez GA, Zaretsky JM, Sun L, Hugo W, Wang X, Parisi G, Saus CP, Torrejon DY, Graeber TG, Comin-Anduix B, Hu-Lieskovan S, Damoiseaux R, Lo RS, Ribas A. Interferon Receptor Signaling Pathways Regulating PD-L1 and PD-L2 Expression. Cell Rep. 2017 May 9;19(6):1189-1201.

Zatreanu, D., Robinson, H.M.R., Alkhatib, O. et al. Polθ inhibitors elicit BRCA-gene synthetic lethality and target PARP inhibitor resistance. Nat Commun 12, 3636 (2021).

Zhou, J., Gelot, C., Pantelidou, C. et al. A first-in-class polymerase theta inhibitor selectively targets homologous-recombination-deficient tumors. Nat Cancer 2, 598-610 (2021).

Rodriguez-Berriguete G, Ranzani M, Prevo R, Puliyadi R, Machado N, Bolland HR, Millar V, Ebner D, Boursier M, Cerutti A, Cicconi A, Galbiati A, Grande D, Grinkevich V, Majithiya J, Piscitello D, Rajendra E, Stockley M, Boulton SJ, Hammond EM, Heald R, Smith GCM, Robinson H, Higgins GS. Small-molecule Polθ inhibitors provide safe and effective tumor radiosensitization in preclinical models. Clin Cancer Res. 2023 Jan 23:CCR-22-2977.

## Claims

1. An agent comprising an amino acid sequence, wherein the amino acid sequence comprises KKAKNIVLLKGLEVINDWHF (SEQ ID NO: 1).

2. The agent according to claim 1, wherein the agent has the formula:

X-Y-Z

wherein

X is a half-life extending moiety;
Y is the amino acid sequence comprising SEQ ID NO: 1; and
Z is a membrane transit moiety.

3. The agent according to claim 2, wherein the membrane transit moiety is a cell penetrating peptide (CPP).

4. The agent according to claim 3, wherein the CPP is a cyclic CPP.

5. The agent according to claim 4, wherein the cyclic CPP is cyclo[CrRrGrKkRrC] (SEQ ID NO: 2).

6. The agent according to any one of claims 2 to 5, wherein the half-life extending moiety is a peptide or polypeptide, a carbohydrate molecule, a synthetic polymer, or a lipophilic substituent.

7. The agent according to claim 6, wherein the lipophilic substituent is 15-carboxypentadecanoyl-Adoa-Adoa, wherein "Adoa" is 8-amino-3,6-dioxaoctanoic acid.

8. A composition comprising the agent according to any one of claims 1 to 7, optionally wherein the composition further comprises:

(i) a STING stimulating agent;
(ii) a DNA damage inducing agent; and/or
(iii) a checkpoint inhibitor.

9. The composition according to claim 8, wherein the DNA damage inducing agent is a PARP inhibitor, an ATM inhibitor, a topoisomerase inhibitor, a DNA crosslinking agent, or a radionuclide.

10. An agent according to any one of claim 1 to 7 for use in therapy.

**11.** An agent according to any one of claim 1 to 7 for use in a method of treating cancer.

**12.** The agent for use according to claim 10 or claim 11, wherein the method further comprises administering:

(i) a STING stimulating agent;
(ii) a DNA damage inducing agent; and/or
(iii) a checkpoint inhibitor.

**13.** The agent for use according to claim 12, wherein the (i) STING stimulating agent, (ii) the DNA damage inducing agent and/or (iii) the checkpoint inhibitor is administered simultaneously or sequentially.

**14.** The agent for use according to claim 12 or claim 13, wherein the DNA damage inducing agent is a PARP inhibitor, an ATM inhibitor, a topoisomerase inhibitor, a DNA crosslinking agent, or a radionuclide.

**15.** The agent for use according to any one of claims 10 to 14, wherein the agent is administered systemically.

*Figure 1*

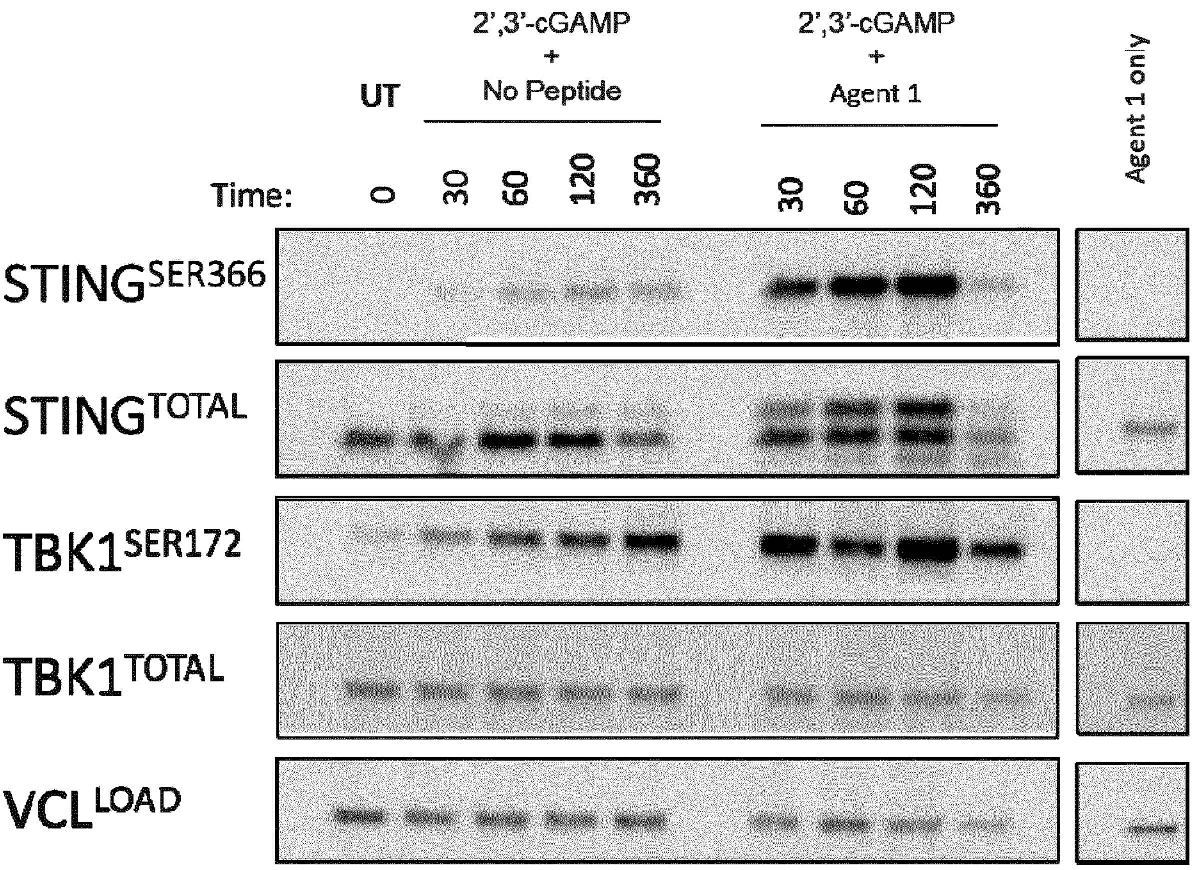

2',3'-cGAMP + No Peptide
2',3'-cGAMP + Agent 1
UT
Agent 1 only
Time:
0
30
60
120
360
30
60
120
360
STING SER366
STING TOTAL
TBK1 SER172
TBK1 TOTAL
VCL LOAD

*Figure 2*

A

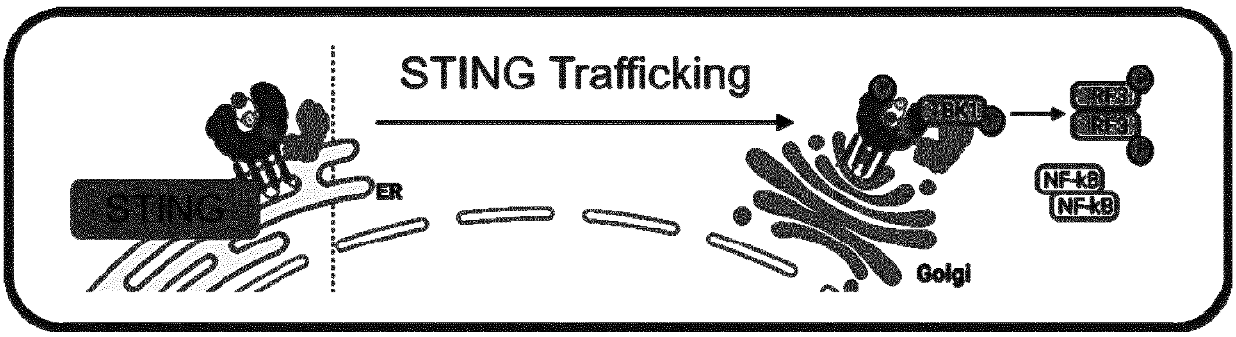

*Figure 2 (continued)*

B

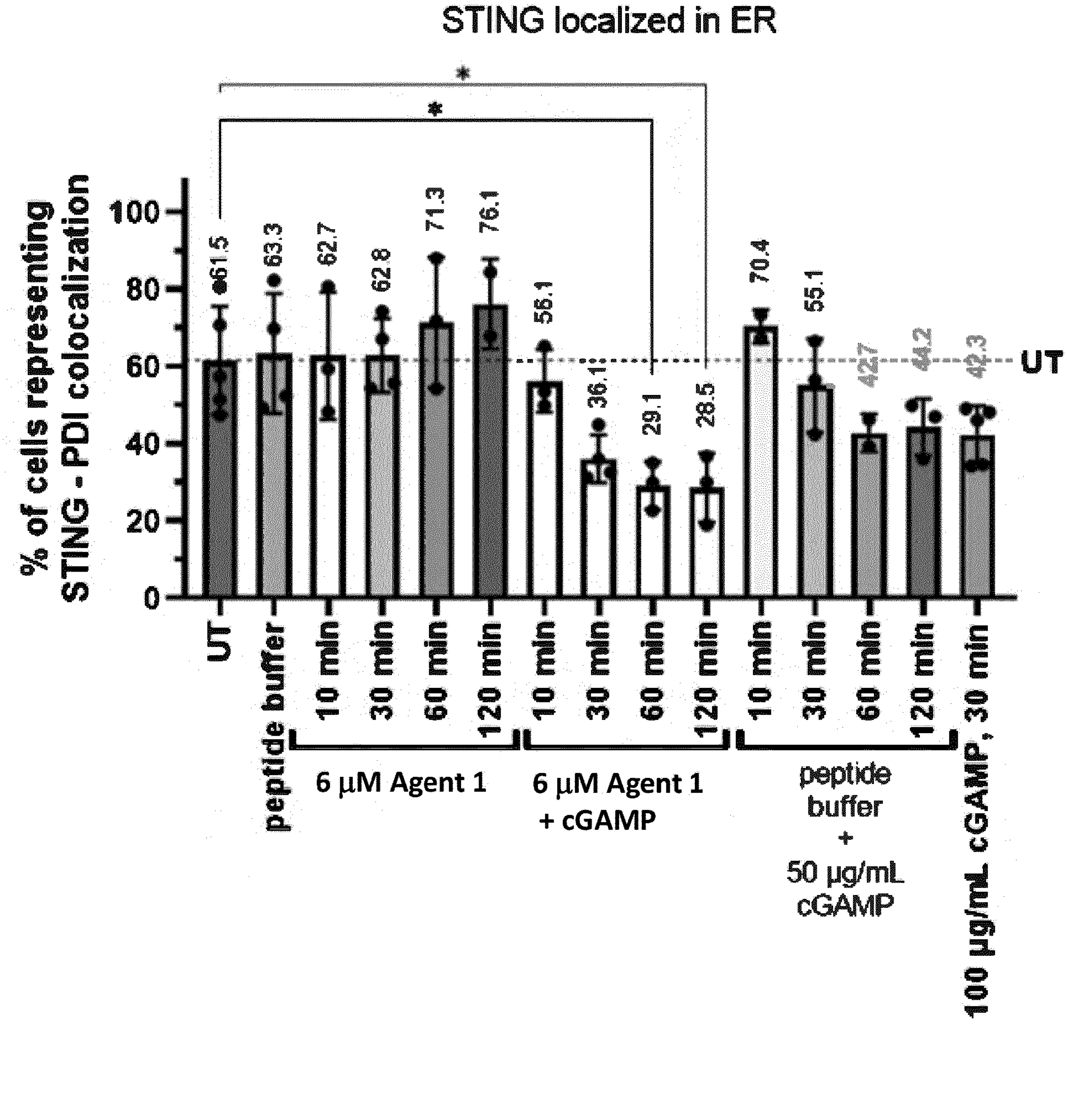

*Figure 2 (continued)*

C

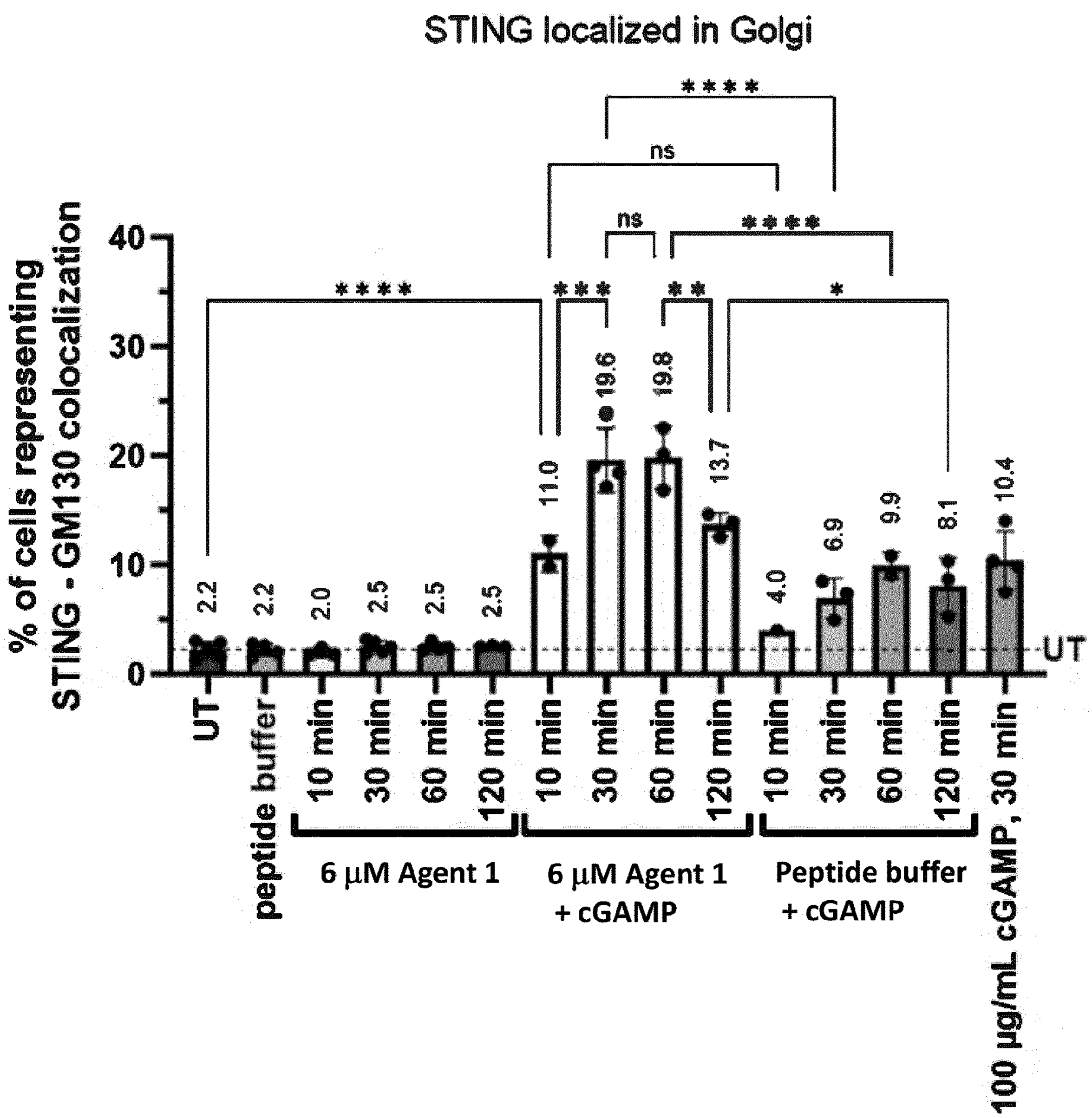

## *Figure 3*

**A**

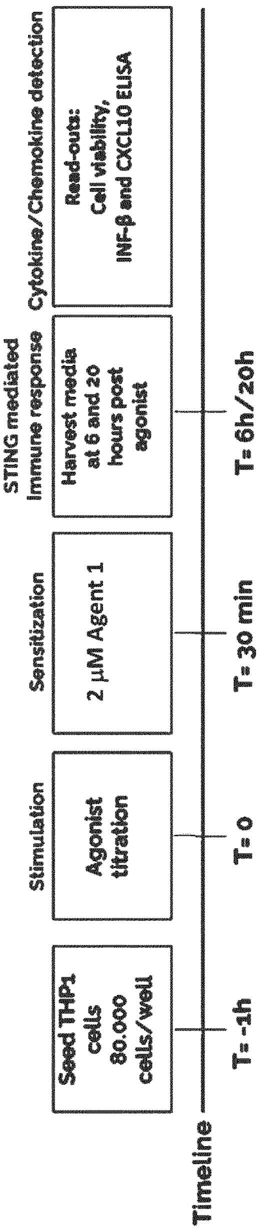
Timeline
Seed THP1 cells 80.000 cells/well
T= -1h
Stimulation
Agonist titration
T= 0
Sensitization
2 μM Agent 1
T= 30 min
STING mediated Immune response
Harvest media at 6 and 20 hours post agonist
T= 6h/20h
Cytokine/Chemokine detection
Read-outs: Cell viability, INF-β and CXCL10 ELISA

## *Figure 3 (continued)*

B

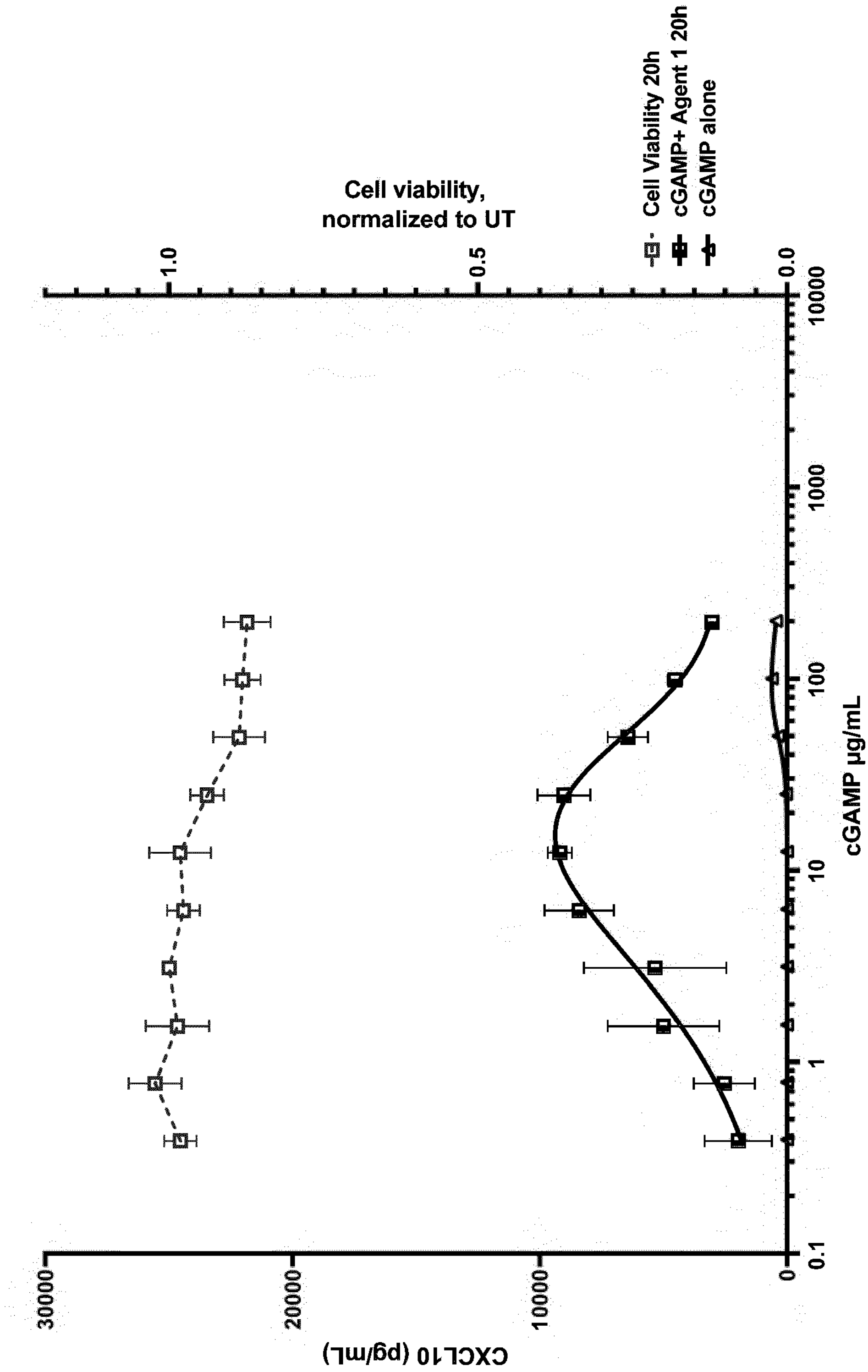

## *Figure 3 (continued)*

C

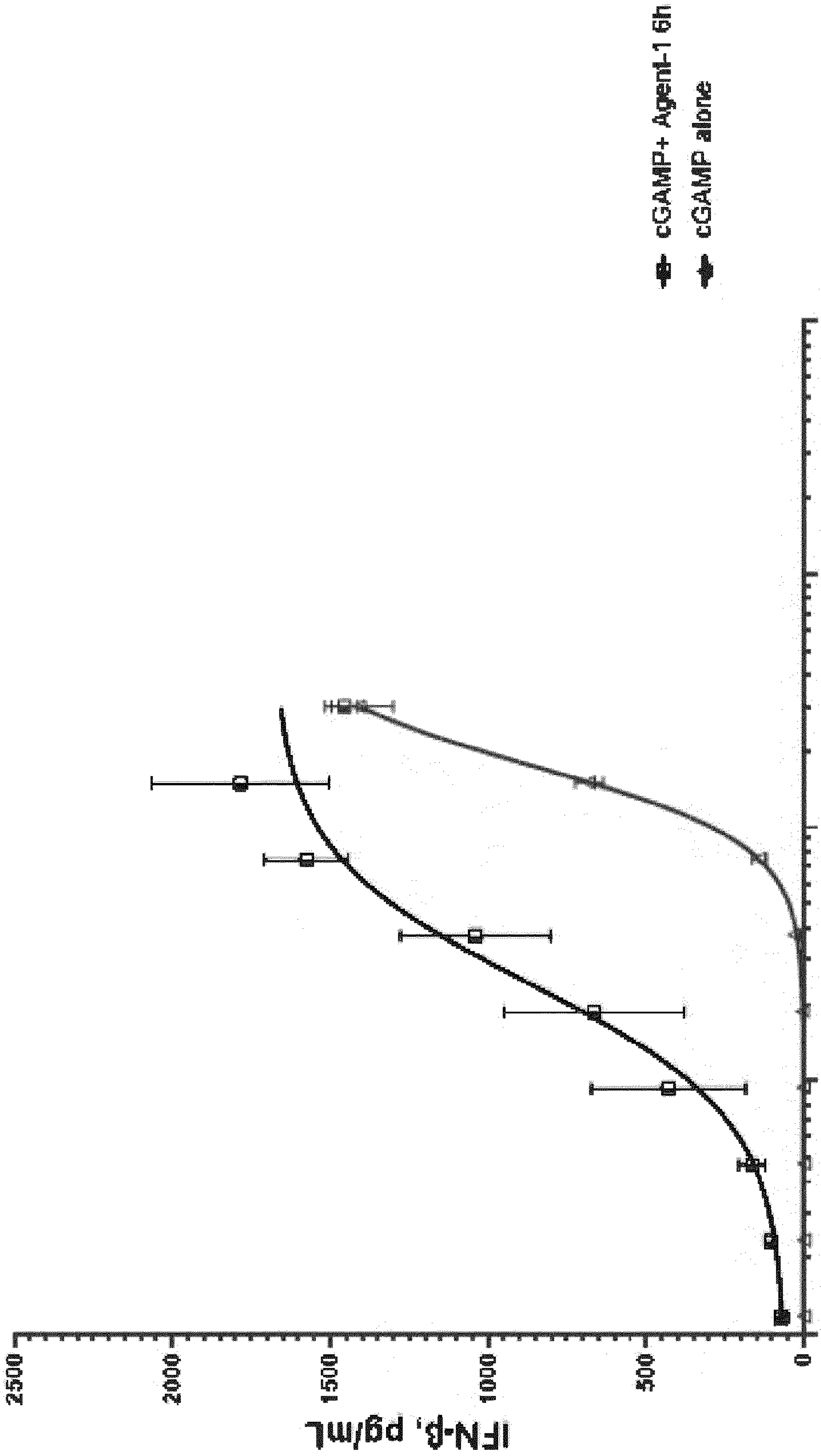

# *Figure 3 (continued)*

D

| 2´3´-cGAMP (n=4) | CXCL10 20h | CXCL10 20h (+ 2 µM Agent 1) | IFN-β 6h | IFN-β 6h (+ 2 µM Agent 1) |
|---|---|---|---|---|
| $EC_{50}$ (µg/mL) | 29.8 ± 16 | 2.7 ± 1.2 | nd | 25.3 ± 23.7 |
| $E_{Max}$ (pg/mL) | 2169 ± 1774 | 21459 ± 11073 | 1633 ± 608 | 1949 ± 1729 |

E

| diABZI C3 (n=4) | CXCL10 20h | CXCL10 20h (+ 2 µM ST317) | IFN-β 6h | IFN-β 6h (+ 2 µM ST317) |
|---|---|---|---|---|
| $EC_{50}$ (µM) | 0.064 ± 0.022 | 0.036 ± 0.007 | 0.40 ± 0.13 | 0.71 ± 0.51 |
| $E_{Max}$ (pg/mL) | 3007 ± 1360 | 20053 ± 9462 | 688 ± 630 | 982 ± 753 |

| ADU-S100 (n=2) | CXCL10 20h | CXCL10 20h (+ 2 µM ST317) | IFN-β 6h | IFN-β 6h (+ 2 µM ST317) |
|---|---|---|---|---|
| $EC_{50}$ (µM) | 6.48 ± 0.12 | 4.45 ± 2.96 | 59.7 ± 14 | 21.8 ± 11.3 |
| $E_{Max}$ (pg/mL) | 2925 ± 1401 | 33880 ± 18327 | 4477 ± 4062 | 3063 ± 2105 |

*Figure 4*

A

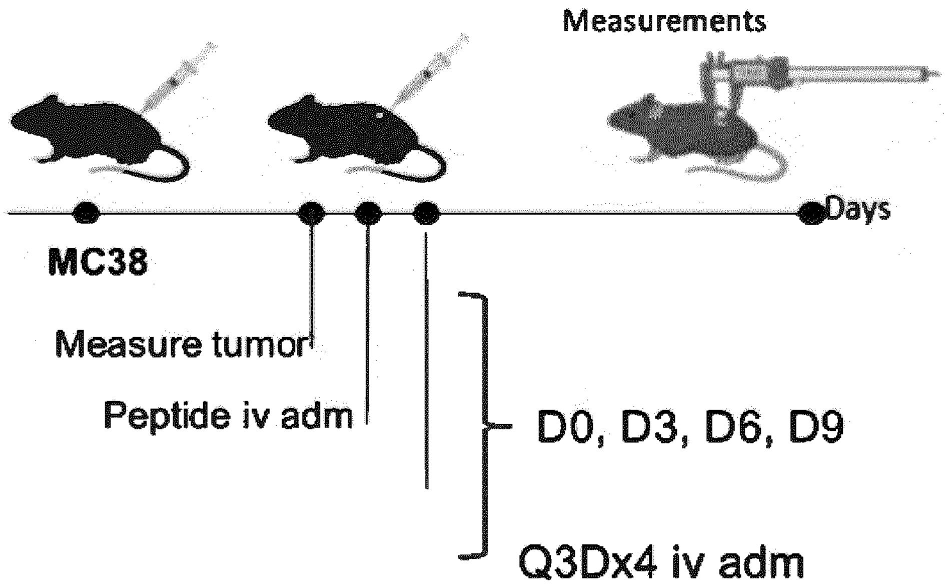

Measurements
Days
MC38
Measure tumor
Peptide iv adm
D0, D3, D6, D9
Q3Dx4 iv adm


B

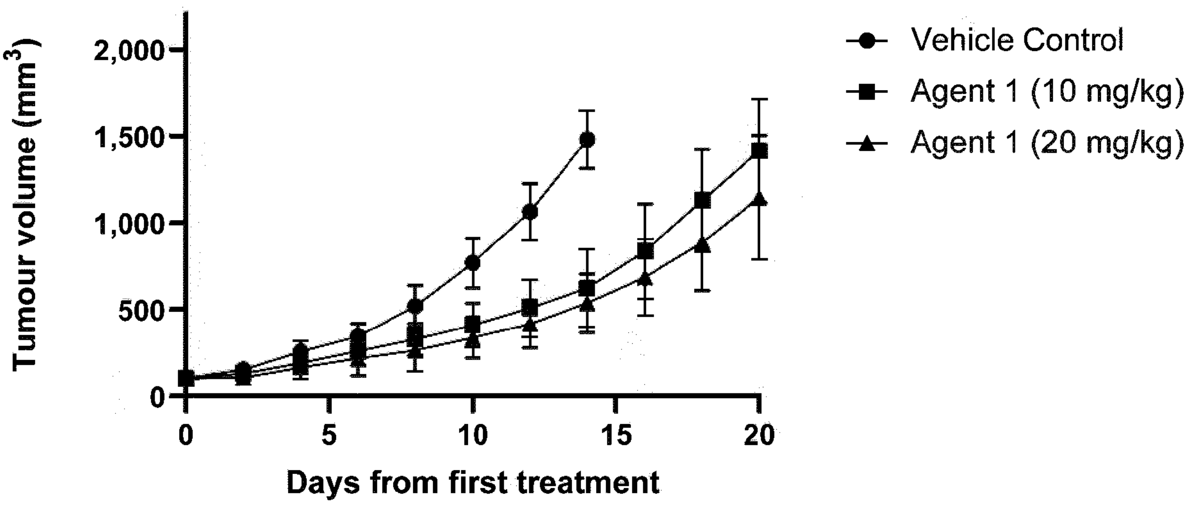

Tumour volume (mm3)
2,000
1,500
1,000
500
0
0
5
10
15
20
Days from first treatment
Vehicle Control
Agent 1 (10 mg/kg)
Agent 1 (20 mg/kg)

*Figure 4 (continued)*

C

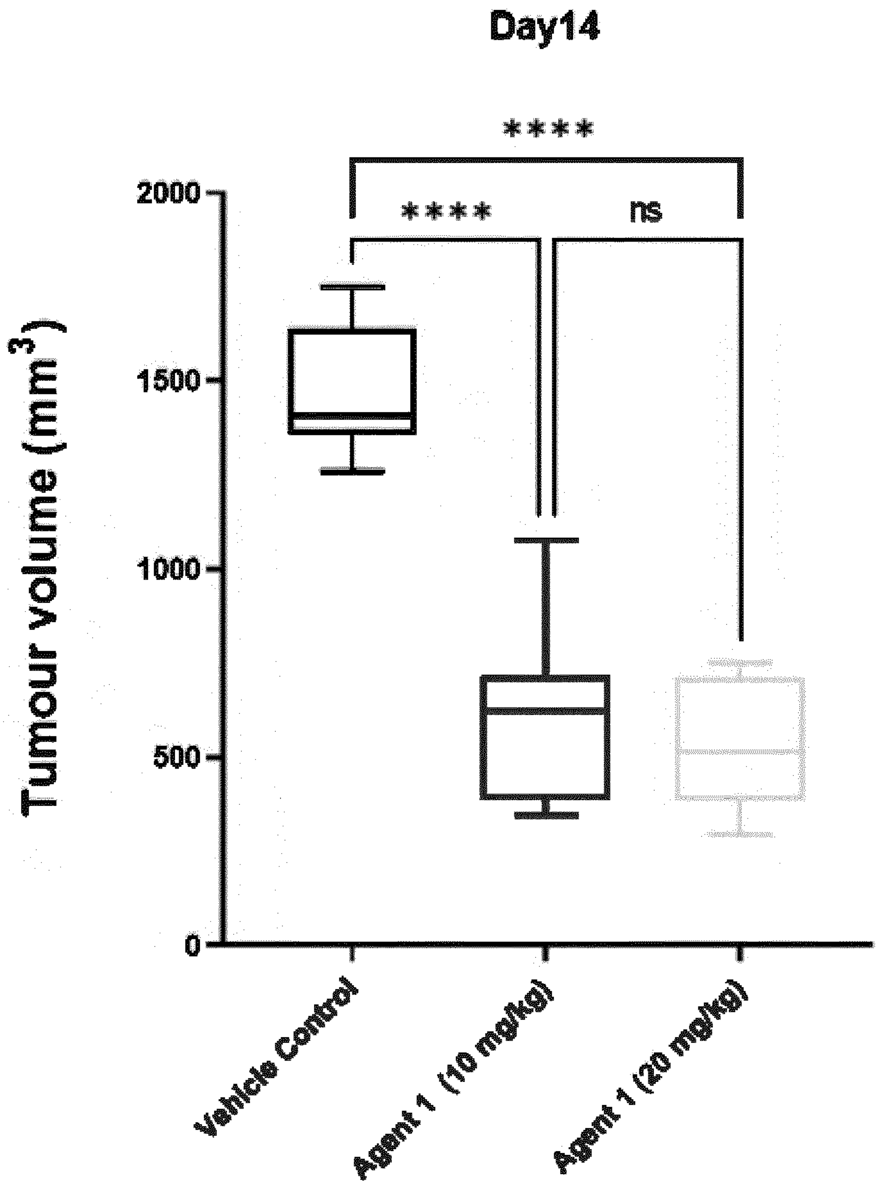

*Figure 5*

**A**

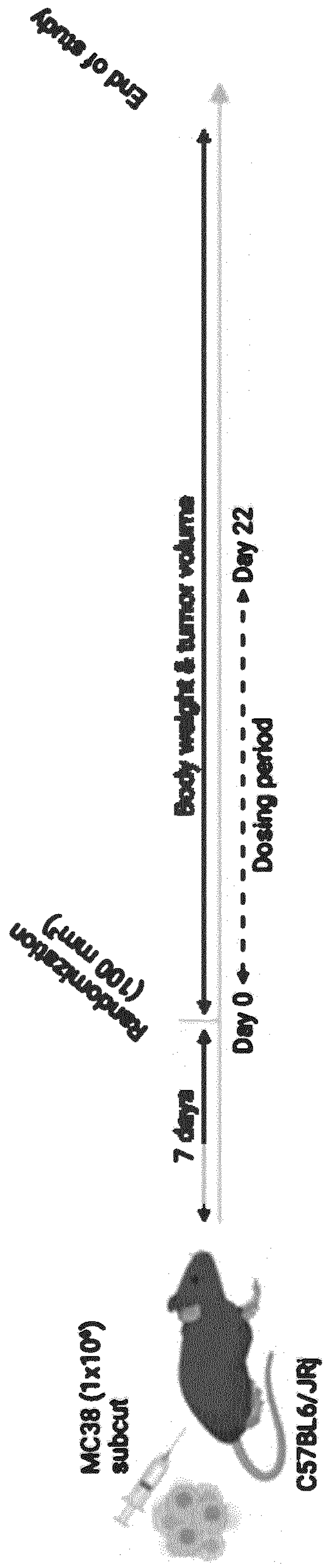
End of study
Body weight & tumor volume
Randomization $(100\ mm^3)$
7 days
Day 0
Dosing period
Day 22
MC38 $(1x10^6)$ subcut
C57BL6/JRj

## *Figure 5 (continued)*

B

| Group | Mice (n) | Treatment | Dose level | ROA | Dosing schedule |
|---|---|---|---|---|---|
| 1 | 12 | Vehicle (saline) | N/A | i.t. | Q3Dx4 |
| 2 | 11 | ADU-S100 | 0.5 µg | i.t. | Q3Dx4 |
| 3 | 12 | Agent 1 | 10 mg/kg BW | i.v.<br>(saline i.t.) | Q3Dx6 |
| 4 | 11 | ADU-S100<br>+ Agent 1 | 0.5 µg<br>10 mg/kg BW | i.t.<br>i.v. | Q3Dx4<br>Q3Dx6 |

## *Figure 5 (continued)*

C

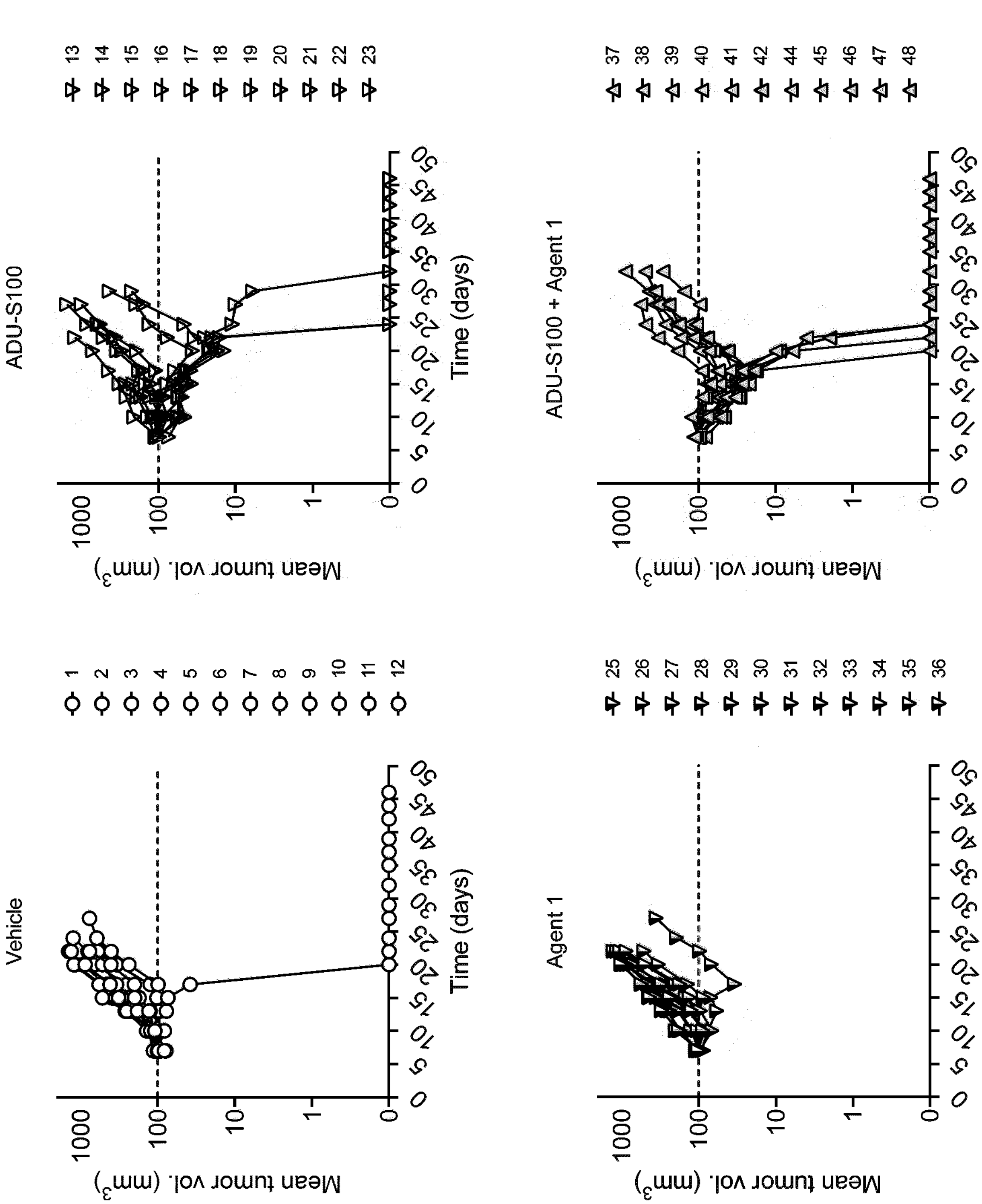

*Figure 5 (continued)*

D

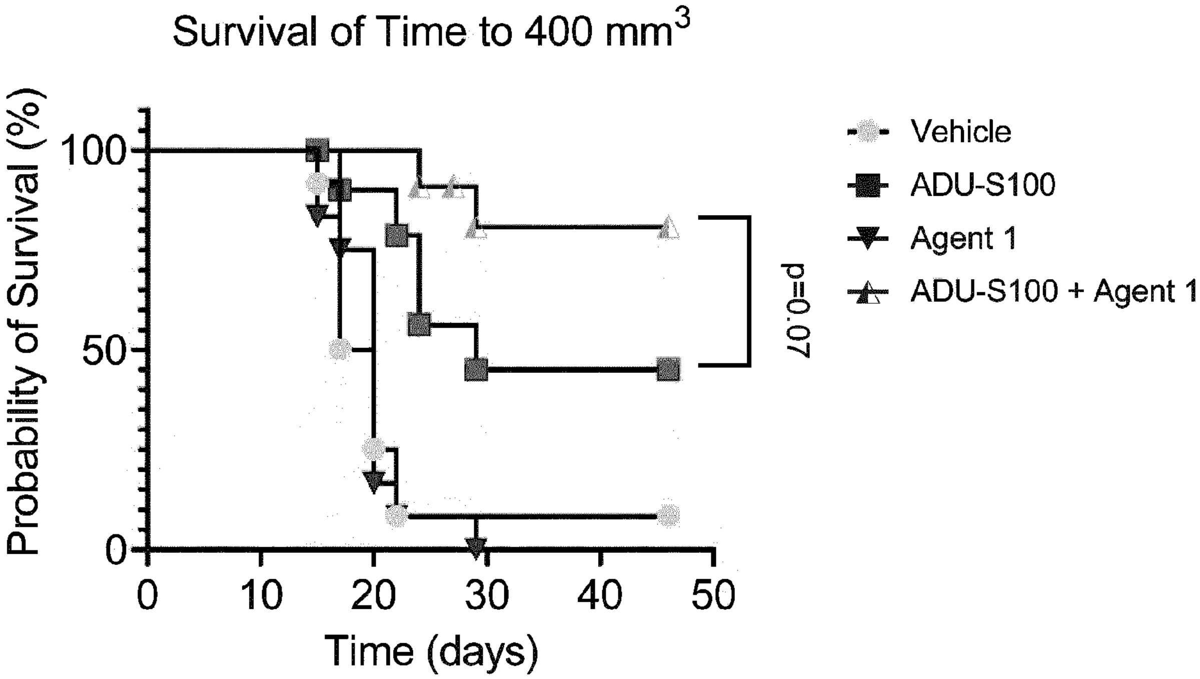


| | Vehicle | ADU-S100 | Agent 1 | ADU-S100 + Agent 1 |
|---|---|---|---|---|
| Median survival | 18.5 | 29 | 20 | Undefined |

*Figure 6*

**A**

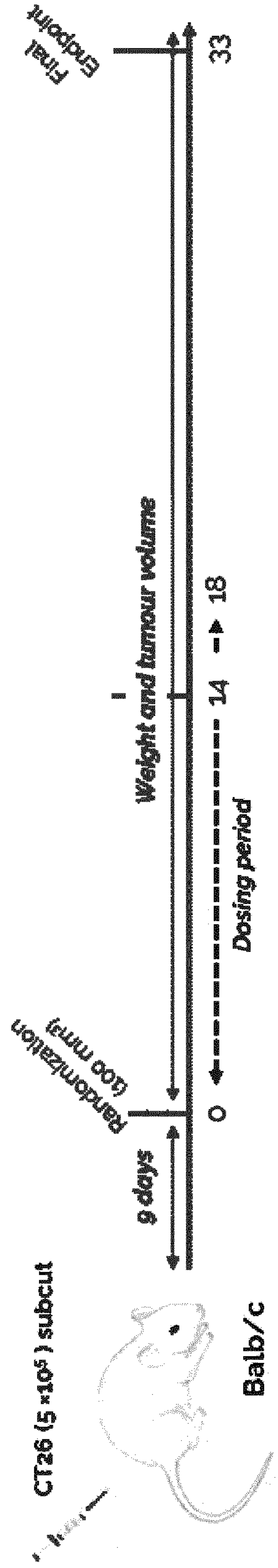
CT26 (5 ×10^5) subcut
Balb/c
9 days
Randomization (100 mm3)
Weight and tumour volume
Final Endpoint
0
14
18
33
Dosing period

# *Figure 6 (continued)*

**B**

| Group | Mice No. | Treatment | Dose Level | Dosing Volume | ROA | Dosing Frequency & Duration |
|---|---|---|---|---|---|---|
| 1 | 15 | Vehicle | N/A | 5 ml/kg | *i.p.* | BIW × 3 weeks (day 0, 3, 7, 10, 14, 17) |
| 2 | 15 | a-PD-1 | 0.2 mg flat dose | 0.2 ml | *i.p.* | BIW × 3 weeks (day 0, 3, 7, 10, 14, 17) |
| 3 | 15 | Agent-1 | 10 mg/kg | 5 ml/kg | *i.v.* | Q3D × 7 doses (day 0, 3, 6, 9, 12, 15, 18) |
| 4 | 15 | a-PD-1 | 0.2 mg/mouse | 0.2 ml | *i.p.* | BIW × 3 weeks (day 0, 3, 7, 10, 14, 17) |
| | | Agent-1 | 10 mg/kg | 5 ml/kg | *i.v.* | Q3D × 7 doses (day 0, 3, 6, 9, 12, 15, 18) |

# *Figure 6 (continued)*

C

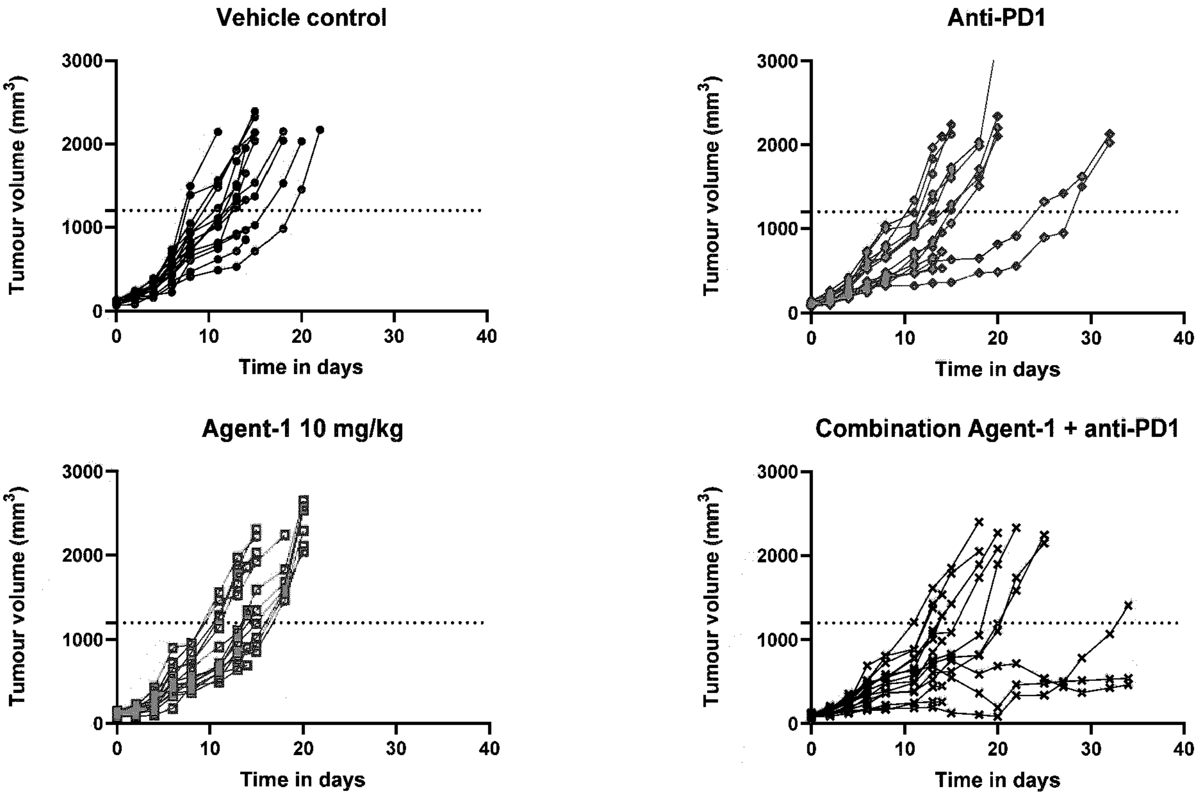

# *Figure 6 (continued)*

**D**

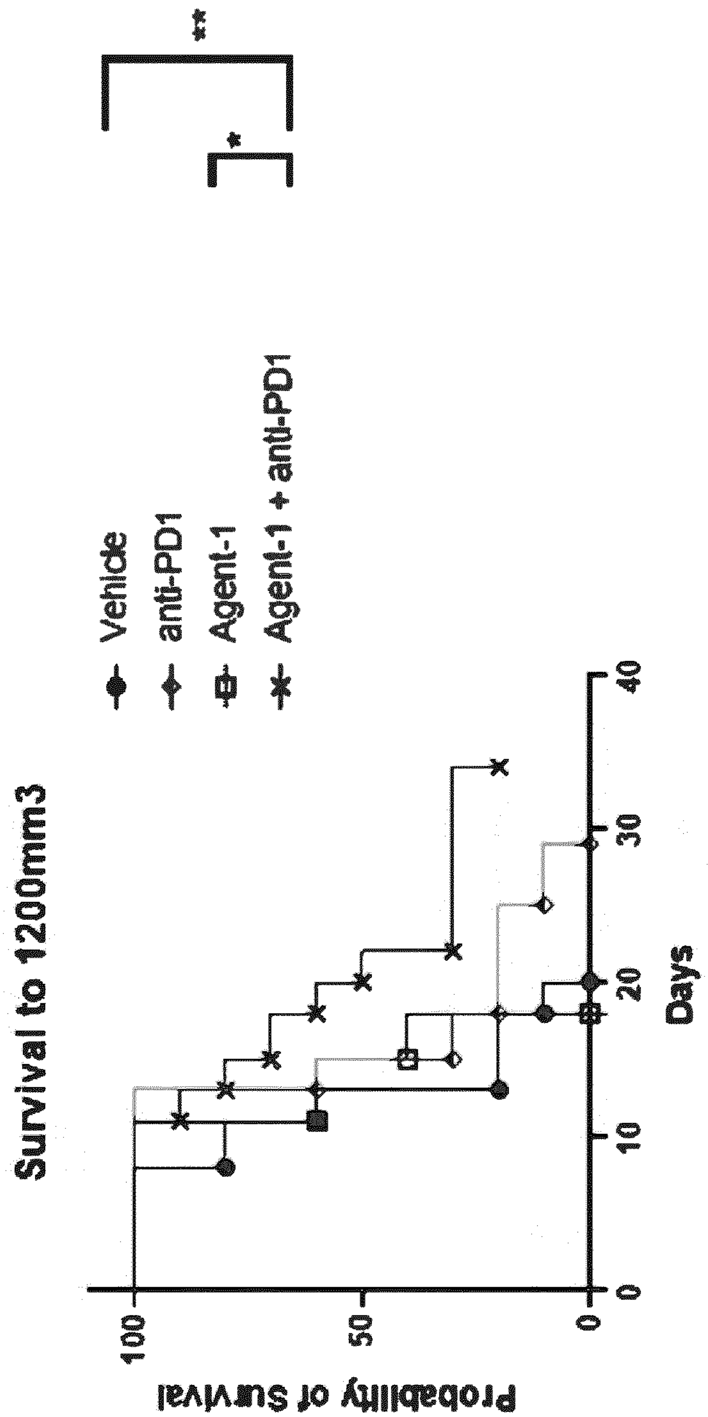


| | Vehicle | anti-PD1 | Agent-1 | Agent-1 + anti-PD1 |
|---|---|---|---|---|
| Median survival | 13 | 15 | 15 | 21 |

***Figure 7***

| Input Name | AA Sequence | EMX Hits | EMX Score | JMX HMLGY Score | Hits in IEDB |
|---|---|---|---|---|---|
| Agent 1_core | KKAKNIVLLKGLEVINDWHF | 10 | 10.28 | **3.80** | 0 |
| Agent 2_core | KKYKNIVLLKGLEVINDYHF | 15 | 21.58 | 2.73 | 0 |

## *Figure 8*

**A**

| Protein ID | Structure | Mw (g/mol) |
|---|---|---|
| **Agent 1** | C16d-Adoa-Adoa-KKAKNIVLLKGLEVINDWHF-c[CrRrGrKkRrC]-$NH_2$ | 4378.4 |
| **Agent 2** | KKYKNIVLLKGLEVINDYHFGRKKRRQRRRPQ-$NH_2$ | 4037.8 |
| **Agent 3** | KKAKNIVLLKGLEVINDWHF-c[CrRrGrKkRrC]-$NH_2$ | 3819.7 |

**B**

| Percentage Stimulation above ≥0.5%, SEM=2 | | | |
|---|---|---|---|
| **Protein ID** | **Percentage Antigenicity** | **Strength of Response (Mean %Stimulation)** | **Response Index (RI)** |
| Agent 1 | 22.73 | 1.29 | 0.294 |
| Agent 3 | 22.73 | 1.34 | 0.306 |
| Agent 2 | 31.82 | 1.40 | 0.445 |

*Figure 8 (continued)*

C

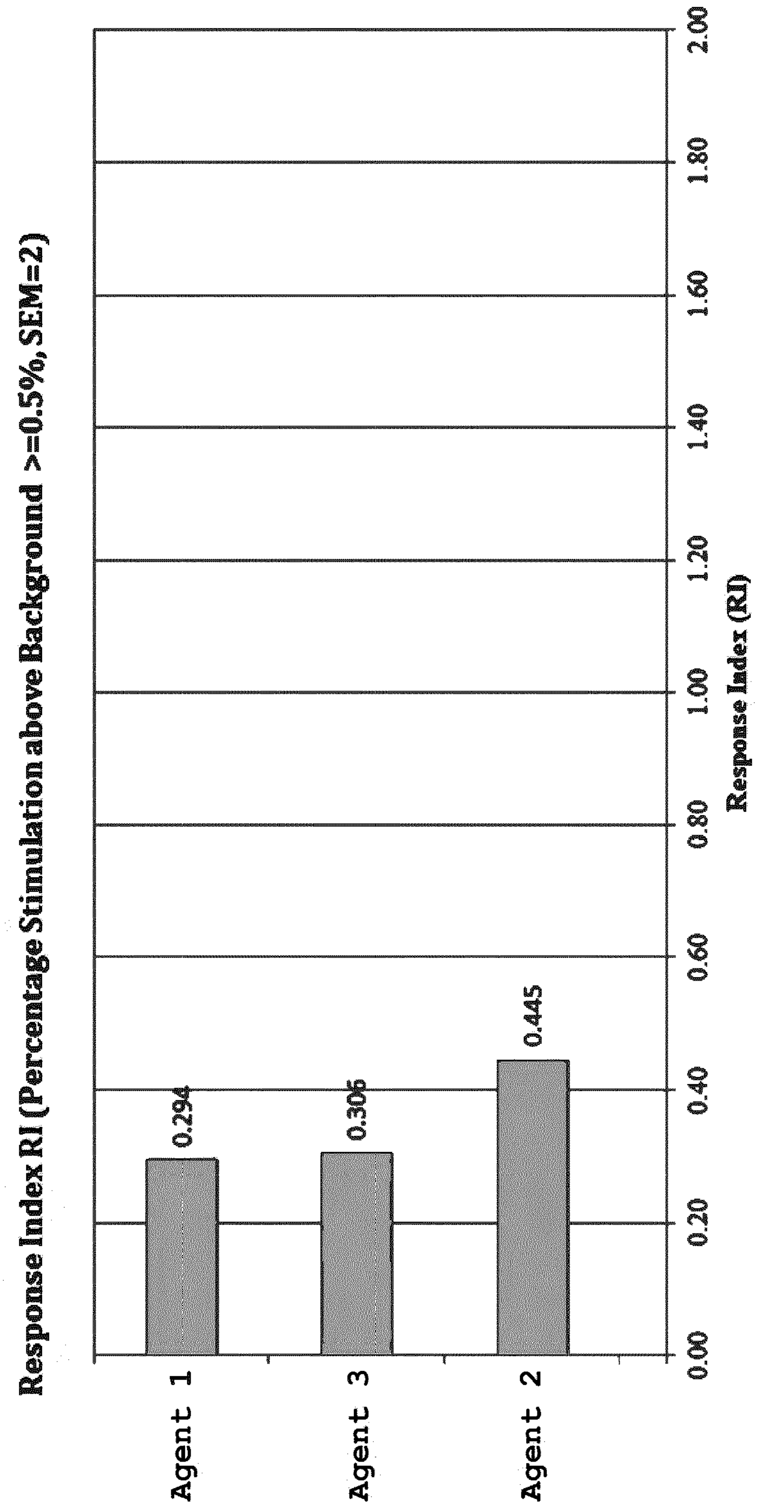

## *Figure 9*

| **Blood (37 °C)** | **Agent 2 T½ (min)** | **Agent 1 T½ (min)** | **Agent 3 T½ (min)** |
|---|---|---|---|
| Mouse (@ 5.0 µg/mL) | 4 | 174 | 11 |
| Rat (@ 5.0 µg/mL) | 3 | 86 | 8 |
| NHP (@ 4.4 µg/mL) | nd | 172 | nd |
| Human | 50 (@ 5.0 µg/mL) | 127 (@ 4.4 µg/mL) | 60 (@ 5.0 µg/mL) |

*Figure 10*

A

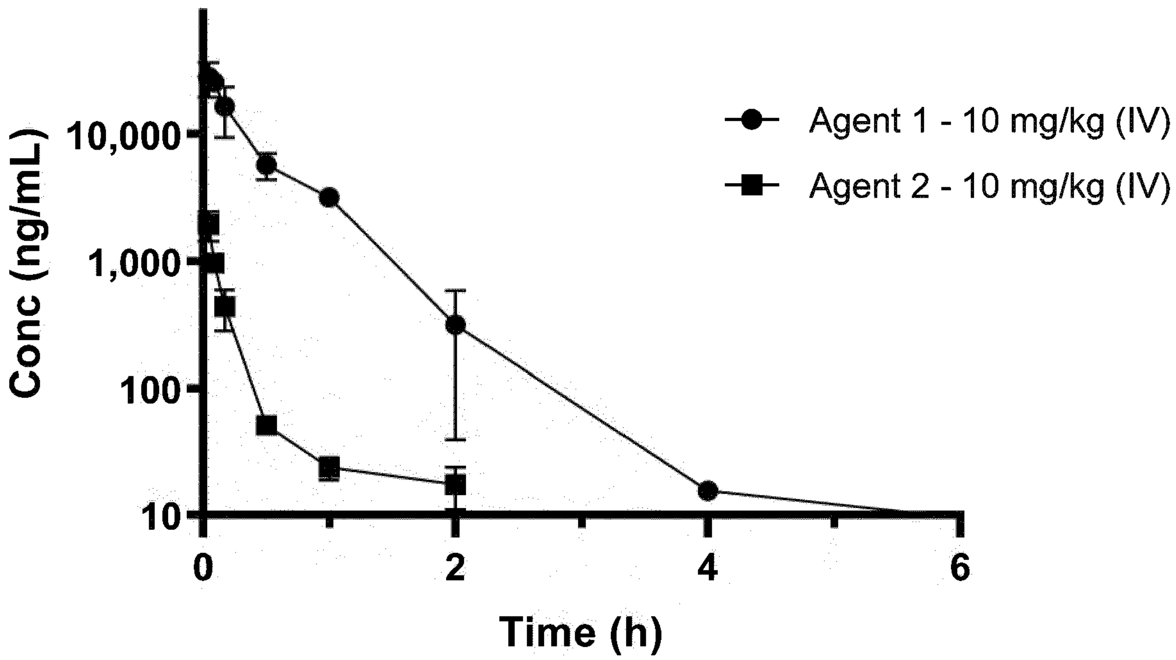


| **PK parameters** mouse | **Agent 2** *Mean* | **Agent 1** *Mean* |
|---|---|---|
| **$C_0$ (ng/mL)** | 3,910 | 30,500 |
| **$Cl_{obs}$ (L/h/kg)** | 26.8 | 0.83 |
| **$AUC_{0-inf}$ (ng·h/mL)** | 373 | 12,100 |

## *Figure 10 (continued)*

**B**

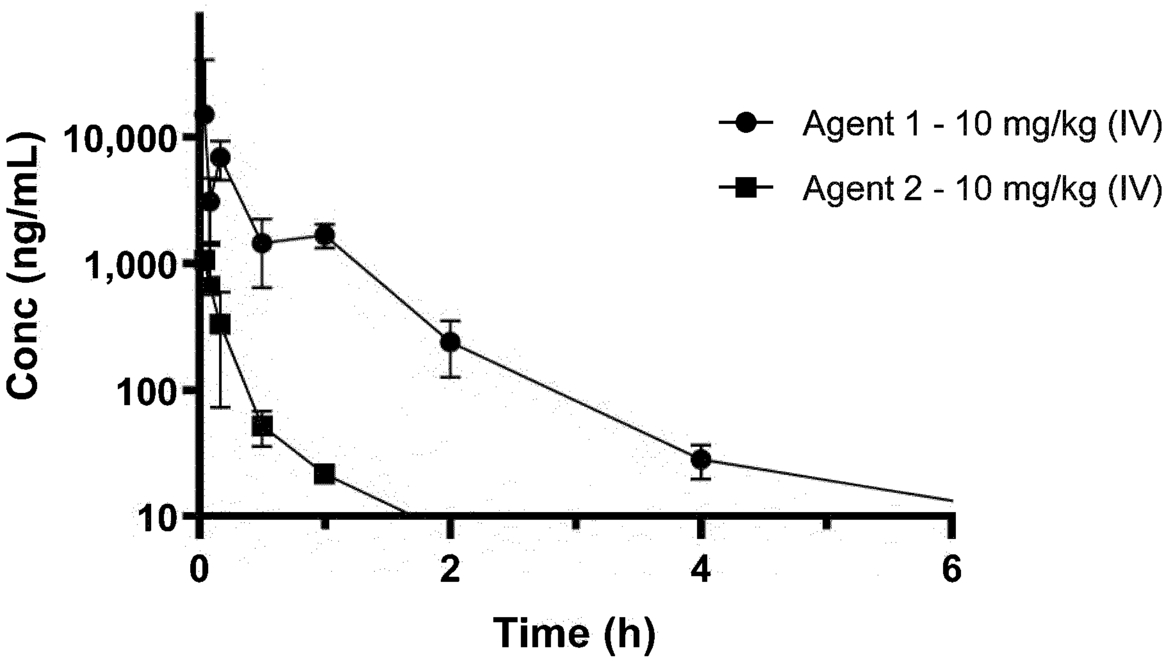


| **PK parameters** | **Agent 2** | **Agent 1** |
|---|---|---|
| rat | *Mean* | *Mean* |
| **$C_0$ (ng/mL)** | 1,730 | 72,700 |
| **$Cl_{obs}$ (L/h/kg)** | 44.5 | 1.6 |
| **$AUC_{0-inf}$ (ng·h/mL)** | 225 | 6,080 |

***Figure 10 (continued)***

C

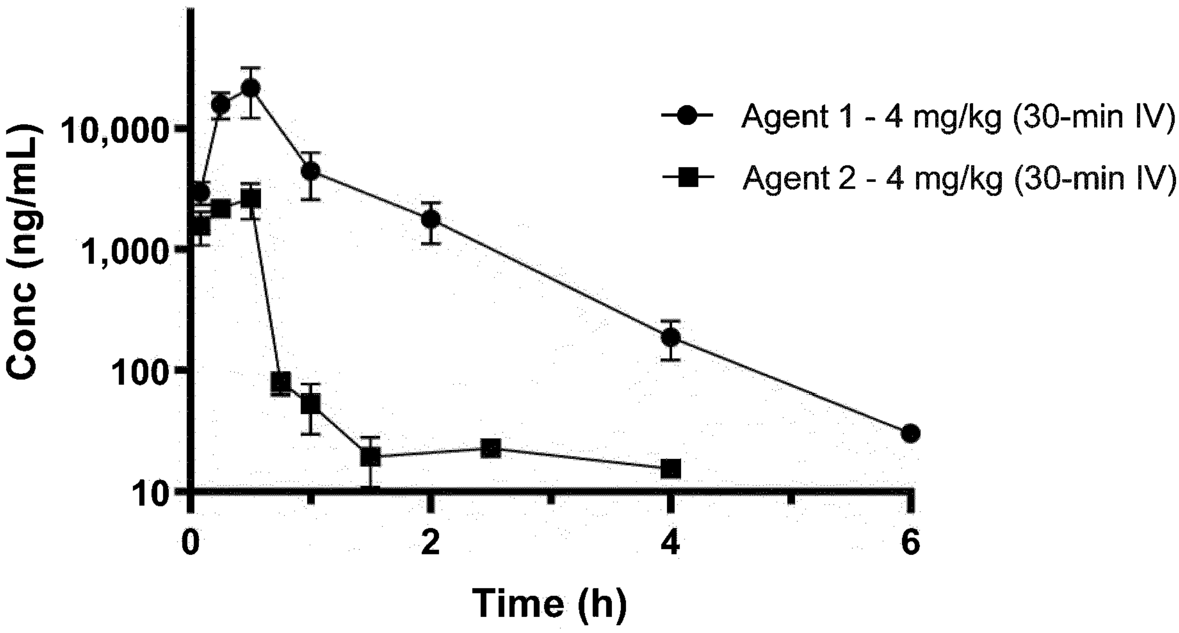


| **PK parameters** NHP | **Agent 2** *Mean* | **Agent 1** *Mean* |
|---|---|---|
| **$C_0$ (ng/mL)** | 2,760 | 22,020 |
| **$Cl_{obs}$ (L/h/kg)** | 3.23 | 0.26 |
| **$AUC_{0\text{-}inf}$ (ng·h/mL)** | 1,250 | 16,170 |

European Patent Office

## EUROPEAN SEARCH REPORT

Application Number
EP 24 17 0219

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2019/154901 A1 (UNIV AARHUS [DK]) 15 August 2019 (2019-08-15) * page 17, line 29 * * page 17, line 32 * * page 2, line 28 - line 31 * | 1-15 | INV. A61K47/54 A61K47/64 A61K39/395 A61P35/00 C07K14/47 C07K16/28 ADD. A61K39/00 |
| A | WO 2018/029256 A1 (UNIV AARHUS [DK]) 15 February 2018 (2018-02-15) * page 49, line 14 * | 1-15 | |
| A | Olesen Claus Elsborg ET AL: "Systemic delivery of ST317, a cell penetrating STING pathway sensitizer, results in strong anti-tumor activity, Cancer Res 2023;83(7_Suppl):Abstract nr 1852", Proceedings of the American Association for Cancer Research Annual Meeting 2023, 4 April 2023 (2023-04-04), pages 1-4, XP093085382, DOI: 10.1158/1538-7445.AM2023-1852 Retrieved from the Internet: URL:https://aacrjournals.org/cancerres/article/83/7_Supplement/1852/720888/Abstract-1852-Systemic-delivery-of-ST317-a-cell [retrieved on 2023-09-25] * the whole document * | 1-15 | TECHNICAL FIELDS SEARCHED (IPC) A61K A61P C07K |

The present search report has been drawn up for all claims

1

| Place of search | Date of completion of the search | Examiner |
|---|---|---|
| The Hague | 27 June 2024 | Sixta, Georg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 17 0219

This annex lists the patent family members relating to the patent documents cited in the above-mentioned European search report. The members are as contained in the European Patent Office EDP file on
The European Patent Office is in no way liable for these particulars which are merely given for the purpose of information.

27-06-2024

| Patent document cited in search report | Publication date | Patent family member(s) | Publication date |
|---|---|---|---|
| WO 2019154901 A1 | 15-08-2019 | AU 2019219072 A1 | 13-08-2020 |
| | | CA 3089916 A1 | 15-08-2019 |
| | | CN 112351993 A | 09-02-2021 |
| | | EP 3749684 A1 | 16-12-2020 |
| | | JP 2021514183 A | 10-06-2021 |
| | | US 2020399335 A1 | 24-12-2020 |
| | | WO 2019154901 A1 | 15-08-2019 |
| WO 2018029256 A1 | 15-02-2018 | CA 3069780 A1 | 15-02-2018 |
| | | EP 3497119 A1 | 19-06-2019 |
| | | US 2019292236 A1 | 26-09-2019 |
| | | US 2024182538 A1 | 06-06-2024 |
| | | WO 2018029256 A1 | 15-02-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018029256 A
- WO 2019154901 A


### Non-patent literature cited in the description


- **XIE.** *Front. Pharmacol.,* 20 May 2020
- *Sec. Experimental Pharmacology and Drug Discovery, Derakhshankhah and Jafari, Biomedicine & Pharmacotherapy,* 2018, vol. 108, 1090-1096
- *Bottens & Yamada. Cell-Penetrating Peptides (CPPs) as Therapeutic and Diagnostic Agents for Cancer. Cancers,* 2022, vol. 14, 5546
- **AVAL et al.** *J. Clin. Med.,* 2020, vol. 9 (3323
- **AMOUZEGAR et al.** *Cancers,* 2021, vol. 13 (11), 2695
- **W. C. CHAN ; P. D. WHITE.** Fmoc Solid Phase Synthesis - A Practical Approach. Oxford University Press, 2000
- **JANSSON, K ; LAUSTSEN, A. ; KRAPP, C. et al.** IFI16 is required for DNA sensing in human macrophages by promoting production and function of cGAMP. *Nat Commun,* 2017, vol. 8, 14391, https://doi.org/10.1038/ncomms14391
- **LÄTTIG-TÜNNEMANN, G. et al.** Backbone rigidity and static presentation of guanidinium groups increases cellular uptake of arginine-rich cell-penetrating peptides. *Nat. Commun.,* 2011, vol. 2, 453
- **NISCHAN et al.** Covalent Attachment of Cyclic TAT Peptides to GFP Results in Protein Delivery into Live Cells with Immediate Bioavailability. *Angew. Chem. Int. Ed.,* 2015, vol. 54, 1950-1953
- **MOISE L et al.** The two-faced T cell epitope: examining the host-microbe interface with JanusMatrix. *Hum Vaccin Immunother.,* July 2013, vol. 9 (7), 1577-86
- **SAMBROOK, J. ; RUSSEL, D.W.** Molecular Cloning, A Laboratory Manual. Cold Spring Harbor Laboratory Press, 2001
- **AMOUZEGAR et al.** STING Agonists as Cancer Therapeutics. *Cancers,* 2021, vol. 13 (11), 2695
- **LEE et al.** Improvement of STING-mediated cancer immunotherapy using immune checkpoint inhibitors as a game-changer. *Cancer Immunol Immunother,* December 2022, vol. 71 (12), 3029-3042
- **CAÑADAS et al.** Tumor innate immunity primed by specific interferon-stimulated endogenous retroviruses. *Nat. Med.,* 2018, vol. 24, 1143-1150
- **FALAHAT et al.** Epigenetic reprogramming of tumor cell: Intrinsic STING function sculpts antigenicity and T cell recognition of melanoma. *Proc. Natl. Acad. Sci. U.S.A.,* 2021, vol. 118 (e2013598118
- **QIAN et al.** Discovery and Mechanism of Highly Efficient Cyclic Cell-Penetrating Peptides. *Biochemistry,* 10 May 2016, vol. 55 (18), 2601-12
- **SCHREINER B ; MITSDOERFFER M ; KIESEIER BC ; CHEN L ; HARTUNG HP ; WELLER M ; WIENDL H.** Interferon-beta enhances monocyte and dendritic cell expression of B7-H1 (PD-L1), a strong inhibitor of autologous T-cell activation: relevance for the immune modulatory effect in multiple sclerosis. *J Neuroimmunol.,* October 2004, vol. 155 (1-2), 172-82
- **GARCIA-DIAZ A ; SHIN DS ; MORENO BH ; SACO J ; ESCUIN-ORDINAS H ; RODRIGUEZ GA ; ZARETSKY JM ; SUN L ; HUGO W ; WANG X.** Interferon Receptor Signaling Pathways Regulating PD-L1 and PD-L2 Expression. *Cell Rep.,* 09 May 2017, vol. 19 (6), 1189-1201
- **ZATREANU, D. ; ROBINSON, H.M.R. ; ALKHATIB, O. et al.** Polθ inhibitors elicit BRCA-gene synthetic lethality and target PARP inhibitor resistance. *Nat Commun,* 2021, vol. 12, 3636
- **ZHOU, J. ; GELOT, C. ; PANTELIDOU, C. et al.** A first-in-class polymerase theta inhibitor selectively targets homologous-recombination-deficient tumors. *Nat Cancer,* 2021, vol. 2, 598-610
- **RODRIGUEZ-BERRIGUETE G ; RANZANI M ; PREVO R ; PULIYADI R ; MACHADO N ; BOLLAND HR ; MILLAR V ; EBNER D ; BOURSIER M ; CERUTTI A.** Small-molecule Polθ inhibitors provide safe and effective tumor radiosensitization in preclinical models. *Clin Cancer Res.,* 23 January 2023, CCR-22-2977